# EUROPEAN PATENT APPLICATION

(11) **EP 3 085 788 A1**
(43) Date of publication of application: **26.10.2016**
(21) Application number: 15164928.2
(22) Date of filing: 23.04.2015
(51) Int. Cl.: C12N 15/62, C12N 15/81, C12P 7/64, C12R 1/64, C12N 9/42, C12R 1/645

(54) **MUTANT YEAST STRAIN CAPABLE OF DEGRADING CELLOBIOSE**

(71) Applicant: INSTITUT NATIONAL DE LA RECHERCHE AGRONOMIQUE, 75007 Paris (FR); INSTITUT NATIONAL DES SCIENCES APPLIQUEES DE TOULOUSE, 31077 Toulouse Cedex 4 (FR); Centre National de la Recherche Scientifique, 75016 Paris (FR)
(72) Inventor: Marty, Alain, 31000 Toulouse (FR); Guo, Zhongpeng, 31320 Toulouse (FR); Duquesne, Sophie, 31300 Toulouse (FR); Bozonnet, Sophie, 31500 Toulouse (FR); Nicaud, Jean-Marc, 78190 Trappes (FR); O'Donohue, Michael, 31500 Toulouse (FR)
(74) Representative: Gevers & Orès

(57) **Abstract**

The invention relates to a method for obtaining a mutant oleaginous yeast strain capable of growing on cellobiose as carbon source, comprising overexpressing in said strain two β-glucosidase enzymes further comprising a N-terminal signal peptide. The invention also relates to a mutant yeast strain obtained by said method.

## Description

The present invention relates to mutant yeast strains, such as *Yarrowia lipolytica* strains, capable of growing on cellobiose as carbon source and means for obtaining such mutant strains.

It is widely recognized that lignocellulosic biomass (or LC biomass) will form an important part of the future bio-economy. However, the use of this renewable resource as feedstock for industrial activities poses a major challenge, because its deconstruction to sugars and lignin is complex, requiring a series of unit operations. These include costly pretreatment and enzyme hydrolysis steps, the latter requiring the action of several types of enzymes (Pedersen and Meyer, 2010; Wilson, 2009). Indeed, the hydrolysis of cellulose alone requires the synergistic action of endoglucanases (EC 3.2.1.4), cellobiohydrolases (EC 3.2.1.91) and β-glucosidases (EC 3.2.1.21) (Tomme and Warren, 1995). Endoglucanases are active on the internal bonds in cellulose and release free reducing and non-reducing extremities, which are used by cellobiohydrolases as starting points for exo-processive hydrolysis that yields cellodextrins as products. Finally, β-glucosidases convert cellodextrins into glucose (Sun and Cheng, 2002).

One strategy to reduce investment and operational costs in LC biomass processing is to internalize enzyme production and combine enzyme hydrolysis with fermentation. This is known as consolidated bioprocessing (or CBP) and can be achieved using a microorganism that possesses the dual ability to produce biomass-hydrolyzing enzymes and ferment sugars to products of commercial interest, thus allowing a one-pot type bioconversion process in which process integration is maximized (Lynd *et al*., 2005). While CBP is considered to be an ultimate aim for biorefining, the ways to achieve this goal are not simple. By way of example, efforts at engineering *Yarrowia lipolytica* able to degrading xylose - which is not reported as being naturally consumed by *Y. lipolytica -* by overexpressing endogenous putative genes involved in xylose degrading pathway, were not successful (see International Application WO 2013/192520). Although the number of naturally-occurring, biomass-degrading microorganisms is no doubt large, those that possess the ability to hydrolyze LC biomass and ferment free sugars into desired products, such as ethanol, butanol, hydrogen, fatty acid ethyl esters (FAEE) or isopropanol, at industrially-compatible rates and titers, are probably very rare and so far undiscovered (La Grange, 2010). Additionally, many of the best known biomass-degrading microorganisms display low β-glucosidase (cellobiase) activity, meaning that the hydrolysis of cellobiose constitutes a rate-limiting step during the enzymatic processing of cellulose (Duff, 1985; Holtzapple *et al*., 1990; Stockton *et al*., 1991). Therefore, engineering cellobiose-degrading ability into microorganisms is a vital step towards the development of cellulolytic biocatalysts suitable for CBP. In this respect, examples of recent work performed on *Saccharomyces cerevisiae,* the current workhorse of biotechnological processes, are noteworthy (Lee *et al*., 2013; Lian *et al*., 2014; Nan *et al*., 2014). In these studies, even though the engineered *S. cerevisiae* strains exhibited poor cellulose-degrading ability, the fact that they both produce significant cellobiase activity means that their incorporation into a simultaneous saccharification and fermentation (SSF) process is likely to reduce the loading of external cellulases and thus overall process cost (Lee *et al*., 2013).

Although ethanol is the target molecule in many biorefinery concepts, Fatty Acid Esters (FAEs) such as those used in biodiesel, are also attractive targets. This is because FAEs display high energy density and are well-tolerated by production strains (Zhang *et al*., 2012). Currently, FAEs are mainly produced by transesterification of plant oils using an alcohol (methanol or ethanol) and base, acid or enzyme catalysts (Demirba 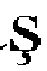 , 2003). However, the high cost of this process and various issues surrounding the production of plant oils for non-food purposes makes the search for alternative routes both attractive and strategically pertinent. In this respect, microbial production of biofuels (so-called microdiesel and microkerosene) represents a sustainable and quite economical way to produce FAEs. For this purpose, both *Esherichia coli* and *S. cerevisiae* have been engineered to produce structurally-tailored fatty esters (Steen *et al*., 2010; Shi *et al*., 2012; Runguphan and Keasling, 2014). However, neither of these microorganisms is naturally able to accumulate high amounts of lipids, nor are they able to degrade cellulose. Moreover, in these microorganisms the biosynthesis of fatty acid is highly regulated (Nielsen, 2009), thus limiting the possibility to improve lipid production (Runguphan and Keasling, 2014; Shi *et al*., 2012; Valle-Rodríguez *et al*., 2014).

So-called oleaginous microorganisms, which naturally accumulate lipids to more than 20% of their dry cell weight (DCW) (Ratledge, 2005; Thevenieau and Nicaud, 2013), have already been exploited for the production of commercially-useful lipids, such as substitutes for cocoa butter and polyunsaturated fatty acids (Papanikolaou and Aggelis, 2010). Therefore, it is unsurprising that microbial lipid or single cell oil (SCO) is also being considered for biodiesel production, especially because this route implies shorter production times, reduced labor costs and simpler scale-up (Easterling *et al*., 2009). Prominent among the oleaginous microorganisms, *Yarrowia lipolytica* has been extensively studied and is known to accumulate lipids up to 50% of its dry weight depending on culture conditions (Blazeck *et al*., 2014; Ratledge, 2005; Thevenieau and Nicaud, 2013). Advantageously, since *Y. lipolytica* is already widely used in the detergent, food, pharmaceutical, and environmental industries it has been classified by the FDA (Food and Drug Administration) as "Generally Recognized as Safe" (GRAS) for numerous processes (Groenewald *et al*., 2014). Nevertheless, despite these advantages, *Y. lipolytica* displays limited ability for sugar use and is unable to use cellulose or cellobiose as carbon source (Michely *et al*., 2013), while its genome comprises 6 predicted β-glucosidase genes (*BGLs*) (Wei *et al*., 2014). However, in the absence of biochemical data it is impossible to assert that these 6 predicted β-glucosidase genes actually encode β-glucosidases, since family GH3 contains glycoside hydrolases that display other specificities and also because *Y. lipolytica* does not grow on cellobiose and has not been found to express a detectable level of β-glucosidase activity.

In a recent paper, the use of cellobiose by *Y. lipolytica* was tackled for the first time, thus opening the way towards the development of an efficient yeast-based CBP microorganism capable of consuming cellulose-derived glucose and converting it into lipids and derivatives thereof (Lane *et al*., 2014).

The inventors have shared this aim (*i.e*., providing a cellobiose-degrading mutant *Y. lipolytica*), but have employed a different strategy that relies upon the activation of endogenous β-glucosidase activity. The inventors have identified two genes, *BGL1* (YALIF16027g) and *BGL2* (YALI0B14289g), encoding active β-glucosidases in *Y. lipolytica,* referred to as SEQ ID NO: 4 (YALI_BGL1) and SEQ ID NO: 6 (YALI_BGL2) respectively. The two active β-glucosidases, one of which was mainly cell-associated while the other was present in the extracellular medium, were purified and characterized. The specific growth rate of mutant *Y. lipolytica* co-expressing *BGL1* (wherein the coding sequence is referred to as SEQ ID NO: 3) and *BGL2* (wherein the coding sequence is referred to as SEQ ID NO: 5) on cellobiose was 0.16 h⁻¹, similar to that of the control grown on glucose in defined media. Significantly, *Y. lipolytica* Δ*pox* co-expressing both *BGLs* grew better than the strains expressing single *BGLs* in simultaneous saccharification and fermentation on cellulose.

In addition, the comparison of the specific activities of YALI_BGL1 (also referred to as Bgl1) and YALI_BGL2 (also referred to as Bgl2) on cellobiose (108 units/mg and 25 units/mg protein respectively) with that of the commercially available β-glucosidase from *Aspergillus niger* (5.2 units/mg protein), the enzyme that is generally used to complement the cellulolytic cocktail of *T. reesei* (Yan and Lin, 1997), is rather flattering for the former. Moreover, the *K*_{M} values describing the cellobiolytic reactions catalyzed by Bgl1 and Bgl2 are approximately 10 and 4-fold lower than those of the β-glucosidases from *S. fibuligera* (2.8 mM Bgl1) and *A. niger* (2.7 mM) (Yan and Lin, 1997), meaning that the minimum concentration of cellobiose required for effective catalysis to occur is much lower. Likewise, comparing the apparent performance constants, *k*_{cat}/*K*_{M}, of *Y. lipolytica* Bgls with those of other reported β-glucosidases (Belancic *et al*., 2003; Daroit *et al*., 2008; Galas and Romanowska, 1996; González-Pombo *et al*., 2008; Leclerc *et al*., 1987; Machida *et al*., 1988; Yan and Lin, 1997) suggests that the enzymes described in this study hydrolyze cellodextrins more efficiently.

The bi-functional *Y. lipolytica* (expressing *BGL1* and *BGL2*) is of an interest for biotechnological processes related to lipid production from lignocellulosic biomass. In addition, overexpression of *BGL1* and *BGL2* in oleaginous yeast strains other than *Y. lipolytica* is also of an interest for biotechnological processes.

Accordingly, the present invention provides a method for obtaining an oleaginous yeast strain capable of growing on cellobiose as carbon source, wherein said method comprises overexpressing in said strain a β-glucosidase (EC 3.2.1.21) having at least 80% identity, or by order of increasing preference at least 83%, 85%, 90%, 92%, 95%, 96%, 97%, 98% or 99% identity, with the polypeptide of sequence SEQ ID NO: 1 (mature YALI_BGL1) further comprising a N-terminal signal peptide and a β-glucosidase (EC 3.2.1.21) having at least 80% identity, or by order of increasing preference at least 82%, 85%, 90%, 92%, 95%, 96%, 97%, 98% or 99% identity, with the polypeptide of sequence SEQ ID NO: 2 (mature YALI_BGL2) further comprising a N-terminal signal peptide.

The signal peptide (or signal sequence) drives secretion of the β-glucosidase into the extracellular space (e.g., periplasm, external medium) of the yeast. After secretion, the signal peptide is usually cleaved (removed) by a peptidase leading to a mature β-glucosidase. The signal peptide can further allow glycosylation in the case of β-glucosidase bearing potential sites of glycosylation.

Signal peptides are well known in the art (see for review von Heijne, 1985). In particular, signal peptides able to drive secretion of a protein into the extracellular space in yeast and methods to perform such a secretion are well known in the art (see Sreekrishna *et al*., 1997; Hashimoto *et al*., 1998; Koganesawa *et al*.; 2001; Gasmi *et al*., 2011; Madzak and Beckerich, 2013). Methods for identifying a signal peptide (signal sequence) are also well known in the art. One can use the programs Signal-BLAST (Franck and Sippl, 2008) and/or SignalP 4.1 Server (Petersen *et al*., 2011).

The signal peptide can be from a protein from any organism, such as mammal, bacteria, yeast, preferably from a protein form a yeast, more preferably from *Yarrowia.*

The signal peptide of the β-glucosidase having at least 80% identity with the polypeptide of sequence SEQ ID NO: 1 and the signal peptide of the β-glucosidase having at least 80% identity with the polypeptide of sequence SEQ ID NO: 2 can be identical or different, in terms of space of secretion (periplasm or external medium) and/or in terms of amino acid sequence. By way of example, the signal peptide of the β-glucosidase having at least 80% identity with the polypeptide of sequence SEQ ID NO: 1 drives secretion of said β-glucosidase into the periplasm space or the external medium and the signal peptide of the β-glucosidase having at least 80% identity with the polypeptide of sequence SEQ ID NO: 2 drives secretion of said β-glucosidase into the periplasm space or the external medium.

Advantageously, the signal peptide of the β-glucosidase having at least 80% identity with the polypeptide of sequence SEQ ID NO: 1 drives secretion of said β-glucosidase into the periplasm space. It can be chosen from the signal peptides SEQ ID NO: 34 or SEQ ID NO: 35.

Advantageously, the signal peptide of the β-glucosidase having at least 80% identity with the polypeptide of sequence SEQ ID NO: 2 drives secretion of said β-glucosidase into the external medium. It can be chosen from the signal peptides SEQ ID NO: 36, SEQ ID NO: 37, SEQ ID NO: 38 or SEQ ID NO: 39.

The signal peptide can be the naturally occurring signal sequence of the β-glucosidase to overexpress or a modified signal sequence, such as the *Yarrowia* alkaline extracellular protease (Aep; Fabre *et al*., 1991) or the extracellular lipase (Lip2p) signal sequences (Pignède *et al*., 2000; Nicaud *et al*., 2002).

The term overexpressing a β-glucosidase in a yeast strain, herein refers to artificially increasing the quantity of said β-glucosidase produced in a yeast strain compared to a reference (control) yeast strain (wherein said β-glucosidase is not overexpressed). This term also encompasses expression of a β-glucosidase in a yeast strain which does not naturally contain a gene encoding said β-glucosidase.

An advantageous method for overexpressing both β-glucosidases comprises introducing into the genome of said yeast strain DNA constructs comprising a nucleotide sequence encoding said β-glucosidases, placed under the control of a promoter.

Nucleotide sequences encoding YALI_BGL1 and YALI_BGL2 are provided in SEQ ID NO: 3 and SEQ ID NO: 5 respectively.

Unless otherwise specified, the percent of identity between two sequences which are mentioned herein is calculated from an alignment of the two sequences over their whole length, not comprising the signal sequence. One can use the BLAST program (Tatusova and Madden TL, 1999) with the default parameters (open gap penalty = 2; extension gap penalty = 5; matrix = BLOSUM 62).

Methods for determining the presence of a signal sequence (signal peptide) in a protein are well known in the art. One can use the programs Signal-BLAST (Franck and Sippl, 2008) and/or SignalP 4.1 Server (Petersen *et al*., 2011).

Methods for determining whether an enzyme has a β-glucosidase activity (EC 3.2.1.21) are known in the art. By way of example, β-glucosidase activity can be measured by quantifying the release of *p*NP (*p*-nitrophenol) from *p*NPGlc as described in Guo *et al*., 2011.

Advantageously, the β-glucosidase having at least 80% identity with the polypeptide of sequence SEQ ID NO: 1 (mature YALI_BGL1) is from a *Yarrowia* strain, preferably selected from *Y. lipolytica* and *Y. galli,* more preferably from a *Y. lipolytica* strain.

In a preferred embodiment, the β-glucosidase having at least 80% identity with the polypeptide of sequence SEQ ID NO: 1 (mature YALI_BGL1) is selected from the group consisting of the β-glucosidase of SEQ ID NO: 1 (mature YALI_BGL1), and SEQ ID NO: 8 (YAGA_BGL1 without its naturally occurring N-terminal signal sequence), preferably SEQ ID NO: 1 (mature YALI_BGL1).

In another preferred embodiment, the β-glucosidase having at least 80% identity with the polypeptide of sequence SEQ ID NO: 1 (mature YALI_BGL1) further comprising a N-terminal signal peptide is selected from the group consisting of SEQ ID NO: 4 (YALI_BGL1; BGL1 from *Yarrowia lipolytica* CLIB122, comprising its naturally occurring N-terminal signal sequence) and SEQ ID NO: 7 (YAGA_BGL1; BGL1 from *Yarrowia galli* CBS 9722, comprising its naturally occurring N-terminal signal sequence), preferably SEQ ID NO: 4.

The β-glucosidases included in sequences SEQ ID NO: 4 (YALI_BGL1) and SEQ ID NO: 7 (YAGA_BGL1) have respectively 100% and 84.08% identity with the polypeptide of sequence SEQ ID NO: 1 (mature YALI_BGL1).

Advantageously, the β-glucosidase having at least 80% identity with the polypeptide of sequence SEQ ID NO: 2 (mature YALI_BGL2) is from a *Yarrowia* strain, preferably from a *Y. lipolytica, Y. galli, Y. yakushimensis* or *Yarrowia alimentaria* strain, more preferably from a *Y. lipolytica* strain.

In another preferred embodiment, the β-glucosidase having at least 80% identity with the polypeptide of sequence SEQ ID NO: 2 (mature YALI_BGL2) has the amino acid sequence SEQ ID NO: 9. This sequence SEQ ID NO: 9 corresponds to the consensus amino acid sequence obtained from mature YALI_BGL2, YAGA_BGL2, YAYA_BGL2 and YAAL_BGL2 (as described above).

In another preferred embodiment, the β-glucosidase having at least 80% identity with the polypeptide of sequence SEQ ID NO: 2 (mature YALI_BGL2) is selected from the group consisting of the β-glucosidase of SEQ ID NO: 2 (mature YALI_BGL2), SEQ ID NO: 11 (YAGA_BGL2 without its naturally occurring N-terminal signal sequence), SEQ ID NO: 13 (YAYA_BGL2 without its naturally occurring N-terminal signal sequence) and SEQ ID NO: 15 (YAAL_BGL2 without its naturally occurring N-terminal signal sequence), preferably SEQ ID NO: 2 (mature YALI_BGL2).

In another preferred embodiment, the β-glucosidase having at least 80% identity with the polypeptide of sequence SEQ ID NO: 2 (mature YALI_BGL2) further comprising a N-terminal signal peptide is selected from the group consisting of SEQ ID NO: 6 (YALI_BGL2; BGL2 from *Yarrowia lipolytica* CLIB122, comprising its naturally occurring N-terminal signal sequence), SEQ ID NO: 10 (YAGA_BGL2; BGL2 from *Yarrowia galli* CBS 9722, comprising its naturally occurring N-terminal signal sequence), SEQ ID NO: 12 (YAYA_BGL2; BGL2 from *Yarrowia yakushimensis* CBS 10253, comprising its naturally occurring N-terminal signal sequence) and SEQ ID NO: 14 (YAAL_BGL2; BGL2 from *Yarrowia alimentaria* CBS 10151, comprising its naturally occurring N-terminal signal sequence), preferably SEQ ID NO: 6.

The β-glucosidases included in sequences SEQ ID NO: 6 (YALI_BGL2), SEQ ID NO: 10 (YAGA_BGL2), SEQ ID NO: 12 (YAYA_BGL2) and SEQ ID NO: 14 (YAAL_BGL2) have respectively 100%, 93.55%, 85.21% and 82.14% identity with the polypeptide of sequence SEQ ID NO: 2 (mature YALI_BGL2).

In another preferred embodiment, both β-glucosidases (the β-glucosidase having at least 80% identity with the polypeptide of sequence SEQ ID NO: 1 and the β-glucosidase having at least 80% identity with the polypeptide of sequence SEQ ID NO: 2) are from a *Yarrowia* strain, more preferably from a *Y. lipolytica* or *Y. galli* strain, and most preferably from a *Y. lipolytica* strain.

Oleaginous yeast strains, which naturally accumulate lipids to more than 20% of their dry cell weight, are well known in the art (Ratledge, 1994 and 2005). They include the genus *Candida, Cryptoccocus*, *Lipomyces*, *Rhodosporidium* (*e.g., Rhodosporidium toruloides*), *Rhodotorula (e.g., Rhodotorula glutinis*), *Trichosporon* and *Yarrowia.*

In a preferred embodiment, the oleaginous yeast strain is a *Yarrowia* strain, preferably a *Yarrowia lipolytica* strain.

Advantageously, said yeast strain is auxotrophic for leucine (Leu) and optionally for the decarboxylase orotidine-5'-phosphate (Ura-).

Said yeast can also be a mutant yeast strain wherein the expression or activity of the endogenous isoforms of acyl-coenzymeA oxidases (AOX, EC 6.2.1.3) involved, at least partially, in the β-oxidation of fatty acids, is inhibited. In yeasts, 6 genes (*POX1*, *POX2*, *POX3, POX4, POX5* and *POX6*) encode these isoforms. Said inhibition of the expression or activity can be total or partial. Total or partial inhibition of the expression or activity of these enzymes leads to accumulation by yeast of dodecanedioic acid without use of accumulated fat. More particularly, the coding sequence of the genes *POX1-6* and the peptide sequence of AOX1-6 from *Y. lipolytica* CLIB122 are available in either at Génolevures database (http://genolevures.org/) or at GRYC database (http://gryc.inra.fr/) or in GenBank database under the following accession numbers or names: *POX1* / AOX1 = YALI0E32835g / YALI0E32835p, *POX2* / AOX2 = YALI0F10857g / YALI0F10857p; *POX3* / AOX3 = YALI0D24750g / YALI0D24750p; *POX4* / AOX4 = YALI0E27654g / YALI0E27654p; *POX5* / AOX5 = YALI0C23859g / YALI0C23859p; *POX6* / AOX6 = YALI0E06567g / YALI0E06567p. The peptide sequences of the acyl-CoA oxidases of *Y. lipolytica* have 45% identity or 50% similarity with those from other yeasts. The degree of identity between the acyl-CoA oxidases varies from 55% to 70% (or from 65 to 76% similarity) (see International Application WO 2006/064131). A method of inhibiting the expression of the 6 endogenous AOX in a *Y. lipolytica* strain is described in Beopoulos *et al*., 2008 and International Applications WO 2006/064131, WO 2010/004141 and WO 2012/001144.

The yeast strain can further comprise other mutations such as those described in International Applications WO 2006/064131, WO 2010/004141, WO 2012/001144, WO 2014/178014 and WO 2014/136028 which are useful for obtaining a fatty acids producing yeast strain.

In particular, the yeast strain can be genetically modified to improve lipid accumulation. Said yeast strain having improved properties for lipid accumulation can be a mutant yeast strain, preferably a *Y. lipolytica* mutant strain, wherein at least one protein, preferably at least one endogenous protein, selected from the group consisting of an acyl-CoA:diacylglycerol acyltransferase 2 (encoded by *DGA1*), an acyl-CoA:diacylglycerol acyltransferase 1 (encoded by *DGA2*), a glycerol-3-phosphate dehydrogenase NAD+ (encoded by *GPD1*), an acetyl-CoA carboxylase (encoded by *ACC1*) and a hexokinase (encoded by *HXK1*) is overexpressed, and/or the expression or activity of at least one endogenous protein selected from the group consisting of the glycerol 3-phosphate dehydrogenase (encoded by *GUT2*), the triglyceride lipase (encoded by *TGL4*) and the peroxin 10 (encoded by *PEX10*) is inhibited.

Advantageously, the 5 proteins, acyl-CoA:diacylglycerol acyltransferase 2, acyl-CoA:diacylglycerol acyltransferase 1, glycerol-3-phosphate dehydrogenase NAD+, acetyl-CoA carboxylase and hexokinase, are overexpressed, and the expression or activity of the 3 endogenous proteins, glycerol 3-phosphate dehydrogenase, triglyceride lipase and peroxyne, are inhibited in said mutant yeast strain.

A method of overexpressing the endogenous genes *DGA1*, *DGA2*, *GPD1* and *ACC1* and inhibiting the expression or activity of the endogenous genes *GUT2*, *TGL4* and *PEX10* in a *Y. lipolytica* strain is described in International Application WO 2014/136028.

A method of overexpressing the endogenous genes *DGA2*, *GPD1* and *HXK1*, and inhibiting the expression or activity of the endogenous gene *TGL4* in a *Y. lipolytica* strain is described in Lazar *et al*., 2014.

Overexpression of a β-glucosidase (endogenous, ortholog, heterologous) in a yeast strain according to the present invention can be obtained in various ways by methods known *per se.*

Overexpression of a β-glucosidase as defined in the present invention may be performed by placing one or more (preferably two or three) copies of the open reading frame (ORF) of the sequence encoding said β-glucosidase under the control of appropriate regulatory sequences. Said regulatory sequences include promoter sequences, located upstream (at 5' position) of the ORF of the sequence encoding said β-glucosidase, and terminator sequences, located downstream (at 3' position) of the ORF of the sequence encoding said β-glucosidase.

Promoter sequences that can be used in yeast are well known to those skilled in the art and may correspond in particular to inducible or constitutive promoters. Examples of promoters which can be used according to the present invention, include the promoter of a *Y. lipolytica* gene which is strongly repressed by glucose and is inducible by the fatty acids or triglycerides such as the promoter of the *POX2* gene encoding the acyl-CoA oxidase 2 (AOX2) of *Y. lipolytica* and the promoter of the *LIP2* gene described in International Application WO 01/83773. One can also use the promoter of the *FBA1* gene encoding the fructose-bisphosphate aldolase (see Application US 2005/0130280), the promoter of the *GPM* gene encoding the phosphoglycerate mutase (see International Application WO 2006/0019297), the promoter of the *YAT1* gene encoding the transporter ammonium (see Application US 2006/0094102), the promoter of the *GPAT* gene encoding the O-acyltransferase glycerol-3-phosphate (see Application US 2006/0057690), the promoter of the *TEF* gene (Muller et al., 1998; Application US 2001/6265185), the hybrid promoter hp4d (described in International Application WO 96/41889), the hybrid promoter XPR2 described in Mazdak *et al.* (2000) or the hybrid promoters UAS1-TEF or UAStef-TEF described in Blazeck *et al.* (2011, 2013, 2014).

Advantageously, the promoter is the promoter of the *TEF* gene.

Terminator sequences that can be used in yeast are also well known to those skilled in the art. Example of terminator sequences which can be used according to the present invention include the terminator sequence of the *PGK1* gene and the terminator sequence of the *LIP2* gene described in International Application WO 01/83773.

The nucleotide sequence of the coding sequences of the heterologous genes can be optimized for expression in yeast by methods well known in the art (see for review Hedfalk, 2012).

Overexpression of an endogenous β-glucosidase can be obtained by replacing the sequences controlling the expression of said endogenous β-glucosidase by regulatory sequences allowing a stronger expression, such as those described above. The skilled person can replace the copy of the gene encoding an endogenous β-glucosidase in the genome, as well as its own regulatory sequences, by genetically transforming the yeast strain with a linear polynucleotide comprising the ORF of the sequence coding for said endogenous β-glucosidase under the control of regulatory sequences such as those described above. Advantageously, said polynucleotide is flanked by sequences which are homologous to sequences located on each side of said chromosomal gene encoding said endogenous β-glucosidase. Selection markers can be inserted between the sequences ensuring recombination to allow, after transformation, to isolate the cells in which integration of the fragment occurred by identifying the corresponding markers. Advantageously also, the promoter and terminator sequences belong to a gene different from the gene encoding the endogenous β-glucosidase to be overexpressed in order to minimize the risk of unwanted recombination into the genome of the yeast strain.

Overexpression of an endogenous β-glucosidase can also be obtained by introducing into the yeast strain of extra copies of the gene encoding said endogenous β-glucosidase under the control of regulatory sequences such as those described above. Said additional copies encoding said endogenous β-glucosidase may be carried by an episomal vector, that is to say capable of replicating in yeast. Preferably, these additional copies are carried by an integrative vector, that is to say, integrating into a given location in the yeast genome (Madzak *et al*., 2004). In this case, the polynucleotide comprising the gene encoding said endogenous β-glucosidase under the control of regulatory regions is integrated by targeted integration. Said additional copies can also be carried by PCR fragments whose ends are homologous to a given locus of the yeast, allowing integrating said copies into the yeast genome by homologous recombination. Said additional copies can also be carried by auto-cloning vectors or PCR fragments, wherein the ends have a zeta region absent from the genome of the yeast, allowing the integration of said copies into the yeast genome by random insertion as described in Application US 2012/0034652.

Targeted integration of a gene into the genome of a yeast cell is a molecular biology technique well known to those skilled in the art: a DNA fragment is cloned into an integrating vector, introduced into the cell to be transformed, wherein said DNA fragment integrates by homologous recombination in a targeted region of the recipient genome (Orr-Weaver *et al*., 1981).

Methods for transforming yeast are also well known to those skilled in the art and are described, *inter alia*, by Ito *et al.* (1983), Klebe *et al*., (1983) and Gysler *et al*., (1990).

Any gene transfer method known in the art can be used to introduce a gene encoding a β-glucosidase. Preferably, one can use the method with lithium acetate and polyethylene glycol described by Gaillardin *et al*., (1987) and Le Dall *et al*., (1994).

The present invention also provides means for carrying out said overexpression.

This includes, in particular, recombinant DNA constructs for expressing one or both β-glucosidase(s) as defined above in a yeast cell (*e.g., Y. lipolytica* strain). These DNA constructs can be obtained and introduced in said yeast strain by the well-known techniques of recombinant DNA and genetic engineering.

Recombinant DNA constructs of the invention include in particular expression cassettes, comprising a polynucleotide encoding one or both β-glucosidase(s) as defined above, under the control of a promoter functional in yeast cell as defined above.

The expression cassettes generally also include a transcriptional terminator, such as those describes above. They may also include other regulatory sequences, such as transcription enhancer sequences.

Recombinant DNA constructs of the invention also include recombinant vectors containing an expression cassette comprising a polynucleotide encoding one or both β-glucosidase(s) as defined above, under transcriptional control of a suitable promoter.

Recombinant vectors of the invention may also include other sequences of interest, such as, for instance, one or more marker genes, which allow for selection of transformed yeast cells.

The invention also comprises host cells containing a recombinant DNA construct of the invention. These host cells can be prokaryotic cells (such as bacteria cells) or eukaryotic cells, preferably yeast cells.

The invention also provides a method for obtaining a mutant oleaginous yeast strain, preferably a mutant *Yarrowia* strain *(e.g., Y. lipolytica* strain), capable of growing on cellobiose as carbon source as defined above, comprising transforming an oleaginous yeast cell with a recombinant DNA construct for expressing both β-glucosidases as defined above or with two recombinant DNA constructs for expressing both β-glucosidases respectively as defined above.

The term "two recombinant DNA constructs for expressing both β-glucosidases respectively as defined above" designates "a recombinant DNA construct for expressing a β-glucosidase having at least 80% identity with the polypeptide of sequence SEQ ID NO: 1 as defined above and a recombinant DNA construct for expressing a β-glucosidase having at least 80% identity with the polypeptide of sequence SEQ ID NO: 2 as defined above".

The invention also comprises an oleaginous yeast strain, preferably a *Yarrowia* strain *(e.g., Y. lipolytica* strain), genetically transformed with a recombinant DNA construct for expressing both β-glucosidases as defined above or with two recombinant DNA constructs for expressing both β-glucosidases respectively as defined above, and overexpressing both β-glucosidases as defined above. In said mutant (transgenic) yeast strain one to two recombinant DNA construct(s) of the invention is/are comprised in a transgene stably integrated in the yeast genome, so that it is passed onto successive yeast generations. Thus the mutant (transgenic) yeast strain of the invention includes not only the yeast cell resulting from the initial transgenesis, but also their descendants, as far as they contain one or two recombinant DNA construct(s) of the invention. The overexpression of both β-glucosidases as defined above in said yeast strains provides them an ability to grow on cellobiose as carbon source, when compared with an oleaginous yeast strain devoid of said transgene(s).

The present invention also comprises a mutant oleaginous yeast strain as defined above, preferably a mutant *Yarrowia* strain *(e.g., Y. lipolytica* strain), wherein a β-glucosidase having at least 80% identity with the polypeptide of sequence SEQ ID NO: 1 and a β-glucosidase having at least 80% identity with the polypeptide of sequence SEQ ID NO: 2 are overexpressed. This mutant oleaginous yeast strain is obtainable by a method of the invention and contains one or two recombinant expression cassette(s) of the invention.

The present invention further comprises a mutant oleaginous yeast strain as defined above, preferably a mutant *Yarrowia* strain *(e.g., Y. lipolytica* strain) comprising, stably integrated in its genome, a recombinant DNA construct for expressing both β-glucosidases as defined above or two recombinant DNA constructs for expressing both β-glucosidases respectively as defined above.

The present invention also provides the use of a mutant oleaginous yeast strain, preferably a mutant *Yarrowia* strain *(e.g., Y. lipolytica* strain), as defined above for producing lipids from lignocellulosic biomass, in particular from cellobiose or cellulose.

As used herein, the term producing lipids refers to the accumulation and optionally secretion of lipids.

The present invention also provides a method of producing lipids, comprising a step of growing a mutant oleaginous yeast strain, preferably a mutant *Yarrowia* strain *(e.g., Y. lipolytica* strain), of the invention on a lignocellulosic biomass, in particular on cellobiose or cellulose.

Methods for extracting and purifying lipids produced by cultured yeast strains are well known to those skilled in the art (Papanikolaou et al. 2001, 2002 and 2008; André et al., 2009). For example, the total lipids can be extracted according to the method described by Papanikolaou et al., 2001, and fractionated according to the methods described by Guo *et al*., 2000 and Fakas *et al*., 2006.

The present invention also provides an isolated β-glucosidase (EC 3.2.1.21) having an amino acid sequence selected from the group consisting of SEQ ID NO: 7, 8 and 10 to 15.

The present invention also provides the use of an isolated β-glucosidase (EC 3.2.1.21) having an amino acid sequence selected from the group consisting of SEQ ID NO: 7, 8 and 10 to 15 for degrading cellobiose.

The present invention will be understood more clearly from the further description which follows, which refers to non-limitative examples illustrating the overexpression of Bgl1 and Bgl2 in *Y. lipoytica.*

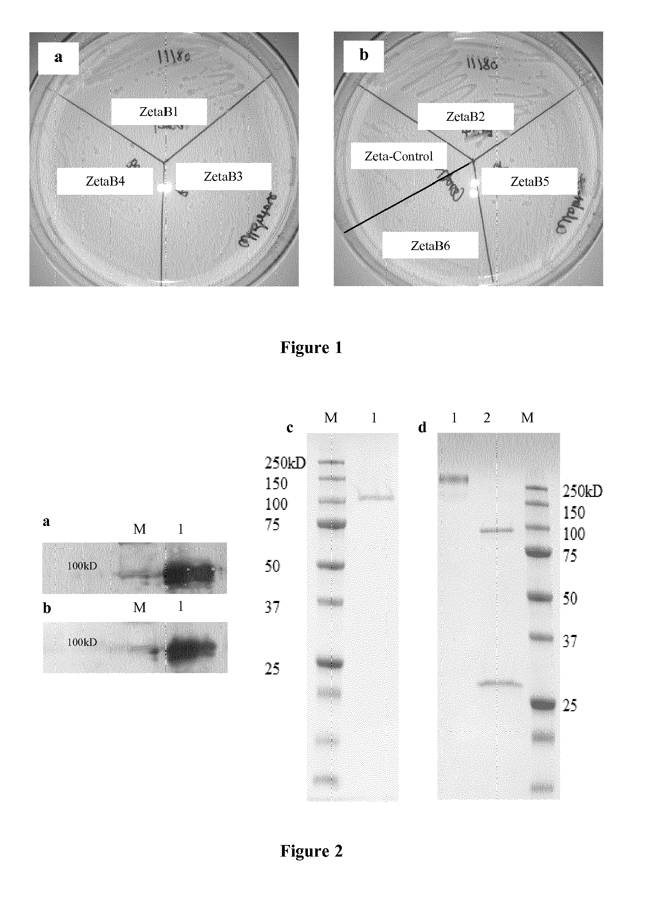

**Figure 1****:** Screening of *Y. lipolytica* expressing the 6 putative β-glucosidases on (a) indication plate containing YNBcasa medium supplemented with 1 mM p-nitrophenyl-β-D-glucoside (pNPG), and (b and c) YNBC plate with cellobiose as sole carbon source.
**Figure 2****:** Western blot detection of the expressed β-glucosidases (a) M, molecular weight standards; lane 1, intracellular Bgl1, and (b) lane 1, extracellular Bgl1, and SDS-PAGE analysis of the purified β-glucosidases from *Y. lipolytica* JMY1212 transformants (c) lane 1, purified Bgl1-His6, and (d) lane 1, purified Bgl2; lane 2, endo-H treated Bgl2 (The lower band in lane 2 represents the expected size of Endo-H).

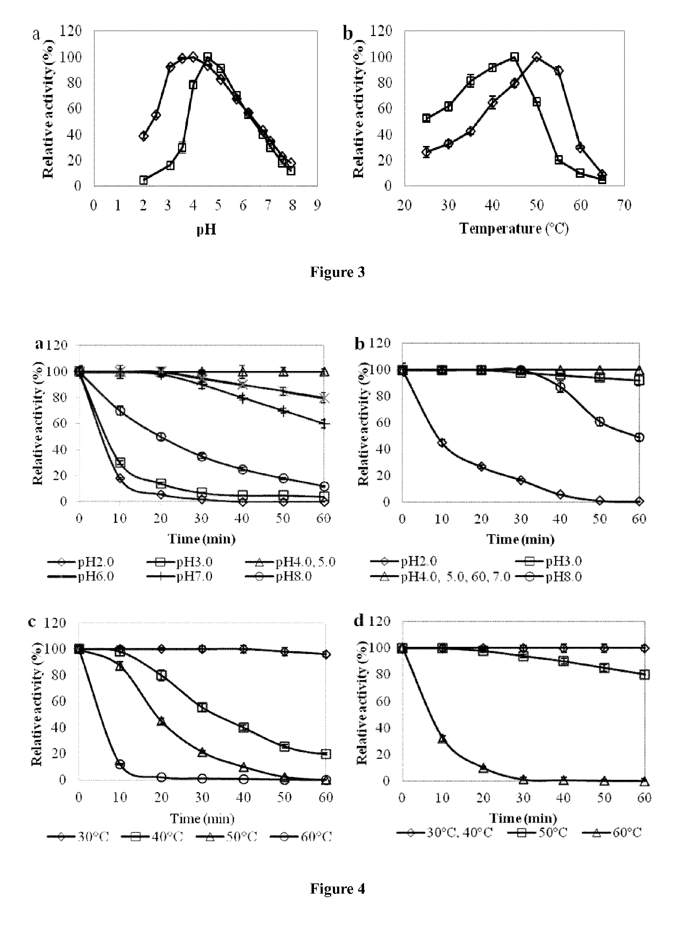

**Figure 3****:** Optimal pH (a) and temperature (b) of Bgl1 (square) and Bgl2 (diamond) from *Y. lipolytica* JMY1212. Each data point represents the mean of three independent experiments and the error bar indicates the standard deviation.
**Figure 4****:** Stability of Bgl1 (a) and Bgl2 (b) from *Y. lipolytica* JMY1212 at pH from 2.0-8.0 as a function of time at 30°C, and stability of Bgl1 (c) and Bgl2 (d) at temperature from 30°C to 60°C as a function of time at pH 5. Each data point represents the mean of three independent experiments and the error bar indicates the standard deviation. Only one curve is giving to represent the stability Bgl2 at pH 4.0, 5.0 and 6.0 (b) and at 30 °C and 40°C (d) as 100% of enzyme activity remained for these conditions.

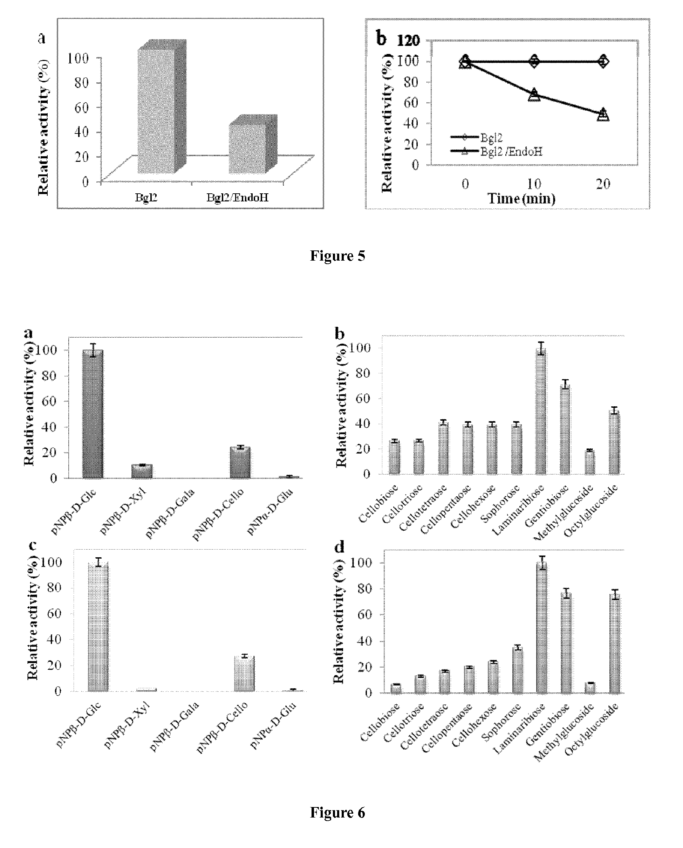

**Figure 5****:** The hydrolytic activity of Bgl2 on pNPG (a) and the stability of Bgl2 at 40°C as a function of time at pH5.0 before and after deglycosylation. Each data point represents the mean of three independent experiments and the error bar indicates the standard deviation.
**Figure 6****:** Comparison of the hydrolytic activity of β-glucosidases from *Y. lipolytica* JMY1212. Bgl1-His on (a) pNP-derived substrates, and (b) natural glycosyl substrates with different β-configurations; Bgl2 on (c) pNP-derived substrates, and (d) natural glycosyl substrates with different β-configurations.

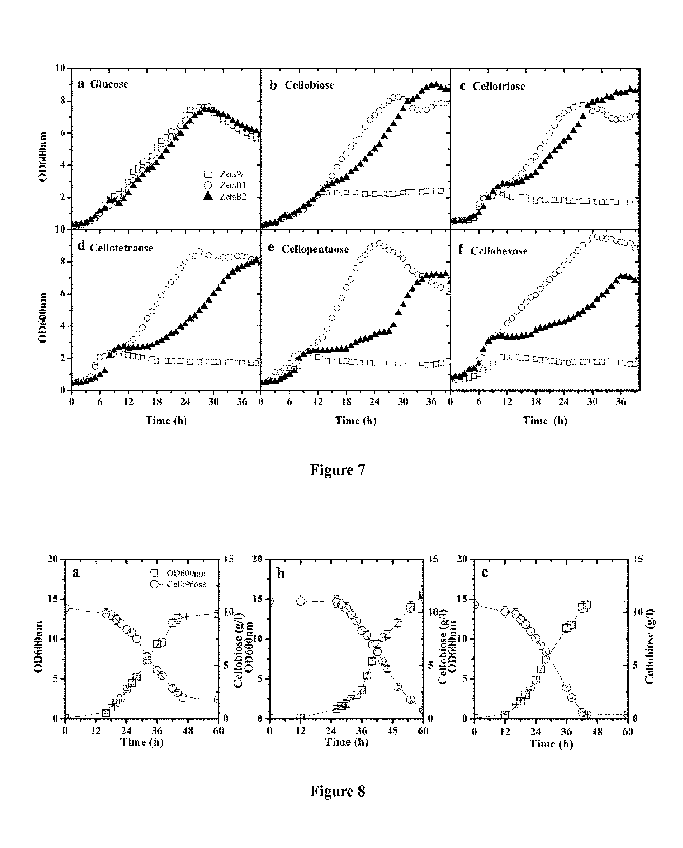

**Figure 7****:** Comparison of *Y. lipolytica* ZetaW (control), ZetaB1 (*P_{TEF}-BGL1*), ZetaB2 (*P_{TEF}-BGL2*) during aerobic growth on 5 g/L (a) glucose, (b) cellobiose, (c) cellotriose, (d) cellotetraose, (e) cellopentaose and (f) cellohexaose as carbon and energy source. Shown is OD₆₀₀ₙₘ, optical density at 600 nm, versus time. Each data point represents the mean of five independent experiments and the standard deviation is less than 5%.
**Figure 8****:** Comparison of *Y. lipolytica* (a) ZetaB1 (*P_{TEF}-BGL1*), (b) ZetaB2 (*P_{TEF}-BGL2*) and (c) Zeta-B12 (*P_{TEF}-BGL1*, *P_{TEF}-BGL2*) during aerobic growth on 10 g/L cellobiose. Shown are OD₆₀₀ₙₘ, optical density at 600 nm, and cellobiose concentration versus time. Each data point represents the mean of five independent experiments and the error bar indicates the standard deviation.

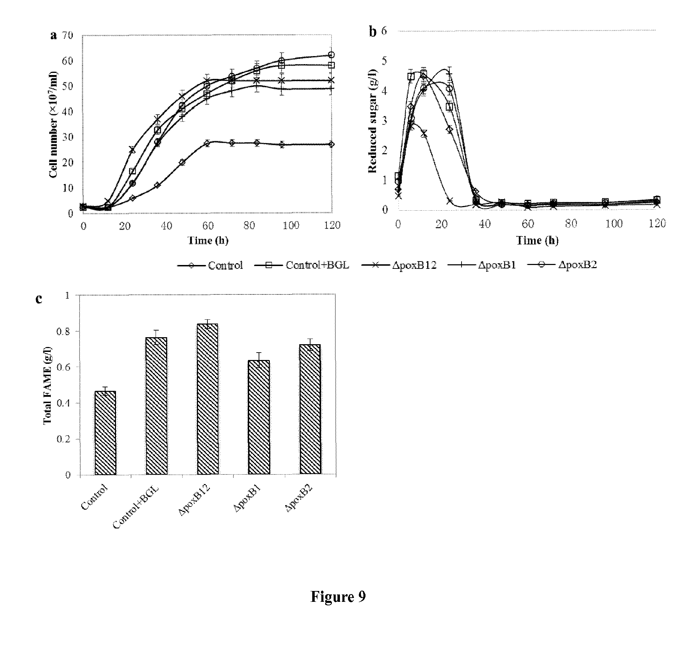

**Figure 9****:** Growth and lipid production on cellulose medium of *Y. lipolytica* strains. Growth during SSF on 50 g/L cellulose supplemented with Celluclast 1.5L. (a) growth expressed as cell number versus time; (b) the concentration of reduced sugar versus time; (c) lipid content at 60h. Strains are *Y. lipolytica* Δ*pox*B1 (*P_{TEF}-BGL1*), Δ*pox*B2 (*P_{TEF}*-*BGL2*) and Δ*pox*B12 (*P_{TEF}-BGL1*, *P_{TEF}-BGL2*) and Δ*pox*W (wild type) under the same condition without (control) or with (control+BGL) extra β-glucosidase (Novozyme 188). Each data point represents the mean of five independent experiments and the error bar indicates the standard deviation.

### EXAMPLE: CELLOBIOSE-DEGRADING ABILITY IN YARROWIA LIPOLYTICA STRAIN USING ENDOGENOUS GENE ACTIVATION

### 1. Materials and Methods

### 1.1. Strains and media

The genotypes of the microbial strains used in the present study are summarized in Table 1 below.

**Table 1: Microbial strains used in the present study**

| Strains | Relevant genotype | Source of reference |
|---|---|---|
| *E. coli* DH5 | Φ80dlacZΔm15, *recA1, endA1*, *gyrA96*, *thi-1*, *hsdR17* (rk⁻, mk⁺), *supE44*, *relA1*, *deoR*, Δ(*lacZY*A-argF) U169 | Invitrogen |
| *Y. lipolytica* JMY1212 (Zeta) | *MATA*, *ura3*-*302, leu2-270-LEU2-zeta*, *xpr2-322* Δ*lip2*, Δ*lip7*, Δ*lip8* | Bordes *et al*., 2007. |
| *Y. lipolitica* Δ*pox* JMY1233 | *MATA*, *leu2-270*, *ura3-302*, *xpr2-322*, *pox1-6*Δ | Beopoulos *et al*., 2008. |
| ZetaW | *MATA*, *xpr2-322*, Δ*lip2*, Δ*lip7*, Δ*lip8* | This study |
| ZetaB1 | *P_{TEF}-BGL1* | This study |
| ZetaB2 | *P_{TEF}-BGL2* | This study |
| ZetaB12 | *P_{TEF}-BGL1, P_{TEF}-BGL2* | This study |
| Δ*pox*W | *MATA*, *xpr2-322*, *pox1-6*Δ | This study |
| Δ*pox*B1 | *P_{TEF}-BGL1* | This study |
| Δ*pox*B2 | *P_{TEF}-BGL2* | This study |
| Δ*pox*B12 | *P_{TEF}-BGL1*, *P_{TEF}-BGL2* | This study |

*Escherichia coli* DH5 was purchased from Invitrogen (Paisley, UK) and used for plasmid construction. The *Y. lipolytica* strains were routinely cultivated in a medium composed of 1% w/v yeast extract, 1% w/v Bacto peptone, and 1% w/v glucose (YPD), solid media contained 1.5% agar. Transformants were selected on solid YNB medium (0.17% w/v YNB, 1% glucose or cellobiose w/v, 0.5% w/v ammonium chloride, with (for Ura⁺) or without (for Leu⁺) 0.2% w/v casamino acids and 50 mM sodium-potassium phosphate buffer, pH 6.8), supplemented with uracil (440 mg/L) or leucine (440 mg/L) depending on the auxotrophic requirements. The detection of β-glucosidase activity in solid YNBcasa medium was achieved by incorporating 1.0 mM *p*-nitrophenyl-β-D-glucoside (*p*NPGlc) (Guo *et al*., 2011). For β-glucosidase characterization, enzymes were produced in YTD medium (1% w/v yeast extract, 2% w/v tryptone, 5% w/v glucose and 100 mM phosphate buffer, pH 6.8). To compare the efficiency of recombinant β-glucosidase to degrade cellobiose and cellodextrin with respect to cell growth, yeasts were aerobically cultivated in YNBcasa medium, containing 5 g/L cellobiose or cello-oligosaccharides (C3-C6), and defined medium containing vitamins, trace elements (Verduyn *et al*., 1992) and salts, including 3.5 g/L (NH₄)₂SO₄, 3.0 g/L K₂HPO₄, 3.0 g/L NaH₂PO₄ and 1.0 g/L MgSO₄•7H₂O with 10 g/L cellobiose. For lipid production using cellulose as the carbon source, *Y. lipolytica* strains were grown in defined media supplemented with 50 g/L Avicel PH-101.

### 1.2. Plasmid constructions

The plasmids constructed in the present study are summarized in Table 2, and all primers are listed in Table 3 below.

**Table 2: Plasmids used or created in the present study**

| Plasmids | Description | Source of reference |
|---|---|---|
| JMP62UraTEF | *URA3*, *TEF_{P}-XPR_{T}* | Haddouche *et al*., 2011 |
| JMP62LeuTEF | *LEU2*, *TEF_{P}-XPR_{T}* | Nicaud *et al*., 2002 |
| JMP62UraTB1 | *URA3*, *TEF_{P}-BGL1-XPR_{T}* | This study |
| JMP62UraTB2 | *URA3*, *TEF_{P}-BGL2-XPR_{T}* | This study |
| JMP62LeuTB2 | *LEU2*, *TEF_{P}-BGL2-XPR_{T}* | This study |
| JMP62UraTB12 | *URA3*, *TEF_{P}-BGL1-XPR_{T}*, *TEF_{P}-BGL2-XPR_{T}* | This study |

**Table 3: The sequences of the oligonucleotide primers used in this study**

| Primer names | Sequence (5'-3') Restriction sites are italic/underlined | Restriction sites | SEQ ID NO: |
|---|---|---|---|
| FA1 | CG^aGGATCCCGCGATGATCTTCTCTCTGCAACTACT AC | *Bam*HI | 16 |
| RB1 | CGCCTAGGCTACAAAGTGAAAGTCTCACATAGC | *Avr*II | 17 |
| FA2 | CCCAAGCTTGGGTTTGGAGGGGGTGAAAAA | *Hind*III | 18 |
| RB2 | CCCAAGCTTGGGCTAAAGACCTAACCAATTCTTAG TCT | *Hind*III | 19 |
| FA3 | CGGGATCCCGCGATGATTGCAAAAATACCCC | *Bam*HI | 20 |
| RB3 | CGCCTAGGCTACTGGAGAGTAAAGGACTCG | *Avr*II | 21 |
| FA4 | CGGGATCCCGCGATGCTCGCATTCGTCCTAC | *Bam*HI | 22 |
| RB4 | CGGGATCCCGCTACTTGAGAGTGAAGCTGGTG | *Bam*HI | 23 |
| FA5 | CGGGATCCCGCGATGGCTCCACCCCCGCCTCCT | *Bam*HI | 24 |
| RB5 | CGCCTAGGTTAAGCAATCGTGATGCGACCAAGG | *Avr*II | 25 |
| FA6 | CGCCTAGGCGCGATGGAGGAATTATCGGAGGC | *Avr*II | 26 |
| RB6 | CGCCTAGGCTACCGGCTGAACTTCTCTTC | *Avr*II | 27 |
| RB-His1 | ᵇCGCCTAGGTTAATGATGGTGATGATGGTGGCTGCCG CGCGGCACCAGCCTAGGCAAAGTGAAAGTCTCA | | 28 |
| RB-His2 | CCCAAGCTTGGGTTAATGATGGTGATGATGGTGGCTG CCGCGCGGCACCAGCCTAGGAAGACCTAACCAATT CTTA | | 29 |
| RB-His3 | CGCCTAGGTTAATGATGGTGATGATGGTGGCTGCCGC GCGGCACCAGCCTAGGCTGGAGAGTAAAGGA | | 30 |
| RB-His4 | CGGGATCCCGTTAATGATGGTGATGATGGTGGCTGCC GCGCGGCACCAGCCTAGGCTTGAGAGTGAAGCT | | 31 |
| RB-His5 | CGCCTAGGTTAATGATGGTGATGATGGTGGCTGCCGC GCGGCACCAGCCTAGGAGCAATCGTGATGC | | 32 |
| RB-His6 | CGCCTAGGTTAATGATGGTGATGATGGTGGCTGCCGC GCGGCACCAGCCTAGGCTGAACTTCTCTTCC | | 33 |

| | | | |
|---|---|---|---|
| ^{a} restriction site with corresponding restriction enzyme. ^{b} His-tag introduced into the corresponding genes. | | | |

Briefly, these vectors contain the *Y. lipolytica TEF* promoter and either the *URA3ex* or *LEU2ex* excisable selection markers, which are flanked by *loxP* sites and a Zeta fragment that serves as the homologous integration site (Fickers *et al.,* 2003). Regarding β-glucosidases, six putative gene candidates (Sequences YALI0F16027g, YALI0F01672g, YALI0D18381g, YALI0B14289g, YALI0B14333g, YALI0E20185g available at Genome Resources from Yeast Chromosomes: http://gryc.inra.fr/) were identified (See Table 4 below).

**Table 4: Six putative β-glucosidase coding genes identified by the conserved glycosyl hydrolase family 3 N and/or 3C terminal domain.**

| Gene Locus_tag | Similar to | GenBank Accession numbers | Identities | *Signal Peptide |
|---|---|---|---|---|
| YALI0B14289g | *Saccharomycopsis fibuligera* Beta-glucosidase 1 | AAA34314.1 | 50.42% | Identified |
| YALI0B14333g | *Saccharomycopsis fibuligera* Beta-glucosidase 2 | AAA34315.1 | 45.22% | - |
| YALI0D18381g | *Talaromyces emersonii* Beta-glucosidase | AAL34084.2 | 27.20% | Identified |
| YALI0E20185g | *Candida albicans* Beta-glucosidase | XP_716473.1 | 26.99% | - |
| YALI0F01672g | *Kluyveromyces marxianus* Beta-glucosidase | ACY95404.1 | 50.56% | - |
| YALI0F16027g | *Saccharomycopsis fibuligera* Beta-glucosidase 2 | AAA34315.1 | 49.5% | Identified |

| | | | | |
|---|---|---|---|---|
| *Identification of signal peptide is done by SignalP 4.1 | | | | |

For the expression of wild-type and His6-tagged proteins, the genes were amplified by PCR using FA (1-6) as forward primers and RB (1-6) or RB-His (1-6) as reverse primers, respectively. The PCR fragments were digested using either *Bam*HI/*Av*II, or *Hind*III/*Avr*II, and inserted into the plasmid JMP62 UraTEF at the corresponding sites.

After construction, all expression vectors were verified by DNA sequencing (GATC Biotech, Konstanz, Germany). For *Y. lipolytica* transformation, vectors were digested using *Not*I, thus generating a linear DNA with Zeta sequences at both extremities, and purified. Then the linear DNA fragments were introduced into the Zeta docking platform of *Y. lipolytica* JMY1212 Zeta, or randomly into the genome of Δ*pox* strain using the lithium acetate method (Duquesne *et al.,* 2012). Transformants were tested for β-glucosidase activity on YNB glucose plate containing *p*NPGlc and for growth on cellobiose using solid YNB cellobiose plates. Clones displaying both activities were retained for further analysis.

### 1.3. Measurement of enzyme activity

β-Glucosidase activity was measured by quantifying the release of *p*NP (*p*-nitrophenol) from *p*NPGlc as described previously (Guo *et al.,* 2011). One unit of *p*NPGlcase activity was defined as the amount of enzyme required to release 1 µmol *p*NP per min. Cellobiose phosphorylase activity was assayed by measuring the formation of Glc-1P from cellobiose as described previously (Reichenbecher *et al.,* 1997). One unit of activity (U) was defined as the amount of enzyme required to release 1 µmol Glc-1P per min. All protein concentrations were measured using the Bradford method and bovine serum albumin as a standard (Bradford, 1976).

### 1.4. Western Blot Analysis

Western blotting of proteins was performed as described by Duquesne *et al.* (2014). Crude supernatant and cell-free extracts of *Y. lipolytica* JMY1212 expressing putative β-glucosidases fused with the His6 tag were concentrated 10-fold using an ultra-centrifugation filter unit (Amicon® Ultra-4 10 kDa cut-off, Merk Millipore, Bedford, MA, USA). Blots were sequentially treated with mouse non position-specific His-Tag antibody 1:2500 (THE™ from Genscript, Piscataway, NJ, US) and the alkaline phosphatase-conjugated goat anti-mouse IgG.

### 1.5. Subcellular fractionation and enzyme localization

Fractionation of yeast cells was carried out as described by Cummings and Fowler (1996), with slight modifications. Briefly, yeasts were cultivated until a cellular density of 6×10⁷ cells/mL was reached. Then, to quantify total β-glucosidase activity, a 50 mL sample was taken and subjected to centrifugation at 8,000×g for 5 min at 4°C thus isolating a cell pellet and supernatant. The cell pellet was disrupted in Tris-HCl buffer (50 mM, pH 7.4, 3 mM EDTA and 0.5 mM PMSF) using a MP FastPrep-24 Instrument (MP Biomedicals Inc.). β-Glucosidase activity in both the cell lysate and the supernatant was determined as described earlier in order to estimate total β-glucosidase activity. Using a second 50 mL yeast culture, a cell pellet containing approximately 2×10⁸ cells/mL was obtained by centrifugation and then treated with zymolyase 100T at 10 mg/mL (Seikagaku corp coger) in 15 mL of sorbitol buffer (1 M sorbitol, 50 mM Tris-HCl, pH 7.4, 2 mM dithiothreitol, 10 mM MgCl₂, 20 mM-sodium azide, 0.5 mM PMSF) at 30°C with gentle shaking. Protoplast formation was monitored using a microscope until ≥99% of the cells was lysed when SDS was added (1 % SDS w/v). The solid protoplast fraction was then separated from the supernatant by centrifugation (1000 rpm for 5 min at 4°C) and the latter was designated as the periplasmic fraction. The protoplasts were re-suspended in Tris-HCl buffer (50 mM Tris-HCl, pH 7.4) and disrupted by vortex in the presence of glass beads (0.4-0.45 mm). The homogenate was centrifuged (20,000× g for 2 h at 4°C) and the supernatant and solid fractions were designated as the cytoplasmic and membrane fraction respectively. Prior to enzyme assays, the membrane fraction was suspended in citrate buffer.

### 1.6. Purification of B-glucosidases

*Y. lipolytica* JMY1212 overproducing Bgl1-His6 and Bgl2 were grown in 200 mL YTD medium at 130 rpm, 28°C for 36 h before centrifugation at 8,000× g for 5 min. For purification of Bgl1-His6, the cell pellet was washed, suspended in 50 mL phosphate buffer (50 mM, pH 7.4) and homogenized over a 3-min period using a MP FastPrep-24 Instrument. After centrifugation (8,000×g for 5 min at 4°C), the supernatant was applied to 2 mL of TALON Metal Affinity Resin (Clontech, Takara-Bio, Kyoto, Japan) and protein was eluted using imidazole buffer according to the manufacturer's instructions.

For purification of Bgl2, the culture supernatant was concentrated 5-fold using an Amicon® Ultra-4 Centrifugal Filter Unit with 30kDa cut-off (Merk Millipore, Bedford, MA, USA). The concentrated sample was then loaded onto a Q Sepharose™ High Performance column (Hiload, 1.6×10 cm, Pharmacia Biotech), equilibrated with Tris-buffer (20 mM, pH8.0). The column was washed first with equilibration buffer (2 bed volumes) before applying a linear gradient of 0-1.0 M NaCl in Tris-buffer (20 mM, pH7.4) at a flow rate of 1.0 mL/min (Pharmacia Biotech ÄKTA). Eluted fractions were collected and assayed for β-glucosidase activity. All fractions displaying activity were pooled, desalted and concentrated using an Amicon ultra-filtration unit equipped with a PM-10 membrane (Millipore), before being applied to a Superdex 200 column (1.0×30 cm, Pharmacia Biotech) equilibrated in Tris-sodium buffer (20 mM Tris-HCl, 150 mM NaCl, pH 7.4). Protein species were separated at a flow rate of 0.5 mL/min. Fractions were collected and analyzed by SDS-PAGE in order to ascertain purity and estimate the approximate molecular weights of Bgl1-His6 and Bgl2. All fractions satisfying the purity criterion (>95% purity) were pooled and retained for further work.

### 1.6. Deglycosylation and N-terminal amino acid sequencing

Purified Bgl1-His6 and Bgl2 were treated with endoglycosidase H (New England Biolabs, Beverly, MA, USA) according to the manufacturer's instructions. After deglycosylation, the protein species displaying *Mᵣ* (relative molecular mass) closest to those of the theoretical *Mᵣ* (predicted using Protparam, http://web.expasy.org/protparam/) of Bgl1-His6 and Bgl2 were excised and submitted to N-terminal amino acid sequencing (PISSARO platform, Rouen, France).

### 1.7. Physicochemical characteristics of B-glucosidases

Optimal temperatures and pH for the activity of Bgl1 and Bgl2 were determined using *p*NPGlc as the substrate. Assays were either performed at pH 5.0 and various temperatures (30-70°C), or at 30°C in variable pH conditions (2.0 to 8.0) using either 50 mM glycine-HCl (pH 2.0), 50 mM citrate/acetate (pH 3.0-7.2), or potassium phosphate (pH 7.0-8.2) buffer. When the temperature was varied, the pH of the citrate buffer was adjusted accordingly. Stability of Bgl1 and Bgl2 depending on pH and temperature was analyzed as follows: enzymes were incubated at 30°C for up to 2h at various pH values (2.0 to 8.0), or at various temperatures (30-70°C) for up to 2h in 50 mM citrate buffer, pH 5.0. Residual glucosidase activity was then assayed at 30°C in 50 mM citrate buffer, pH 5.0.

### 1.8. Substrate specificity and enzyme kinetics

The substrate specificity of Bgl1-His6 and Bgl2 was investigated by assaying for activity on the aryl-glycosides *p*NP-β-D-glucopyranoside, *p*NP-α-D-glucopyranoside, *p*NP-β-D-galactopyranoside, pNP-β-D-xylopyranoside and pNP-β-D-cellobioside, and on the oligosaccharides cellobiose, cellotriose, cellotetraose, cellopentaose, cellohexaose, sophorose, laminaribiose, gentiobiose, methylglucoside and octylglucoside. When using aryl-substrates, the standard assay method was employed, simply replacing *p*NPGlc by another substrate as appropriate. For oligosaccharides, the release of glucose was quantified using an enzyme kit (D-Fructose/D-Glucose Assay Kit, liquid stable, Megazyme). To study the Michaelis-Menten parameters *K*_{M}, *Vₘₐₓ* and *k_{cat}*, Bgl1 (0.120 nM) or Bgl2 (0.13 nM) were added to reaction mixtures containing different substrate concentrations: 0.25-5 mM cellobiose, 0.25-5 mM cellotriose, 0.25-5 mM cellotetraose, 0.25-5 mM cellopentaose, 0.25-5 mM cellohexaose, 0.2-4 mM sophorose, 0.1-2 mM laminaribiose, 0.1-2 mM gentiobiose, 0.5-20 mM methylglucoside and 0.2-4 mM octylglucoside. Initial rates were fitted to the Michaelis-Menten kinetic equation using a nonlinear regression (SigmaPlot 10) to extract the apparent *K*_{M} and *k_{cat}* (Segel, 1993).

### 1.9. Yeast growth and lipid production

Yeast growth on cellobiose and cellodextrins was performed in a 40-well microplate. A single colony from a fresh YPD plate was transferred into 5 mL of defined medium containing 10g/L of glucose and pre-cultured until the mid-exponential phase. The cells were then harvested, washed, suspended in sterile water and used to inoculate 200 µL YNBcasa media containing 5 g/L cellobiose or cellodextrins in the microplate, achieving an initial OD₆₀₀ of 0.1. This culture was grown in a microplate reader (Spectrostar Omega, BMG Labtech, Germany) at 30°C with continuous shaking (150 rpm) and automatic OD₆₀₀ recording.

Similarly, for lipid production a fresh yeast culture in exponential phase was used to inoculate 50 mL defined medium containing 50 g/L Avicel in Erlenmeyer flasks, achieving an initial OD₆₀₀ of 1.0. Celluclast 1.5L (60 FPU/mL, gift from Novozymes, Denmark) was added (7.5 U/g cellulose) and growth was pursued for 5 days (30°C, 150 rpm). Samples were taken at regular intervals to determine concentrations of biomass, glucose, cellobiose and citric acid. In parallel, two control experiments were conducted under the same conditions, with or without the addition of extra β-glucosidase (810 IU/mL Novozyme 188, gift from Novozyme, Denmark) at 12.0 IU/g cellulose as recommended (Lan et al., 2013).

### 1.10. Analysis of product formation and determination of dry cell weight

To determine the concentration of substrates and extracellular metabolites, three aliquots (1.5 mL each) of cultures were rapidly frozen in liquid nitrogen and then thawed on ice before centrifugation (8,000×g for 5 min at 4°C) to recover supernatants for analysis. Glucose, cellobiose and citric acid were measured using an Aminex HPX87-H column (Bio-Rad Laboratories, Germany), operating at 50°C using a mobile phase (5 mM H₂SO₄) flowing at a rate of 0.5 mL/min. Glucose and cellobiose were detected using a Shodex RI-101 refractive index detector (Showa Denko, New York, NY), while citric acid was detected using an UV detector at 210 nm (Dionex, Sunnyvale, CA).

To determine the dry cell weight, three aliquots (5 mL each) of cultures were filtered through pre-weighed PES filters (0.45 µm; Sartorius Biolab, Germany). The biomass retained by the filters was washed, dried in a microwave oven at 150 W for 15 min, and then placed in a desiccator before weighing. The biomass yield was calculated as the ratio of the amount of biomass obtained divided by the amount of carbon source consumed.

Lipids were extracted from freeze-dried cells (∼10 mg) and methylated as described previously (Browse *et al.,* 1986). During the lipid extraction, C17:0 (Sigma) (50µg) was added as the internal standard and fatty acid methyl esters (FAMEs) were analyzed by gas chromatography (6890N Network GC System, Agilent, USA). The measurements were performed in a split mode (1 µL at 250°C), with helium as the carrier gas (2 mL/min). FAMEs were separated on a HP-5 GC column (30 m×0.32 mm I.D., 0.5-µm film thickness, Agilent, USA). The temperature program was 120°C, ramped to 180°C (10°C/min) for 6 min, 183°C (0.33°C/min) for 9 min, and 250°C (15°C/min) for 5 min. Detection was performed using a flame ionization detector (FID) at 270°C (2.0 pA). FAMEs were quantified by comparing their profiles with that of standards of known concentration.

To measure the intracellular concentrations of Glc-1P, glycogen and trehalose, aliquots (1.5 mL each) of the above cultures were immediately frozen using liquid nitrogen and then stored at -80 °C. Before analysis, samples were thawed on ice and cells were collected by centrifugation (8,000×g for 5 min at 4°C) and disrupted using glass beads as described above. Cellular Glc-1P was determined using an enzymatic kit (G1P Colorimetric Assay Kit, Sigma), following the supplier's instructions. Cellular glycogen and trehalose concentrations were measured as described previously (Parrou and François, 1997). Free glucose was quantified using the D-Fructose/D-Glucose Assay Kit.

### 2. Results

### 2.1. Identification of genes encoding active β-glucosidases in Y. lipolytica

Analysis of the *Y. lipolytica* genome using BLAST revealed the presence of six sequences that were identified as putative family GH3 β-glucosidases (See Table 4 above) on the basis of high amino sequence identity with other yeast β-glucosidases. However, in the absence of biochemical data it was impossible to assert at this stage that these sequences actually encode β-glucosidases, since family GH3 contains glycoside hydrolases that display other specificities and also because *Y. lipolytica* does not grow on cellobiose and has not been found to express a detectable level of β-glucosidase activity (See Figure 1). In this respect, it was observed that overexpression of *BGL1* (YALI0F16027g) or *BGL2* (YALI0B14289g) in *Y. lipolytica* (strains ZetaB1 and ZetaB2 respectively) conferred the ability to grow on solid medium containing cellobiose as the sole carbon source. Additionally, when these recombinant strains were grown on YNB-*p*NPGlc plates, yellow halos surrounding the colonies were clearly visualized, indicating β-glucosidase activity (Figure 1). Finally, after growth in liquid YTD medium, β-glucosidase activity could be measured in the cell extract of ZetaB1 (3.2±0.2 IU/mg) and in the culture supernatant of ZetaB2 (2.6±0.1 U/mL), while much lower activities were measured in the culture supernatant of ZetaB1 (0.33±0.02 U/mL) and in the cell extract of ZetaB2 (0.42±0.01 IU/mg).

To further investigate the production of β-glucosidases, western blot analysis was carried out using anti-His6 antibodies. This revealed that only Bgl1 was detectable in both the culture supernatant and cell extract (Figure 2a, b), consistent with the fact that expression of Bgl2-His6 failed to reveal any detectable β-glucosidase activity, although expression of the native *BGL2* sequence was successful.

### 2.2. Localization of β-glucosidases in ZetaB1 and ZetaB2

To determine the localization of Bgl1 and Bgl2, yeast cells expressing these enzymes were fractionated, generating on one hand extracellular samples (culture supernatant), and on the other cell-associated periplasmic, cytoplasmic and membrane fractions. Measurement of the β-glucosidase activities in each of these fractions revealed that Bgl1 was primarily localized in the periplasm, while Bgl2 was mainly in the supernatant (Table 5 below).

**Table 5: Distribution of β-glucosidase activity in recombinant strains ZetaB1 and ZetaB2**

| Fraction | Relative enzyme activity^{a} | |
|---|---|---|
| | Bgl1 (%) | Bgl2 (%) |
| Total | 100 | 100 |
| Growth medium | 2.3±0.4 | 79.6±1.2 |
| Periplasm | 60.7±1.0 | 25.8±1.0 |
| Cytoplasm | 30.0±0.5 | 4.8±0.8 |
| Membrane | 8.1±0.7 | 3.7±0.3 |

| | | |
|---|---|---|
| ^{a} triplicate experiments. ± the standard deviation. | | |

### Activity was assayed with pNPGlc.

Accounting for the limits of the experimental methods employed, Bgl1 was also quite present in the cytoplasmic fraction and the presence of Bgl2 in the periplasm was also significant. Overall, these data are consistent with the conclusion that Bgl1 is probably localized in the periplasmic space, while Bgl2 is secreted to the culture medium.

### 2.3. Production, purification and characterization of Bgl1 and Bgl2

Production of Bgl1-His6 and native Bgl2 was achieved by growing the appropriate *Y. lipolytica* strains on YTD in aerobic cultivations, with expression of both enzymes increasing until complete depletion of glucose was reached (36 h).

Regarding purification of Bgl1-His6, yeast cells arising from a 200-mL culture volume yielded approximately 550 U (170 mg) of enzyme in the crude cell extract. However, after purification only 17 % of Bgl1 was recovered (Table 6 below).

**Table 6: Purification of intracellular Bgl1-His6 and extracellular Bgl2 produced by Y. lipolytica overexpressing strains.**

| Enzyme and purification method | | Total protein (mg) | Total activity (U) | Specific activity (U/mg) | Fold purification | Yield (%) Recovery |
|---|---|---|---|---|---|---|
| Bgl1-His6 | Filtrate | 169.7 | 543.0 | 3.2 | - | 100 |
| | TALON His-tag^{a} | 0.9 | 92.5 | 102.8 | 32.1 | 17.0 |
| Bgl2 | Culture filtrate | 2302.5 | 530.2 | 0.23 | - | 100 |
| | Ultra filtration | 1986.4 | 510.5 | 0.26 | 1.1 | 96.3 |
| | Ion exchange | 235.3 | 478.5 | 2.0 | 7.7 | 90.2 |
| | Gel filtration | 1.8 | 46.4 | 25.8 | 112.2 | 8.8 |

| | | | | | | |
|---|---|---|---|---|---|---|
| ^{a}Specific activity was tested on pNPGlc | | | | | | |

In the case of Bgl2, a two-step protocol using anion exchange chromatography and gel filtration allowed its purification to near homogeneity, but led to significant loss of protein (8.8% recovery). SDS-PAGE analysis of the two purified protein samples revealed that the *M*ᵣ of Bgl1 was slightly higher than expected (theoretical *M*ᵣ= 92.1 kDa) (Figure 2c), while that of Bgl2 was significantly higher (> 250 kDa) than the expected *M*ᵣ of 94.6 kDa with presence of the potential signal peptide (Figure 2d). To understand this anomaly, the amino acid sequence of Bgl2 was analyzed using the glycosylation predictor GlycoEP (http://www.imtech.res.in/raghava/glycoep/; Chauhan *et al.,* 2013). This revealed that Bgl2 harbors 18 potential N-glycosylation sites. Therefore, to investigate the actual glycosylation state of recombinant Bgl2, the purified protein was deglycosylated using endoglycosidase H treatment. After deglycosylation and SDS-PAGE analysis, the *M*ᵣ of the recombinant Bgl2 was estimated to be approximately 95 kDa, consistent with the theoretical *Mr* (Figure 2d). Finally, N-terminal amino acid sequence analysis of Bgl1 and Bgl2 confirmed the identity of the two proteins and revealed the signal peptide of the two Bgls (SEQ ID NO: 4 and 6 respectively).

Preliminary characterization of Bgl1 and Bgl2 using pNPGlc as the substrate revealed that Bgl1 was 5-fold more active (102.8 U/mg) on this substrate than Bgl2 (25.8 U/mg). The activity of Bgl1 was highest at approximately pH 4.5 and 45°C, and was stable in the pH range of 4.0-5.0 and below 40°C. Regarding Bgl2, it was found to display highest activity at pH 4.0 and 50°C, and was stable in the pH range of 3.5-7.0 and below 50°C (Figures 3 and 4). It is noteworthy that deglycosylation of Bgl2 led to a 60% decrease in specific activity, which was probably due to its instability at 40°C (Figure 5).

### 2.4. Substrate specificity and kinetic parameters of Bgl1 and Bgl2

The substrate specificity of the purified β-glucosidases was examined using different substrates displaying α and β configurations. The results showed that both β-glucosidases were maximally active against *p*NPGlc (Figure 6). However, using activity on *p*NPGlc as the benchmark, it is noteworthy that both enzymes were active on *p*NP-β-D-cellobioside (Bgl1, 24% and Bgl2, 27%), but only Bgl1 displayed significant activity (10%) on *p*NP-β-D-xylopyranoside. Neither enzyme displayed activity on *p*NP-β-D-galactopyranoside and *p*NP-α-D-glucopyranoside.

When the activity of Bgl1 and Bgl2 on cellobiose was compared with that on other oligosaccharides, it was found that both enzymes displayed highest activity on laminaribiose (β-1, 3-linkage), followed by gentiobiose (β-1, 6-linkage), octylglucoside, sophorose (β-1, 2-linkage), cello-oligosaccharides (C3-C6) and cellobiose (β-1, 4-linkage). It is noteworthy that the hydrolytic activity of Bgl1 was less dependent on the chain length of cello-oligosaccharides, while hydrolytic activity of Bgl2 increased as the length of cello-oligosaccharides increased. Both enzymes recognized methylglucoside as substrate, but the hydrolytic activities were low compared with the other substrates (Figure 6), indicating that correct occupation of subsite +1 is important for catalysis.

The determination of the apparent kinetic parameters of reactions catalyzed by Bgl1 and 2 and containing various glucosyl disaccharides and cello-oligosaccharides revealed that the values of *K*_{M}(app) and *k_{cat}*/*K*_{M} for Bgl2-catalyzed reactions increased as a function of degree of polymerization (DP) of the cello-oligosaccharides (see Table 7 below).

**Table 7: Kinetic parameters of Y. lipolytica Bgls for various glycoside-substrates^{a}**

| Substrate | Linkage | Bgl1 | | | Bgl2 | | |
|---|---|---|---|---|---|---|---|
| | | *K*_{M} (mM) | *k_{cat}* (s⁻¹) | *k_{cat}*/*K*_{M} (mM⁻¹s⁻¹) | *K*_{M} (mM) | *k_{cat}* (s⁻¹) | *k_{cat}*/*K*_{M} (mM⁻¹s⁻¹) |
| Cellobiose | Glc×2, β-1, 4 | 0.26 | 21.1 | 81.1 | 0.79 | 5.1 | 6.5 |
| Cellotriose | Glc×3, β-1, 4 | 0.43 | 20.5 | 47.7 | 0.99 | 9.5 | 9.6 |
| Cellotetraose | Glc×4, β-1, 4 | 1.89 | 30.9 | 16.3 | 1.86 | 20.6 | 11 |
| Cellopentaose | Glc×5, β-1, 4 | 2.18 | 29.5 | 13.5 | 2.24 | 27.5 | 12.3 |
| Cellohexaose | Glc×6, β-1, 4 | 3.01 | 31.5 | 10.5 | 2.37 | 30.5 | 12.9 |
| Sophorose | Glc×2, β-1, 2 | 2.25 | 28.4 | 14.8 | 2.4 | 41.2 | 17.2 |
| Laminaribiose | Glc×2, β-1, 3 | 0.68 | 75.6 | 110.7 | 0.89 | 211.1 | 237.2 |
| Gentiobiose | Glc×2, β-1, 6 | 1.16 | 43.6 | 37.6 | 1.84 | 186.5 | 101.4 |
| Methylglucoside | C=1 | 15 | 15 | 1 | 6.23 | 34.1 | 5.5 |
| Octylglucoside | C=8 | 0.86 | 32.8 | 38.1 | 1.3 | 111.1 | 85.2 |

| | | | | | | | |
|---|---|---|---|---|---|---|---|
| ^{a}The mean values of three independent experiments are shown and the standard deviation is below 10%. Hydrolytic activities for the substrate were determined from the amount of released glucose and the kinetic parameters were calculated as described in Materials and Method. | | | | | | | |

In the case of Bgl1, increased DP was associated with increased *K*_{M}(app) values, but not *k_{cat}*/*K*_{M} values. Overall, considering the performance constant (*k_{cat}*/*K*_{M}), cellobiose and cellohexaose were the best substrates for Bgl1 and Bgl2 respectively. Additionally, the performance constant of Bgl1 measured on cellobiose was 12.5-fold higher than that describing Bgl2. Regarding other glucosyl substrates (*i.e.* those containing linkages other than β-1,4), both Bgls displayed the highest performance constants on laminaribiose. Nevertheless, comparison of the performance constants on each of the substrates revealed that Bgl2 is less regioselective, since the *k_{cat}*/*K*_{M} values were always lower in reactions catalyzed by Bgl1 (86% for sophorose, 47% for laminaribiose, 37% for gentiobiose, 18% for methylglucoside and 45% for octylglucoside). Finally, the lowest performance constants for both Bgls were measured for reactions containing methylglucoside.

### 2.5. Cellobiose and cello-oligosaccharide fermentation with Y. lipolytica recombinant strains

Yeast strains ZetaB1 expressing *BGL1* and ZetaB2 expressing *BGL2* were grown in micro cultivation plates under aerobic conditions in the presence of cellobiose or cellodextrins as sole carbon sources, using wild type *Y. lipolytica* ZetaW as the control. The maximum specific growth rates (µₘₐₓ) of the transformants on cellobiose were essentially the same as that of the control grown on glucose (Figure 7a , b). ZetaB1 grew faster than ZetaB2 on cellobiose and cellodextrins (Figure 7b-f), while the control was unable to grow on either of these substrates.

Further characterization of the recombinant strains in shake flask cultures showed that ZetaB1 consumed 8 g/L cellobiose over 48 h. However, upon further incubation, the remaining cellobiose (2 g/L) was not consumed (Figure 8). In contrast, ZetaB2 consumed all of the cellobiose (10 g/L) over 64 and 72 h respectively (Figure 8). Furthermore, ZetaB1 sustained a specific aerobic growth rate (µₘₐₓ) of 0.16 h⁻¹ (identical to that on glucose), whereas ZetaB2 exhibited a long lag phase on cellobiose after which two subsequent growth phases (µₘₐₓ values of 0.08 h⁻¹ and then 0.16 h⁻¹) were observed (Figure 8 and Table 8 below).

**Table 8: Comparison of growth and biomass yield of Y. lipolytica JMY1212 control and recombinant strains in aerobic cellobiose cultivation**

| Parameter | Control | ZetaB1 | ZetaB2 | ZetaB12 |
|---|---|---|---|---|
| µₘₐₓ (h⁻¹) on glucose | 0.15±0.01 | 0.16±0.01 | 0.16±0.01 | 0.16±0.01 |
| µₘₐₓ (h⁻¹) on cellobiose | N.A. | 0.16±0.01 | 0.15±0.01 | 0.16±0.01 |
| Y_{X/S} (DCW-g/g cello) | N.A. | 0.52±0.01 | 0.53±0.01 | 0.50±0.01 |
| Residue cellobiose 60h (%) | N.A. | 17.2±1.0 | 7.2.±0.1 | 1.0.±0.3 |

| | | | | |
|---|---|---|---|---|
| ± the standard deviation. N.A.= Not available | | | | |

In order to combine the advantages procured by the overexpression of *BGL1* and *BGL2* (*i.e.* faster growth rate and higher cellobiose utilization respectively), the two *BGL* sequences were cloned into JMY1212, thus yielding ZetaB12. During cultivation on cellobiose, the performance of ZetaB12 was the best among all the recombinant strains. It showed similar growth rate to that of ZetaB1 and consumed 10g/L of cellobiose within 40 h.

### 2.6. Characterization of cellulose-based lipid production by recombinant Y. lipolytica strains

A strategy to increase lipid accumulation was based on the disruption of the ß-oxidative metabolism, through the deletion of the 6 *POX* genes (*POX1* to *POX6*) that encode the peroxisomal acyl-coenzyme oxidases as has been done in Δ*pox* strain (Beopoulos *et al.,* 2008). To investigate whether the recombinant *Y. lipolytica* strains could be useful in a consolidated bioprocess for lipid production, recombinant *Y. lipolytica* Δ*pox*B1, Δ*pox*B2, Δ*pox*B12 and Δ*pox*PT were grown on cellulose in the presence of Celluclast 1.5L. Even though this cocktail is reputedly β-glucosidase-deficient, to avoid any problems (*i.e.* spurious results linked to the presence of β-glucosidase in Celluclast) the Celluclast loading was kept low (7.5 FPU/g cellulose), and control experiments containing the prototrophic *Y. lipolytica* Δ*pox*W strain grown in the presence of Celluclast 1.5L with or without β-glucosidase supplementation were performed. During the initial 6 h of cultivation an accumulation of reducing sugars was observed in all of the cultures, which was attributed to Celluclast 1.5L-mediated cellulose hydrolysis. However, further monitoring revealed that after 12-h growth, less reducing sugars were present in the *Y. lipolytica* Δ*pox*B12 culture (2.7 g/L) compared to the other cultures (Figure 9b). Moreover, this observation was correlated with continued yeast growth, whereas the growth of the other cultures stagnated over the same period (Figure 9b). After 60 h of cultivation, the growth of the Δ*pox*B12 reached a stationary phase. At this point the amount of FAMEs had reached 0.8 g/L (Figure 9c), but further growth did not result in an increase in cellular lipid content, reflecting a limitation of the available energy source. Besides the longer lag phase, the growth of Δ*pox*B1 and Δ*pox*B2 was similar to that of the control culture supplemented with β-glucosidase. Regarding the control culture, in the absence of β-glucosidase supplementation, growth ceased after 60 h and the cell density of the culture was approximately half that of the other cultures. Moreover, continuous addition of cellulases to the control culture did not procure any obvious increase in growth. When the control was supplemented with β-glucosidase, the amount of cellulose that remained unconsumed (25 g/L) was similar to that of cultures of the Δ*pox* strain expressing β-glucosidases after 5 days of growth and was less than that of the cultures with Δ*pox*PT (30 g/L).

### REFERENCES

André, A., et al., 2009. Eng. in Life Sci. 9:468-478.
Belancic, A., et al., 2003. J. Agric. Food Chem. 51:1453-1459.
Beopoulos, A., et al., 2008. Appl. Environ. Microbiol. 74:7779-7789.
Blazeck, J., 2011. Appl. Environ Microbiol. 77: 7905-7914.
Blazeck, J., 2013. Appl. Microbiol Biotechnol. 97:3037-3052.
Blazeck, J., et al., 2014. Nat. Commun. 5:3131.
Bordes, F., et al., 2007. J. Microbiol. Methods. 70:493-502.
Bradford, M.M., 1976. Anal. Biochem. 72:248-254.
Browse, J., et al., 1986. Anal. Biochem. 152:141-145.
Chauhan, J.S., et al., 2013. PLoS ONE. 8:e67008.
Cummings, C., Fowler, T., 1996. Curr. Genet. 29:227-233.
Daroit, D.J., et al., 2008. J. Microbiol. Biotechnol. 18:933-941.
Demirba 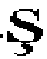 , A., 2003. Energy Convers Manage. 44:2093-2109.
Duff, S.J.B., et al., 1985. Biotechnol. Lett. 7:185-190.
Duquesne, S., et al., 2012. Methods Mol Biol. 861:301-312.
Easterling, E.R., et al., 2009. Bioresour. Technol. 100:356-361.
Fabre, E., et al., 1991. J Biol Chem., 266:3782-3790.
Fakas, S., et al., Appl Microbiol Biotechnol., 2006, 73:676-683.
Fickers, P., et al., 2003. J. Microbiol. Methods. 55:727-737.
Frank, K. and Sippl M.J., 2008. Bioinformatics. 24:2172-2176.
Gaillardin, C., et al., 1987, Curr Genet., 11:369-375.
Galas, E., Romanowska, I., 1996. Appl. Environ. Microbiol. 62:3165-3170.
Gasmi, N., 2011. Appl Microbiol Biotechnol. 89:109-119.
Groenewald, M., et al., 2014. Crit. Rev. Microbiol. 40:187-206.
González-Pombo, P., et al., 2008. Biotechnol. Lett. 30:1469-1475.
Guo, X., et al., J. Appl. Microbiol., 2000, 89:107-115.
Guo, Z.P., et al., 2011. Enzyme Microb. Technol. 49:105-112.
Gysler, C., et al., 1990, Biotechn Techn., 4:285-290.
Haddouche, R., et al., 2011. Appl. Microbiol. Biotechnol. 91: 1327-1340.
Hashimoto, Y., 1998. Protein Engineering. 11:75-77.
Hedfalk, K. (2012) 'Codon Optimisation for Heterologous Gene Expression in Yeast'. In Springer Protocols :Methods in Molecular Biology. Recombinant protein production in yeast :methods and protocols. Volume 866 pp. 47-55. Springer Eds.
Ito, H., et al., 1983, J Bacteriol., 153:163-168.
Klebe, R.J., et al., 1983, Gene, 25:333-341.
Koganesawa, N., et al., 2001, Protein Eng. 14:705-710.
La Grange, D.C., et al., 2010. Appl. Microbiol. Biotechnol. 87:1195-1208.
Lane, S., et al., 2014. Biotechnol Bioeng. In press.
Lazar Z., et al., 2014, Metab. Eng. 2689-99.
Le Dali, M.T., et al., 1994, Curr Genet., 26:38-44.
Leclerc, M., et al., 1987. Appl. Biochem. 9:410-422.
Lee, W.H., et al., 2013. J. Biotechnol. 167:316-322.
Lian, J., et al., 2014. Biotechnol. Bioeng. 111:1521-1531.
Lynd, L.R., et al., 2005. Curr. Opin. Biotechnol. 16:577-583.
Machida, M., et al., 1988. Appl. Environ. Microbiol. 54:3147-3155.
Madzak C., et al., 2000, J Mol Microbiol Biotechnol. 2:207-216.
Madzak, C., et al., 2004, J Biotechnol. 109:63-81.
Madzak, C. and Beckerich, J.M., 2013, Heterologous protein expression and secretion in Yarrowia lipolytica. In : Yarrowia lipolytica: Biotechnological Applications, Microbiology Monographs Vol. 25. Ed : Barth G, Springer, Heidelberg, Germany. pp. 1-76.
Michely, S., et al., 2013. PLoS One. 8:e63356.
Muller, S., et al., 1998, Yeast, 14:1267-1283.
Nan, H., et al., 2014. Appl. Microbiol. Biotechnol. 98:5757-5764.
Nicaud, J-M., et al., 2002. FEMS Yeast Res. 2:371-379.
Orr-Weaver, T.L., et al., 1981, Proc. Natl. Acad. Sci. USA, 78:6354-6358.
Papanikolaou, S., et al., Antonie van Leeuwenhoek, 2001, 80:215-224.
Papanikolaou, S., et al., J. Appl. Microbiol., 2002, 92:737-744.
Papanikolaou, S., et al., Bioresour. Technol., 2008, 99:2419-2428.
Papanikolaou, S., Aggelis, G., 2010. Eur. J. Lipid Sci. Techol. 112:639-654.
Parrou, J.L., François, J., 1997. Anal. Biochem. 1:186-188.
Pedersen, M., Meyer, A.S., 2010. N Biotechnol. 27:739-750.
Petersen, T.N., et al., 2011. N. Methods. 8:785-786.
Pignède, G., et al., 2000. J. Bacteriol. 182:2802-2810.
Ratledge C. (1994). Yeasts, moulds, algae and bacteria as sources of lipids. Technological advances in improved and alternative sources of lipids. B. S. Kamel, Kakuda, Y. London, Blackie academic and professional, 235-291.
Ratledge, C., 2005. Single cell oils for the 21th century, in:Cohen, R. (Eds.), Single cell oils. AOCS Press, Champaign, pp. 1-20.
Reichenbecher, M., et al., 1997. Eur. J. Biochem. 247:262-267.
Runguphan, W., Keasling, J.D., 2014. Metab. Eng. 21:103-113.
Shi, S., et al., 2012. Biotechnol. Biofuels. 5:7-16.
Sreekrishna, K., 1997. Gene. 190:55-62.
Stockton, D.C., et al., 1991. Biotechnol. Lett. 13:57-62.
Steen, E.J., et al., 2010. Nature. 463:559-562.
Sun, Y., Cheng, J., 2002. Bioresour. Technol. 83:1-11.
Tatusova, T.A. and Madden, T.L., 1999. FEMS Microbiol. Lett. 174:247-250.
Thevenieau, F., Nicaud, J-M.,2013. OCL. 20:1-8.
Tomme, P., et al., 1995. Adv. Microb. Physiol. 37:1-81.
Valle-Rodriguez, et al., 2014. Appl. Energ. 115:226-232.
Verduyn, C., et al., 1992. Yeast. 8:501-517.
von Heijne, G., 1985. J Mol Biol. 184: 99-105.
Wei, H., et al., 2014, Biotechnology for Biofuels, 7:148.
Wilson, D.B., 2009. Curr. Opin. Biotechnol. 20:295-299.
Yan, T.R., Lin, C.L., 1997. Biosci. Biotechnol. Biochem. 61:965-970.
Zhang, F., et al., 2012. Nat. Biotechnol. 30:354-359.

## Claims

1. A method for obtaining an oleaginous yeast strain capable of growing on cellobiose as carbon source, wherein said method comprises overexpressing in said strain a β-glucosidase having at least 80% identity with the polypeptide of sequence SEQ ID NO: 1 further comprising a N-terminal signal peptide and a β-glucosidase having at least 80% identity with the polypeptide of sequence SEQ ID NO: 2 further comprising a N-terminal signal peptide.

2. The method of claim 1, wherein the β-glucosidase having at least 80% identity with the polypeptide of sequence SEQ ID NO: 1 and/or the β-glucosidase having at least 80% identity with the polypeptide of sequence SEQ ID NO: 2 are from a *Yarrowia* strain.

3. The method of claim 2, wherein the β-glucosidase having at least 80% identity with the polypeptide of sequence SEQ ID NO: 1 is selected from the group consisting of SEQ ID NO: 1 and SEQ ID NO: 8.

4. The method of any one of claims 1 to 3, wherein the β-glucosidase having at least 80% identity with the polypeptide of sequence SEQ ID NO: 2 has the amino acid sequence SEQ ID NO: 9.

5. The method of any one of claims 1 to 4, wherein the β-glucosidase having at least 80% identity with the polypeptide of sequence SEQ ID NO: 2 is selected from the group consisting of SEQ ID NO: 2, SEQ ID NO: 11, SEQ ID NO: 13 and SEQ ID NO: 15.

6. The method of any one of claims 1 to 5, wherein the signal peptide of the β-glucosidase having at least 80% identity with the polypeptide of sequence SEQ ID NO: 1 and the signal peptide of the β-glucosidase having at least 80% identity with the polypeptide of sequence SEQ ID NO: 2 are identical or different and are selected from the group consisting of SEQ ID NO: 34 to 39.

7. The method of any one of claims 1 to 6, wherein the oleaginous yeast strain is selected from the group consisting of the genus *Candida*, *Cryptoccocus*, *Lipomyces*, *Rhodosporidium*, *Rhodotorula*, *Trichosporon* and *Yarrowia.*

8. The method of claim 7, wherein the oleaginous yeast strain is a *Yarrowia* strain.

9. The method of any one of claims 1 to 8, wherein the expression or activity of the endogenous isoforms of acyl-coenzymeA oxidases in said oleaginous yeast strain is inhibited.

10. The method of claim 9, wherein said oleaginous yeast strain is a *Yarrowia* strain and wherein in said strain at least one protein selected from the group consisting of an acyl-CoA:diacylglycerol acyltransferase 2, an acyl-CoA:diacylglycerol acyltransferase 1, a glycerol-3-phosphate dehydrogenase NAD+, an acetyl-CoA carboxylase and a hexokinase is further overexpressed, and/or the expression or activity of at least one endogenous protein selected from the group consisting of the glycerol 3-phosphate dehydrogenase, the triglyceride lipase and the peroxin 10 is further inhibited.

11. The method of any one of claims 1 to 10, wherein it comprises transforming an oleaginous yeast cell with a recombinant DNA construct for expressing both β-glucosidases as defined in any one of claims 1 to 6, or with two recombinant DNA constructs for expressing both β-glucosidases respectively as defined in any one of claims 1 to 6.

12. A mutant oleaginous yeast strain, wherein a β-glucosidase having at least 80% identity with the polypeptide of sequence SEQ ID NO: 1 and a β-glucosidase having at least 80% identity with the polypeptide of sequence SEQ ID NO: 2 are overexpressed and wherein it is obtainable by the method of any one of claims 1 to 11.

13. Use of a mutant oleaginous yeast strain as defined in claim 12 for producing lipids from a lignocellulosic biomass.

14. A method of producing lipids, comprising a step of growing a mutant oleaginous yeast strain as defined in claim 12 on a lignocellulosic biomass.

15. An isolated β-glucosidase having an amino acid sequence selected from the group consisting of SEQ ID NO: 7, 8 and 10 to 15.

16. Use of an isolated β-glucosidase of claim 15 for degrading cellobiose.


SEQUENCE LISTING

<110>  INSTITUT NATIONAL DE LA RECHERCHE AGRONOMIQUE
       INSTITUT NATIONAL DES SCIENCES APPLIQUEES DE TOULOUSE
       CENTRE NATIONAL DE LA RECHERCHE SCIENTIFIQUE
       MARTY, Alain
       GUO, Zhongpeng
       DUQUESNE, Sophie
       BOZONNET, Sophie
       NICAUD, Jean-Marc
       O'DONOHUE, Michael

<120>  MUTANT YEAST STRAIN CAPABLE OF DEGRADING CELLOBIOSE

<130>  XRN/11-F539-178/EP

<160>  39

<170>  PatentIn version 3.5

<210>  1
<211>  828
<212>  PRT
<213>  Yarrowia lipolytica

<400>  1
   Ser Pro Asp Trp Tyr Pro Thr Pro Glu Ile Gly Ser Ile Thr Ser Asp
   1               5                   10                  15
   Trp Ala Asp Ala Leu Gly Glu Ser Met Asp Ile Leu Ala Gln Leu Thr
               20                  25                  30
   Leu Pro Glu Lys Val Asn Ile Thr Thr Gly Thr Gly Trp Arg Gly Gly
           35                  40                  45
   Gln Cys Val Gly Asn Thr Gly Ala Val Pro Arg Leu Gly Ile Lys Gly
       50                  55                  60
   Leu Cys Leu Gln Asp Gly Pro Leu Gly Val Arg Phe Ala Asp Phe Val
   65                  70                  75                  80
   Asn Val Phe Pro Cys Gln Asn Ala Met Ala Ala Thr Phe Asp Arg Ile
                   85                  90                  95
   Leu Val His Gln Arg Gly Thr Ala Ile Gly Arg Gln Ser Arg Leu Lys
               100                 105                 110
   Gly Val Asp Val His Leu Gly Pro Val Val Gly Pro Leu Gly Arg His
           115                 120                 125
   Ala Thr Gly Gly Arg Asn Trp Glu Gly Phe Ser Pro Asp Pro Tyr Leu
       130                 135                 140
   Ser Gly Lys Leu Ala Phe Glu Ala Ile Leu Gly Val Gln Glu Glu Gly
   145                 150                 155                 160
   Val Leu Ala Thr Ile Lys His Phe Ile Gly Asn Glu Gln Glu His Tyr
                   165                 170                 175
   Arg Arg Ala Glu Glu Trp Arg Asp Gly Phe Gly Phe Lys Asp Leu Lys
               180                 185                 190
   Asp Ala Val Ser Ser Asn Ile Glu Asp Arg Ala Leu His Glu Leu Tyr
           195                 200                 205
   Met Trp Pro Phe Ala Asp Ala Val Arg Ala Asn Val Gly Ser Val Met
       210                 215                 220
   Cys Ser Tyr Asn Tyr Val Asn Gly Thr Gln Ala Cys Gln Asn Ser Asp
   225                 230                 235                 240
   Leu Leu Asn Gly Lys Leu Lys Ser Glu Leu Gly Phe Gln Gly Phe Val
                   245                 250                 255
   Met Ser Asp Trp Phe Ala Gln Gly Ser Gly Val Ser Asn Ala Leu Ala
               260                 265                 270
   Gly Met Asp Met Ser Met Pro Gly Asn Asp Val Asp Glu Leu Glu Thr
           275                 280                 285
   Val Phe Trp Gly Glu Gln Leu Thr Arg Met Val Ala Asn Gly Thr Leu
       290                 295                 300
   Pro Glu Ala Arg Leu Asp Asp Met Val Leu Arg Ile Leu Thr Pro Leu
   305                 310                 315                 320
   Ile Tyr Phe Gly Ile Asp Asp Arg Thr Pro Asn Phe Ser Ser Phe Val
                   325                 330                 335
   Asp Thr Thr Val Gly Ser Pro His Pro Ala Ala Lys His Ser Lys Lys
               340                 345                 350
   Val Lys Asp Val Ile Thr Asn Tyr His Leu Asp Val Arg Asp Gln Phe
           355                 360                 365
   Ala Ala Asn Val Ala Leu Asp Ser Ala Arg Gly Ala Val Val Leu Leu
       370                 375                 380
   Phe Asn Asp Gly Ile Leu Pro Leu Lys Asn Ile Ser Ala Ile Gly Val
   385                 390                 395                 400
   Phe Gly Val Gly Ser Arg Leu Gly Pro Asn Gly Ala Val Cys Gly Glu
                   405                 410                 415
   Asn Met Gln Cys Ser Asp Gly Ala Leu Ile Glu Gly Trp Gly Ser Gly
               420                 425                 430
   Thr Ala Tyr Pro Thr Glu Tyr Glu Ser Pro Tyr Glu Ala Leu His Lys
           435                 440                 445
   Lys Ala Ser Leu Leu Glu Val Ser Val Thr Gly Thr Thr Glu Ser Trp
       450                 455                 460
   Asp Met Arg Leu Pro Leu Glu Leu Ala Gly Asp Thr Asp Val Asn Ile
   465                 470                 475                 480
   Val Tyr Val Leu Ala Asn Ser Gly Glu Ser Thr Ala Asn Val Asp Lys
                   485                 490                 495
   Asn Leu Gly Asp Arg Arg Asn Val Ser Leu Trp His Asn Gly Asp Glu
               500                 505                 510
   Leu Ile Asn Thr Val Ala Ser Gln Gly Gln Thr Val Val Val Val Thr
           515                 520                 525
   Thr Val Gly Gln Val Asp Met Thr Ala Trp Leu Asn His Pro Asn Ile
       530                 535                 540
   Ser Ala Val Leu Leu Thr Ala Pro Ala Gly Asp Tyr Gly Gly Lys Ala
   545                 550                 555                 560
   Ile Ala Asp Val Leu Phe Gly Glu Val Asn Pro Ser Gly Lys Leu Pro
                   565                 570                 575
   Tyr Thr Ile Ala Ala Asn Thr Ser Asp Tyr Ile Pro Ile Val Thr Lys
               580                 585                 590
   Ile Pro Arg Asp Gly Ala Pro Gln Ser Asp Phe Val Glu Gly Ile Tyr
           595                 600                 605
   Leu Asp Tyr Lys Trp Tyr Asp Lys Phe Glu Arg Thr Pro Leu Tyr Glu
       610                 615                 620
   Phe Gly Tyr Gly Leu Ser Tyr Thr Thr Tyr Ser Phe Ser Asn Leu His
   625                 630                 635                 640
   Leu Asp Val Lys Glu Ile Ser Glu Phe Leu Pro Pro Arg Pro Val Pro
                   645                 650                 655
   Val Gln Val Thr Lys Pro Lys Met Thr Asn Ile Asp Ile Glu Asp Leu
               660                 665                 670
   Tyr Val Pro Asn Asp Phe Lys Met Ile Asp Gly Leu Val Tyr Pro Trp
           675                 680                 685
   Ile Leu Asn Ala Ser Ala Pro Leu Ala Asp Ser Gln Thr Gln Phe Pro
       690                 695                 700
   Phe Ala Asn Gly Ala Gly His Val Ser Asp Ala Ser Gly Gly Val Gly
   705                 710                 715                 720
   Gly His Pro Trp Leu Trp Ser Asn Ala Val Thr Val Thr His Asn Thr
                   725                 730                 735
   Thr Asn Cys Gly Asp Val Ala Gly Arg Val Val Ser Gln Leu Tyr Val
               740                 745                 750
   Ala Phe Pro Glu Thr Leu Ile Asp Ser Pro Pro Val Gln Leu Arg Gly
           755                 760                 765
   Phe Asp Lys Ser Lys Leu Leu Asn Pro Gly Glu Ser Gln Met Thr Glu
       770                 775                 780
   Tyr Asn Leu Asn Trp Arg Asp Leu Ala Ile Trp Asp Val Glu Leu Gln
   785                 790                 795                 800
   Ser Trp Arg Val Gln Arg Gly Glu Tyr Ser Val Tyr Ile Gly His Ser
                   805                 810                 815
   Ser Arg Glu Phe Glu Leu Cys Glu Thr Phe Thr Leu
               820                 825

<210>  2
<211>  852
<212>  PRT
<213>  Yarrowia lipolytica

<400>  2

Asp Pro Phe Ser Asp Lys Asp Ala Tyr Lys His Ser Pro Pro Tyr Tyr
1               5                   10                  15

Pro Ala Pro Glu Ile Gly Arg Val Pro Thr Asp Leu Arg Trp Arg Ala
            20                  25                  30

Ala Leu Lys Val Ala Gln Gly Met Val Ala Asn Met Thr Leu Leu Glu
        35                  40                  45

Lys Val Asn Ile Thr Thr Gly Thr Gly Trp Glu Met Gly Pro Cys Val
    50                  55                  60

Gly Asn Thr Gly Thr Val Glu Arg Leu Gly Ile Lys Ser Leu Cys Leu
65                  70                  75                  80

Gln Asp Gly Pro Leu Gly Ile Arg Phe Ala Asp Leu Ile Thr Thr Phe
                85                  90                  95

Pro Ala Gly Ile Thr Ile Ala Ser Thr Phe Ser Arg Gln Leu Val Arg
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg His Ala Asn Gly
    130                 135                 140

Gly Arg Ile Trp Glu Gly Phe Ser Ala Asp Pro Tyr Leu Ala Gly Lys
145                 150                 155                 160

Leu Ala Ala Glu Ala Val Thr Gly Ile Gln Gly Gln Asn Val Met Ala
                165                 170                 175

Val Val Lys His Met Val Gly Asn Glu Gln Glu His Phe Arg Gln Leu
            180                 185                 190

Gly Glu Trp Gln Gly Phe Gly Phe Lys Asp Leu Lys Gln Pro Leu Ser
        195                 200                 205

Ser Asn Ile Asp Asp Arg Thr Leu Asn Glu Ala Tyr Leu Trp Pro Phe
    210                 215                 220

Ala Asp Ala Val Arg Ala Asn Val Gly Ser Val Met Cys Ser Tyr Gln
225                 230                 235                 240

Gln Ile Asn Gly Ser Gln Gly Cys Gln Asn Ala His Ile Leu Asn Gly
                245                 250                 255

Lys Leu Lys Glu Glu Met Gly Phe Gln Gly Phe Val Met Ser Asp Trp
            260                 265                 270

Leu Ala Gln Arg Ser Gly Val Ala Ser Val Leu Ala Gly Leu Asp Met
        275                 280                 285

Ser Met Pro Gly Asp Gly Leu Val Trp Ala Asp Gly Val Pro Leu Met

290                 295                 300

Gly Tyr Glu Leu Thr Arg Ser Val Leu Asn Gly Thr Ile Asp Glu Ser
305                 310                 315                 320

Arg Val Asp Asp Met Val Thr Arg Ile Leu Thr Pro Ile Leu Tyr Leu
                325                 330                 335

Ser Ile Thr Pro Thr Asp Pro Asn Phe Ser Ser Trp Thr Asn Asp Thr
            340                 345                 350

Thr Ser Tyr Lys Tyr Tyr Gly Ala Lys Ala Gly Gly Asn Val Thr Val
        355                 360                 365

Asn Arg His Ile Asp Val Arg Asp Gln Tyr Thr Thr Lys Ala Ala Leu
    370                 375                 380

Asp Gly Ala Asn Ala Ala Leu Val Leu Leu Lys Asn Glu Lys Lys Thr
385                 390                 395                 400

Leu Pro Leu Asn Pro Thr Asn Ile Gly Asn Leu Asn Ile Phe Gly Ile
                405                 410                 415

Gly Ser Lys Thr Gly Pro Leu Gly Ala Val Cys Gly Glu Asn Met Gln
            420                 425                 430

Cys Ser Asp Gly Ala Leu Ile Glu Gly Trp Gly Ser Gly Ser Val Tyr
        435                 440                 445

Pro Thr Asp Tyr Gln Ser Pro Tyr Asp Ala Ile Lys Glu Arg Ala Ser
    450                 455                 460

Lys Asp Asn Ile Thr Ile Gly Gly Thr Thr Gln Ser Trp Gly Asn Leu
465                 470                 475                 480

Ser Asn Val Glu Ile Leu Ser Ala Ala Ala Asp Ala Ser Val Val Phe
                485                 490                 495

Val Leu Ser Asp Ser Gly Glu Ser Thr Gly Ile Val Asp Gly Asn Ile
            500                 505                 510

Gly Asp Arg Asn Asn Leu Thr Leu Trp His Asn Gly Asp Glu Val Val
        515                 520                 525

Lys Ala Val Ala Ser Lys Asn Pro Asn Thr Ile Val Val Val Thr Thr
    530                 535                 540

Val Gly Pro Val Asn Leu Glu Lys Trp Ile Asp Asn Pro Asn Val Thr
545                 550                 555                 560

Ala Val Leu Leu Thr Gly Pro Ala Gly Asp Phe Gly Gly Arg Ala Ala
                565                 570                 575

Ala Ser Ile Leu Phe Gly Asp Ile Ala Pro Ser Gly Lys Leu Pro Phe
            580                 585                 590

Thr Ile Ala Lys Asn Asp Thr Asp Tyr Ile Pro Leu Thr Thr Lys Ile
        595                 600                 605

Pro Glu Asp Gly Leu Pro Gln Asp Tyr Phe Thr Glu Gly Thr Leu Leu
    610                 615                 620

Asp Tyr Lys Arg Phe Asp Glu Asn Gln Val Thr Pro Arg Phe Glu Phe
625                 630                 635                 640

Gly Tyr Gly Leu Ser Tyr Ser Asn Ile Thr Val Glu Asn Leu Glu Ala
                645                 650                 655

Arg Tyr Ala Phe Pro Ser Ile Pro Glu Phe Leu Pro Thr Pro Phe Ala
            660                 665                 670

Pro Ser Asn Pro Asn Lys Pro Lys Asn Ala Phe Thr Pro His Ala Asn
        675                 680                 685

Glu Ser Val Phe Pro Ser Asp Ile Asp Pro Leu Asn Lys Tyr Val Tyr
    690                 695                 700

Pro Tyr Leu Asn Asp Thr Ser Glu Ile Phe Ser Asn Glu Thr His Tyr
705                 710                 715                 720

Pro Tyr Pro Glu Gly Tyr Ser Ser Glu Gln Ser Asn Ser Thr Asn Ile
                725                 730                 735

Asn Gly Gly Ala Val Gly Gly Asn Pro Ala Leu Trp Leu Ser Ala Val
            740                 745                 750

Tyr Ile Val His Ser Val Ser Asn Tyr Gly Pro Tyr Asp Thr Gly Val
        755                 760                 765

Val Thr Gln Met Tyr Ile Ala Phe Pro Gln Asp Asn Asp Asp Leu Lys
    770                 775                 780

Thr Ala Pro Arg Gln Leu Arg Gly Phe Glu Arg Ser Glu Leu Lys Val
785                 790                 795                 800

Gly Glu Arg Gln Gly Ile Leu Tyr Asp Val Gln Trp Arg Asp Leu Ala
                805                 810                 815

Val Trp Asp Val Lys Leu Gln Ser Trp Arg Val Gln Arg Gly Glu Tyr
            820                 825                 830

Lys Val Tyr Val Gly His Ser Ser Arg Asp Phe Val Leu Thr Thr Ser
        835                 840                 845

Phe Thr Leu Lys
    850

<210>  3
<211>  2535
<212>  DNA
<213>  Yarrowia lipolytica

<220>
<221>  CDS
<222>  (1)..(2535)

<220>
<221>  sig_peptide
<222>  (1)..(48)

<400>  3
atg atc ttc tct ctg caa cta cta ctg acg acg gca cta gcg gcc tct      48
Met Ile Phe Ser Leu Gln Leu Leu Leu Thr Thr Ala Leu Ala Ala Ser
1               5                   10                  15

tcg cct gac tgg tac ccc aca ccc gaa atc ggt tca att acc agt gat      96
Ser Pro Asp Trp Tyr Pro Thr Pro Glu Ile Gly Ser Ile Thr Ser Asp
            20                  25                  30

tgg gcc gat gct ctt ggt gag tcc atg gac atc ctg gcc caa ttg act      144
Trp Ala Asp Ala Leu Gly Glu Ser Met Asp Ile Leu Ala Gln Leu Thr
        35                  40                  45

ctt ccc gaa aag gtc aac atc acc acc ggt acc gga tgg aga ggt ggg      192
Leu Pro Glu Lys Val Asn Ile Thr Thr Gly Thr Gly Trp Arg Gly Gly
    50                  55                  60

cag tgt gtt gga aac aca ggg gct gtt cct cgt ctc gga atc aag ggc      240
Gln Cys Val Gly Asn Thr Gly Ala Val Pro Arg Leu Gly Ile Lys Gly
65                  70                  75                  80

ctg tgt ctc caa gac ggt cct ctg ggc gtc cgt ttt gcc gac ttt gtc      288
Leu Cys Leu Gln Asp Gly Pro Leu Gly Val Arg Phe Ala Asp Phe Val
                85                  90                  95

aat gtc ttc cct tgc cag aac gca atg gcc gcc acc ttt gat cgt atc      336
Asn Val Phe Pro Cys Gln Asn Ala Met Ala Ala Thr Phe Asp Arg Ile
            100                 105                 110

cta gtt cac caa cga gga acc gct att gga cgt cag tct aga ctc aag      384
Leu Val His Gln Arg Gly Thr Ala Ile Gly Arg Gln Ser Arg Leu Lys
        115                 120                 125

gga gtc gat gtt cat ctc gga cca gtg gtg gga cct ctt gga cga cac 432
Gly Val Asp Val His Leu Gly Pro Val Val Gly Pro Leu Gly Arg His
    130                 135                 140

gct acc ggc gga aga aac tgg gaa ggt ttc tcc ccc gac cca tat ctg 480
Ala Thr Gly Gly Arg Asn Trp Glu Gly Phe Ser Pro Asp Pro Tyr Leu
145                 150                 155                 160

tct gga aag ctc gcc ttc gaa gca att ctt gga gtc cag gaa gag gga 528
Ser Gly Lys Leu Ala Phe Glu Ala Ile Leu Gly Val Gln Glu Glu Gly
                165                 170                 175

gtt ctt gca acc atc aag cac ttc att gga aac gaa caa gaa cat tac 576
Val Leu Ala Thr Ile Lys His Phe Ile Gly Asn Glu Gln Glu His Tyr
            180                 185                 190

cgg cga gcc gaa gag tgg aga gac ggc ttt gga ttt aaa gac ctg aag 624
Arg Arg Ala Glu Glu Trp Arg Asp Gly Phe Gly Phe Lys Asp Leu Lys
        195                 200                 205

gac gcc gtc tct tcc aac att gaa gac aga gct cta cat gag ttg tac 672
Asp Ala Val Ser Ser Asn Ile Glu Asp Arg Ala Leu His Glu Leu Tyr
    210                 215                 220

atg tgg ccg ttt gcc gat gct gtt agg gct aat gtc ggc tca gtc atg 720
Met Trp Pro Phe Ala Asp Ala Val Arg Ala Asn Val Gly Ser Val Met
225                 230                 235                 240

tgc tcc tac aac tac gtg aac gga acc cag gct tgc cag aac agt gac 768
Cys Ser Tyr Asn Tyr Val Asn Gly Thr Gln Ala Cys Gln Asn Ser Asp
                245                 250                 255

ttg ctc aac gga aag ctc aag tcc gag ctc ggt ttc cag ggc ttt gtc 816
Leu Leu Asn Gly Lys Leu Lys Ser Glu Leu Gly Phe Gln Gly Phe Val
            260                 265                 270

atg tcc gac tgg ttt gct cag gga agc gga gtg tct aac gct ctg gct 864
Met Ser Asp Trp Phe Ala Gln Gly Ser Gly Val Ser Asn Ala Leu Ala
        275                 280                 285

gga atg gac atg agt atg cct gga aat gac gtt gat gag tta gaa act 912
Gly Met Asp Met Ser Met Pro Gly Asn Asp Val Asp Glu Leu Glu Thr
    290                 295                 300

gtc ttc tgg gga gaa cag ctg acc cga atg gtt gcc aac ggt acc ctt 960
Val Phe Trp Gly Glu Gln Leu Thr Arg Met Val Ala Asn Gly Thr Leu
305                 310                 315                 320

cca gaa gcc cgt ctc gat gat atg gtt ctg cga att ctg acc cct ctc 1008
Pro Glu Ala Arg Leu Asp Asp Met Val Leu Arg Ile Leu Thr Pro Leu
                325                 330                 335

atc tac ttt ggg atc gac gat cga aca ccc aac ttc tcc tcc ttt gtc 1056
Ile Tyr Phe Gly Ile Asp Asp Arg Thr Pro Asn Phe Ser Ser Phe Val
            340                 345                 350

gac act aca gtg gga agt ccc cac ccc gct gct aag cac tcc aag aaa 1104
Asp Thr Thr Val Gly Ser Pro His Pro Ala Ala Lys His Ser Lys Lys
        355                 360                 365

gtc aag gat gtt atc acc aac tac cat ctc gat gtg cga gac cag ttt 1152
Val Lys Asp Val Ile Thr Asn Tyr His Leu Asp Val Arg Asp Gln Phe

370                 375                 380

gca gcc aat gtt gct ctt gat agt gct cga gga gct gtt gtt ctg ctt        1200
Ala Ala Asn Val Ala Leu Asp Ser Ala Arg Gly Ala Val Val Leu Leu
385                 390                 395                 400

ttc aat gac ggt att ctt ccc ctg aag aac att tcc gct att gga gta        1248
Phe Asn Asp Gly Ile Leu Pro Leu Lys Asn Ile Ser Ala Ile Gly Val
                405                 410                 415

ttt gga gtc ggc tct aga ctt ggc ccc aat gga gct gtt tgt ggg gaa        1296
Phe Gly Val Gly Ser Arg Leu Gly Pro Asn Gly Ala Val Cys Gly Glu
            420                 425                 430

aac atg cag tgc tca gac gga gct ctt atc gaa gga tgg gga agt gga        1344
Asn Met Gln Cys Ser Asp Gly Ala Leu Ile Glu Gly Trp Gly Ser Gly
        435                 440                 445

act gct tac cct acg gag tac gaa agc cct tac gaa gct ctc cac aaa        1392
Thr Ala Tyr Pro Thr Glu Tyr Glu Ser Pro Tyr Glu Ala Leu His Lys
    450                 455                 460

aag gct tct cta ctt gaa gtg tca gtg aca gga acc acc gag tca tgg        1440
Lys Ala Ser Leu Leu Glu Val Ser Val Thr Gly Thr Thr Glu Ser Trp
465                 470                 475                 480

gat atg aga ctt cct ctt gag ctg gct gga gac act gac gtg aat att        1488
Asp Met Arg Leu Pro Leu Glu Leu Ala Gly Asp Thr Asp Val Asn Ile
                485                 490                 495

gtt tat gta ttg gca aat tca gga gag tct act gcc aac gtt gat aag        1536
Val Tyr Val Leu Ala Asn Ser Gly Glu Ser Thr Ala Asn Val Asp Lys
            500                 505                 510

aac ctt ggg gat cgc cga aat gtc agt cta tgg cac aat ggc gat gaa        1584
Asn Leu Gly Asp Arg Arg Asn Val Ser Leu Trp His Asn Gly Asp Glu
        515                 520                 525

ctt att aat aca gtt gcc agt cag gga cag act gtc gtt gtt gtc act        1632
Leu Ile Asn Thr Val Ala Ser Gln Gly Gln Thr Val Val Val Val Thr
    530                 535                 540

acg gtt gga caa gtt gac atg acc gct tgg ctc aac cac ccc aac atc        1680
Thr Val Gly Gln Val Asp Met Thr Ala Trp Leu Asn His Pro Asn Ile
545                 550                 555                 560

agt gcc gtt ctt ctg act gct cct gct ggt gat tac gga gga aaa gcc        1728
Ser Ala Val Leu Leu Thr Ala Pro Ala Gly Asp Tyr Gly Gly Lys Ala
                565                 570                 575

atc gct gat gtg tta ttt gga gag gtt aat ccc tca gga aag ctg cct        1776
Ile Ala Asp Val Leu Phe Gly Glu Val Asn Pro Ser Gly Lys Leu Pro
            580                 585                 590

tat act atc gca gca aat act tct gat tat att cct att gtc acc aag        1824
Tyr Thr Ile Ala Ala Asn Thr Ser Asp Tyr Ile Pro Ile Val Thr Lys
        595                 600                 605

atc cct cga gat gga gct ccc cag tcc gac ttt gtg gag gga atc tat        1872
Ile Pro Arg Asp Gly Ala Pro Gln Ser Asp Phe Val Glu Gly Ile Tyr
    610                 615                 620

ctt gac tac aag tgg tac gac aag ttt gaa agg act ccc ctc tac gaa        1920

Leu Asp Tyr Lys Trp Tyr Asp Lys Phe Glu Arg Thr Pro Leu Tyr Glu
625                 630                 635                 640

ttt ggt tac ggt ctg tca tac acc acc tac tcc ttc agc aat ctg cat      1968
Phe Gly Tyr Gly Leu Ser Tyr Thr Thr Tyr Ser Phe Ser Asn Leu His
                645                 650                 655

ctt gat gtt aag gag att agt gag ttc ctt cct ccc cgg cct gtt cct      2016
Leu Asp Val Lys Glu Ile Ser Glu Phe Leu Pro Pro Arg Pro Val Pro
            660                 665                 670

gta cag gtt act aaa ccc aag atg acc aat atc gac att gag gac ctt      2064
Val Gln Val Thr Lys Pro Lys Met Thr Asn Ile Asp Ile Glu Asp Leu
        675                 680                 685

tat gtc ccc aat gac ttt aaa atg att gac ggt ctt gtt tac cct tgg      2112
Tyr Val Pro Asn Asp Phe Lys Met Ile Asp Gly Leu Val Tyr Pro Trp
    690                 695                 700

att ctc aac gcc agt gcg ccc ctc gcg gac tct cag act cag ttc ccc      2160
Ile Leu Asn Ala Ser Ala Pro Leu Ala Asp Ser Gln Thr Gln Phe Pro
705                 710                 715                 720

ttt gca aat gga gct gga cat gtc agt gac gct tct gga gga gtg ggt      2208
Phe Ala Asn Gly Ala Gly His Val Ser Asp Ala Ser Gly Gly Val Gly
                725                 730                 735

ggt cac ccc tgg ctt tgg tct aac gct gtt act gtc act cac aac acc      2256
Gly His Pro Trp Leu Trp Ser Asn Ala Val Thr Val Thr His Asn Thr
            740                 745                 750

acc aac tgc ggt gat gtt gct gga cga gta gtt tct cag ctg tac gtt      2304
Thr Asn Cys Gly Asp Val Ala Gly Arg Val Val Ser Gln Leu Tyr Val
        755                 760                 765

gcc ttc cct gaa acc ctt atc gac tct cct ccg gtg cag ctt cga gga      2352
Ala Phe Pro Glu Thr Leu Ile Asp Ser Pro Pro Val Gln Leu Arg Gly
    770                 775                 780

ttc gac aag tcc aag ctc ctg aac cct ggt gag tct cag atg acc gag      2400
Phe Asp Lys Ser Lys Leu Leu Asn Pro Gly Glu Ser Gln Met Thr Glu
785                 790                 795                 800

tac aac ctc aac tgg cga gat ctg gcc att tgg gac gtg gaa ctg cag      2448
Tyr Asn Leu Asn Trp Arg Asp Leu Ala Ile Trp Asp Val Glu Leu Gln
                805                 810                 815

agc tgg aga gtg cag cga ggc gag tat tcc gtg tac att ggt cat tcc      2496
Ser Trp Arg Val Gln Arg Gly Glu Tyr Ser Val Tyr Ile Gly His Ser
            820                 825                 830

agc cga gaa ttc gag cta tgt gag act ttc act ttg tag                  2535
Ser Arg Glu Phe Glu Leu Cys Glu Thr Phe Thr Leu
        835                 840


<210>  4
<211>  844
<212>  PRT
<213>  Yarrowia lipolytica

<400>  4

Met Ile Phe Ser Leu Gln Leu Leu Leu Thr Thr Ala Leu Ala Ala Ser
1               5                   10                  15

Ser Pro Asp Trp Tyr Pro Thr Pro Glu Ile Gly Ser Ile Thr Ser Asp
            20                  25                  30

Trp Ala Asp Ala Leu Gly Glu Ser Met Asp Ile Leu Ala Gln Leu Thr
        35                  40                  45

Leu Pro Glu Lys Val Asn Ile Thr Thr Gly Thr Gly Trp Arg Gly Gly
    50                  55                  60

Gln Cys Val Gly Asn Thr Gly Ala Val Pro Arg Leu Gly Ile Lys Gly
65                  70                  75                  80

Leu Cys Leu Gln Asp Gly Pro Leu Gly Val Arg Phe Ala Asp Phe Val
                85                  90                  95

Asn Val Phe Pro Cys Gln Asn Ala Met Ala Ala Thr Phe Asp Arg Ile
            100                 105                 110

Leu Val His Gln Arg Gly Thr Ala Ile Gly Arg Gln Ser Arg Leu Lys
        115                 120                 125

Gly Val Asp Val His Leu Gly Pro Val Val Gly Pro Leu Gly Arg His
    130                 135                 140

Ala Thr Gly Gly Arg Asn Trp Glu Gly Phe Ser Pro Asp Pro Tyr Leu
145                 150                 155                 160

Ser Gly Lys Leu Ala Phe Glu Ala Ile Leu Gly Val Gln Glu Glu Gly
                165                 170                 175

Val Leu Ala Thr Ile Lys His Phe Ile Gly Asn Glu Gln Glu His Tyr
            180                 185                 190

Arg Arg Ala Glu Glu Trp Arg Asp Gly Phe Gly Phe Lys Asp Leu Lys
        195                 200                 205

Asp Ala Val Ser Ser Asn Ile Glu Asp Arg Ala Leu His Glu Leu Tyr
    210                 215                 220

Met Trp Pro Phe Ala Asp Ala Val Arg Ala Asn Val Gly Ser Val Met
225                 230                 235                 240

Cys Ser Tyr Asn Tyr Val Asn Gly Thr Gln Ala Cys Gln Asn Ser Asp
                245                 250                 255

Leu Leu Asn Gly Lys Leu Lys Ser Glu Leu Gly Phe Gln Gly Phe Val
            260                 265                 270
Met Ser Asp Trp Phe Ala Gln Gly Ser Gly Val Ser Asn Ala Leu Ala
        275                 280                 285
Gly Met Asp Met Ser Met Pro Gly Asn Asp Val Asp Glu Leu Glu Thr
    290                 295                 300
Val Phe Trp Gly Glu Gln Leu Thr Arg Met Val Ala Asn Gly Thr Leu
305                 310                 315                 320
Pro Glu Ala Arg Leu Asp Asp Met Val Leu Arg Ile Leu Thr Pro Leu
                325                 330                 335
Ile Tyr Phe Gly Ile Asp Asp Arg Thr Pro Asn Phe Ser Ser Phe Val
            340                 345                 350
Asp Thr Thr Val Gly Ser Pro His Pro Ala Ala Lys His Ser Lys Lys
        355                 360                 365
Val Lys Asp Val Ile Thr Asn Tyr His Leu Asp Val Arg Asp Gln Phe
    370                 375                 380
Ala Ala Asn Val Ala Leu Asp Ser Ala Arg Gly Ala Val Val Leu Leu
385                 390                 395                 400
Phe Asn Asp Gly Ile Leu Pro Leu Lys Asn Ile Ser Ala Ile Gly Val
                405                 410                 415
Phe Gly Val Gly Ser Arg Leu Gly Pro Asn Gly Ala Val Cys Gly Glu
            420                 425                 430
Asn Met Gln Cys Ser Asp Gly Ala Leu Ile Glu Gly Trp Gly Ser Gly
        435                 440                 445
Thr Ala Tyr Pro Thr Glu Tyr Glu Ser Pro Tyr Glu Ala Leu His Lys
    450                 455                 460
Lys Ala Ser Leu Leu Glu Val Ser Val Thr Gly Thr Thr Glu Ser Trp
465                 470                 475                 480
Asp Met Arg Leu Pro Leu Glu Leu Ala Gly Asp Thr Asp Val Asn Ile
                485                 490                 495
Val Tyr Val Leu Ala Asn Ser Gly Glu Ser Thr Ala Asn Val Asp Lys
            500                 505                 510

Asn Leu Gly Asp Arg Arg Asn Val Ser Leu Trp His Asn Gly Asp Glu
        515                 520                 525

Leu Ile Asn Thr Val Ala Ser Gln Gly Gln Thr Val Val Val Val Thr
    530                 535                 540

Thr Val Gly Gln Val Asp Met Thr Ala Trp Leu Asn His Pro Asn Ile
545                 550                 555                 560

Ser Ala Val Leu Leu Thr Ala Pro Ala Gly Asp Tyr Gly Gly Lys Ala
                565                 570                 575

Ile Ala Asp Val Leu Phe Gly Glu Val Asn Pro Ser Gly Lys Leu Pro
            580                 585                 590

Tyr Thr Ile Ala Ala Asn Thr Ser Asp Tyr Ile Pro Ile Val Thr Lys
        595                 600                 605

Ile Pro Arg Asp Gly Ala Pro Gln Ser Asp Phe Val Glu Gly Ile Tyr
    610                 615                 620

Leu Asp Tyr Lys Trp Tyr Asp Lys Phe Glu Arg Thr Pro Leu Tyr Glu
625                 630                 635                 640

Phe Gly Tyr Gly Leu Ser Tyr Thr Thr Tyr Ser Phe Ser Asn Leu His
                645                 650                 655

Leu Asp Val Lys Glu Ile Ser Glu Phe Leu Pro Pro Arg Pro Val Pro
            660                 665                 670

Val Gln Val Thr Lys Pro Lys Met Thr Asn Ile Asp Ile Glu Asp Leu
        675                 680                 685

Tyr Val Pro Asn Asp Phe Lys Met Ile Asp Gly Leu Val Tyr Pro Trp
    690                 695                 700

Ile Leu Asn Ala Ser Ala Pro Leu Ala Asp Ser Gln Thr Gln Phe Pro
705                 710                 715                 720

Phe Ala Asn Gly Ala Gly His Val Ser Asp Ala Ser Gly Gly Val Gly
                725                 730                 735

Gly His Pro Trp Leu Trp Ser Asn Ala Val Thr Val Thr His Asn Thr
            740                 745                 750

Thr Asn Cys Gly Asp Val Ala Gly Arg Val Val Ser Gln Leu Tyr Val

755                 760                 765

Ala Phe Pro Glu Thr Leu Ile Asp Ser Pro Pro Val Gln Leu Arg Gly
    770                 775                 780

Phe Asp Lys Ser Lys Leu Leu Asn Pro Gly Glu Ser Gln Met Thr Glu
785                 790                 795                 800

Tyr Asn Leu Asn Trp Arg Asp Leu Ala Ile Trp Asp Val Glu Leu Gln
                805                 810                 815

Ser Trp Arg Val Gln Arg Gly Glu Tyr Ser Val Tyr Ile Gly His Ser
            820                 825                 830

Ser Arg Glu Phe Glu Leu Cys Glu Thr Phe Thr Leu
        835                 840

<210>  5
<211>  2610
<212>  DNA
<213>  Yarrowia lipolytica

<220>
<221>  CDS
<222>  (1)..(2610)

<220>
<221>  sig_peptide
<222>  (1)..(51)

<400>  5
atg ctc gca ttc gtc cta ctg ctg acg atg ctg ctc gca gca gcg ctt      48
Met Leu Ala Phe Val Leu Leu Leu Thr Met Leu Leu Ala Ala Ala Leu
1               5                   10                  15

gct gac ccg ttc tca gat aaa gac gct tac aaa cac agc cct cca tac      96
Ala Asp Pro Phe Ser Asp Lys Asp Ala Tyr Lys His Ser Pro Pro Tyr
            20                  25                  30

tac cct gct ccg gag att ggc aga gtt ccc acc gac ctg cga tgg aga      144
Tyr Pro Ala Pro Glu Ile Gly Arg Val Pro Thr Asp Leu Arg Trp Arg
        35                  40                  45

gct gcc ttg aag gtg gcc cag ggt atg gtc gct aac atg aca ctg ctt      192
Ala Ala Leu Lys Val Ala Gln Gly Met Val Ala Asn Met Thr Leu Leu
    50                  55                  60

gag aag gtg aac atc acc acc ggt acg ggc tgg gag atg ggt cct tgt      240
Glu Lys Val Asn Ile Thr Thr Gly Thr Gly Trp Glu Met Gly Pro Cys
65                  70                  75                  80

gtt gga aac act ggt acc gtc gaa cga ctg ggt ata aaa tcg ctg tgc      288
Val Gly Asn Thr Gly Thr Val Glu Arg Leu Gly Ile Lys Ser Leu Cys
                85                  90                  95

ctt caa gac ggc cct ctt ggg att cga ttt gct gac ctc att acc aca      336

Leu Gln Asp Gly Pro Leu Gly Ile Arg Phe Ala Asp Leu Ile Thr Thr
            100                 105                 110

ttc cct gct ggt atc act att gcc tct acc ttc tct cga cag ctg gtt 384
Phe Pro Ala Gly Ile Thr Ile Ala Ser Thr Phe Ser Arg Gln Leu Val
        115                 120                 125

aga gag cga ggt gct gct atg gga cgg gag aat aga cgc aag gga gtg 432
Arg Glu Arg Gly Ala Ala Met Gly Arg Glu Asn Arg Arg Lys Gly Val
    130                 135                 140

gat atc act ctc agc cct gtg gtt gga cca ctg gga aga cat gct aac 480
Asp Ile Thr Leu Ser Pro Val Val Gly Pro Leu Gly Arg His Ala Asn
145                 150                 155                 160

gga ggt cga atc tgg gag ggc ttc tct gct gac ccc tac ctt gct gga 528
Gly Gly Arg Ile Trp Glu Gly Phe Ser Ala Asp Pro Tyr Leu Ala Gly
                165                 170                 175

aag ctc gcc gcc gag gcc gtg aca ggt ata cag ggc cag aac gtc atg 576
Lys Leu Ala Ala Glu Ala Val Thr Gly Ile Gln Gly Gln Asn Val Met
            180                 185                 190

gct gtg gta aag cat atg gtt gga aac gag cag gaa cat ttt cga caa 624
Ala Val Val Lys His Met Val Gly Asn Glu Gln Glu His Phe Arg Gln
        195                 200                 205

ctt ggc gag tgg cag gga ttc gga ttc aag gat ctg aag cag ccc ctc 672
Leu Gly Glu Trp Gln Gly Phe Gly Phe Lys Asp Leu Lys Gln Pro Leu
    210                 215                 220

tct tca aac atc gac gac cga act ctt aac gaa gcg tac ctt tgg ccc 720
Ser Ser Asn Ile Asp Asp Arg Thr Leu Asn Glu Ala Tyr Leu Trp Pro
225                 230                 235                 240

ttt gct gat gct gtt cga gcc aat gtt gga tct gtc atg tgt tcc tat 768
Phe Ala Asp Ala Val Arg Ala Asn Val Gly Ser Val Met Cys Ser Tyr
                245                 250                 255

cag cag atc aat ggc tct cag ggt tgt caa aac gcc cac att ctg aac 816
Gln Gln Ile Asn Gly Ser Gln Gly Cys Gln Asn Ala His Ile Leu Asn
            260                 265                 270

ggt aag ctc aag gag gaa atg ggt ttc cag ggc ttt gtc atg tcc gac 864
Gly Lys Leu Lys Glu Glu Met Gly Phe Gln Gly Phe Val Met Ser Asp
        275                 280                 285

tgg ctt gcc cag cga agt ggc gtg gcg tct gtt ctt gct ggt ctc gat 912
Trp Leu Ala Gln Arg Ser Gly Val Ala Ser Val Leu Ala Gly Leu Asp
    290                 295                 300

atg agc atg cct gga gac ggt ctt gtc tgg gcg gac ggt gtt ccg ctc 960
Met Ser Met Pro Gly Asp Gly Leu Val Trp Ala Asp Gly Val Pro Leu
305                 310                 315                 320

atg gga tac gag ttg acc agg agt gtg ctg aat gga acc att gat gaa 1008
Met Gly Tyr Glu Leu Thr Arg Ser Val Leu Asn Gly Thr Ile Asp Glu
                325                 330                 335

agc cga gtg gac gac atg gtt acc cga atc ctt acc ccc ata ctg tat 1056
Ser Arg Val Asp Asp Met Val Thr Arg Ile Leu Thr Pro Ile Leu Tyr
            340                 345                 350

ctc tca att act ccg acc gac ccc aac ttc agc tct tgg acc aac gac 1104
Leu Ser Ile Thr Pro Thr Asp Pro Asn Phe Ser Ser Trp Thr Asn Asp
        355                 360                 365

act act agc tac aag tac tac gga gcc aag gct ggt gga aac gtc act 1152
Thr Thr Ser Tyr Lys Tyr Tyr Gly Ala Lys Ala Gly Gly Asn Val Thr
    370                 375                 380

gtt aac cga cat att gat gtg aga gac cag tac act acc aag gct gct 1200
Val Asn Arg His Ile Asp Val Arg Asp Gln Tyr Thr Thr Lys Ala Ala
385                 390                 395                 400

ctt gat gga gca aac gct gcg ctt gtt ctt ctc aag aat gag aag aag 1248
Leu Asp Gly Ala Asn Ala Ala Leu Val Leu Leu Lys Asn Glu Lys Lys
                405                 410                 415

act ctt cct ctg aac cct acc aat att gga aac ctc aac att ttc ggt 1296
Thr Leu Pro Leu Asn Pro Thr Asn Ile Gly Asn Leu Asn Ile Phe Gly
            420                 425                 430

att ggt tct aaa acc ggc cca ctt gga gct gtc tgt gga gaa aat atg 1344
Ile Gly Ser Lys Thr Gly Pro Leu Gly Ala Val Cys Gly Glu Asn Met
        435                 440                 445

cag tgt agt gat ggc gcc ctt att gag gga tgg ggg tcc ggt tcc gtc 1392
Gln Cys Ser Asp Gly Ala Leu Ile Glu Gly Trp Gly Ser Gly Ser Val
    450                 455                 460

tac cct acc gat tat caa tct cct tac gac gcc att aag gag aga gcc 1440
Tyr Pro Thr Asp Tyr Gln Ser Pro Tyr Asp Ala Ile Lys Glu Arg Ala
465                 470                 475                 480

tcc aag gac aac atc acc atc gga ggc act acg caa tct tgg ggt aac 1488
Ser Lys Asp Asn Ile Thr Ile Gly Gly Thr Thr Gln Ser Trp Gly Asn
                485                 490                 495

ctg tcg aac gtt gag atc ctt tca gct gct gcc gac gcc agt gtc gtc 1536
Leu Ser Asn Val Glu Ile Leu Ser Ala Ala Ala Asp Ala Ser Val Val
            500                 505                 510

ttt gtt ctt tcc gac tcc ggt gag agt act ggt att gtt gac ggc aac 1584
Phe Val Leu Ser Asp Ser Gly Glu Ser Thr Gly Ile Val Asp Gly Asn
        515                 520                 525

att ggg gat cga aac aac ttg acg ctg tgg cac aat gga gac gag gtt 1632
Ile Gly Asp Arg Asn Asn Leu Thr Leu Trp His Asn Gly Asp Glu Val
    530                 535                 540

gtc aag gct gtg gca tct aag aac ccc aac act atc gtt gtt gtt acc 1680
Val Lys Ala Val Ala Ser Lys Asn Pro Asn Thr Ile Val Val Val Thr
545                 550                 555                 560

act gtc ggc cct gtg aac ctc gaa aag tgg atc gac aac cca aac gtc 1728
Thr Val Gly Pro Val Asn Leu Glu Lys Trp Ile Asp Asn Pro Asn Val
                565                 570                 575

act gcc gtg ctt ctc act gga ccc gct ggt gac ttt gga gga aga gct 1776
Thr Ala Val Leu Leu Thr Gly Pro Ala Gly Asp Phe Gly Gly Arg Ala
            580                 585                 590

gca gcc tct att ctc ttc ggc gac atc gcc cct tca gga aaa ctc cct 1824
Ala Ala Ser Ile Leu Phe Gly Asp Ile Ala Pro Ser Gly Lys Leu Pro
        595                 600                 605

ttc act att gcc aag aat gac acc gac tac att cct ctt act act aag 1872
Phe Thr Ile Ala Lys Asn Asp Thr Asp Tyr Ile Pro Leu Thr Thr Lys
    610                 615                 620

atc cct gaa gac ggc ctt cct caa gac tat ttc act gag ggt act ctt 1920
Ile Pro Glu Asp Gly Leu Pro Gln Asp Tyr Phe Thr Glu Gly Thr Leu
625                 630                 635                 640

ttg gac tac aaa cgg ttc gac gag aac cag gtg act cct agg ttt gaa 1968
Leu Asp Tyr Lys Arg Phe Asp Glu Asn Gln Val Thr Pro Arg Phe Glu
                645                 650                 655

ttt ggc tac ggt ctg tct tac tct aac att acg gtg gag aat ctc gaa 2016
Phe Gly Tyr Gly Leu Ser Tyr Ser Asn Ile Thr Val Glu Asn Leu Glu
            660                 665                 670

gcc cgg tat gct ttc cct agc att cct gag ttc ttg ccc act ccc ttt 2064
Ala Arg Tyr Ala Phe Pro Ser Ile Pro Glu Phe Leu Pro Thr Pro Phe
        675                 680                 685

gcc cct tcg aac ccc aac aag cct aag aac gca ttt act cct cac gcc 2112
Ala Pro Ser Asn Pro Asn Lys Pro Lys Asn Ala Phe Thr Pro His Ala
    690                 695                 700

aat gag tcc gtc ttc ccc agt gac att gat cct ttg aac aag tac gtc 2160
Asn Glu Ser Val Phe Pro Ser Asp Ile Asp Pro Leu Asn Lys Tyr Val
705                 710                 715                 720

tat cca tac ctg aac gat acc tcg gag atc ttc tct aac gag acc cat 2208
Tyr Pro Tyr Leu Asn Asp Thr Ser Glu Ile Phe Ser Asn Glu Thr His
                725                 730                 735

tat ccc tat cct gag ggg tac tcc agt gag cag tcc aac agt acc aac 2256
Tyr Pro Tyr Pro Glu Gly Tyr Ser Ser Glu Gln Ser Asn Ser Thr Asn
            740                 745                 750

att aac ggc ggg gct gtc gga ggc aac cct gct ctg tgg ctc tct gca 2304
Ile Asn Gly Gly Ala Val Gly Gly Asn Pro Ala Leu Trp Leu Ser Ala
        755                 760                 765

gtc tac att gtc cac agc gtg tct aac tat ggt ccc tat gat act gga 2352
Val Tyr Ile Val His Ser Val Ser Asn Tyr Gly Pro Tyr Asp Thr Gly
    770                 775                 780

gtg gtc acc cag atg tac att gcc ttc cct cag gat aac gac gat ctt 2400
Val Val Thr Gln Met Tyr Ile Ala Phe Pro Gln Asp Asn Asp Asp Leu
785                 790                 795                 800

aaa acc gct cct aga cag ctt cga gga ttc gaa cgg tcc gag ctc aag 2448
Lys Thr Ala Pro Arg Gln Leu Arg Gly Phe Glu Arg Ser Glu Leu Lys
                805                 810                 815

gtg gga gaa cgg cag gga att cta tac gat gtt caa tgg cga gat ctc 2496
Val Gly Glu Arg Gln Gly Ile Leu Tyr Asp Val Gln Trp Arg Asp Leu
            820                 825                 830

gcg gtc tgg gat gtc aaa ctt cag agc tgg cgg gtc caa cga gga gag 2544
Ala Val Trp Asp Val Lys Leu Gln Ser Trp Arg Val Gln Arg Gly Glu
        835                 840                 845

tac aag gtt tac gta ggc cac agt tcg cga gac ttt gtt ctg acc acc 2592
Tyr Lys Val Tyr Val Gly His Ser Ser Arg Asp Phe Val Leu Thr Thr

850                 855                 860

agc ttc act ctc aag tag                                           2610
Ser Phe Thr Leu Lys
865

<210>  6
<211>  869
<212>  PRT
<213>  Yarrowia lipolytica

<400>  6

Met Leu Ala Phe Val Leu Leu Leu Thr Met Leu Leu Ala Ala Ala Leu
1               5                   10                  15

Ala Asp Pro Phe Ser Asp Lys Asp Ala Tyr Lys His Ser Pro Pro Tyr
            20                  25                  30

Tyr Pro Ala Pro Glu Ile Gly Arg Val Pro Thr Asp Leu Arg Trp Arg
        35                  40                  45

Ala Ala Leu Lys Val Ala Gln Gly Met Val Ala Asn Met Thr Leu Leu
    50                  55                  60

Glu Lys Val Asn Ile Thr Thr Gly Thr Gly Trp Glu Met Gly Pro Cys
65                  70                  75                  80

Val Gly Asn Thr Gly Thr Val Glu Arg Leu Gly Ile Lys Ser Leu Cys
                85                  90                  95

Leu Gln Asp Gly Pro Leu Gly Ile Arg Phe Ala Asp Leu Ile Thr Thr
            100                 105                 110

Phe Pro Ala Gly Ile Thr Ile Ala Ser Thr Phe Ser Arg Gln Leu Val
        115                 120                 125

Arg Glu Arg Gly Ala Ala Met Gly Arg Glu Asn Arg Arg Lys Gly Val
    130                 135                 140

Asp Ile Thr Leu Ser Pro Val Val Gly Pro Leu Gly Arg His Ala Asn
145                 150                 155                 160

Gly Gly Arg Ile Trp Glu Gly Phe Ser Ala Asp Pro Tyr Leu Ala Gly
                165                 170                 175

Lys Leu Ala Ala Glu Ala Val Thr Gly Ile Gln Gly Gln Asn Val Met
            180                 185                 190

Ala Val Val Lys His Met Val Gly Asn Glu Gln Glu His Phe Arg Gln

195                 200                 205

Leu Gly Glu Trp Gln Gly Phe Gly Phe Lys Asp Leu Lys Gln Pro Leu
    210                 215                 220

Ser Ser Asn Ile Asp Asp Arg Thr Leu Asn Glu Ala Tyr Leu Trp Pro
225                 230                 235                 240

Phe Ala Asp Ala Val Arg Ala Asn Val Gly Ser Val Met Cys Ser Tyr
                245                 250                 255

Gln Gln Ile Asn Gly Ser Gln Gly Cys Gln Asn Ala His Ile Leu Asn
            260                 265                 270

Gly Lys Leu Lys Glu Glu Met Gly Phe Gln Gly Phe Val Met Ser Asp
        275                 280                 285

Trp Leu Ala Gln Arg Ser Gly Val Ala Ser Val Leu Ala Gly Leu Asp
    290                 295                 300

Met Ser Met Pro Gly Asp Gly Leu Val Trp Ala Asp Gly Val Pro Leu
305                 310                 315                 320

Met Gly Tyr Glu Leu Thr Arg Ser Val Leu Asn Gly Thr Ile Asp Glu
                325                 330                 335

Ser Arg Val Asp Asp Met Val Thr Arg Ile Leu Thr Pro Ile Leu Tyr
            340                 345                 350

Leu Ser Ile Thr Pro Thr Asp Pro Asn Phe Ser Ser Trp Thr Asn Asp
        355                 360                 365

Thr Thr Ser Tyr Lys Tyr Tyr Gly Ala Lys Ala Gly Gly Asn Val Thr
    370                 375                 380

Val Asn Arg His Ile Asp Val Arg Asp Gln Tyr Thr Thr Lys Ala Ala
385                 390                 395                 400

Leu Asp Gly Ala Asn Ala Ala Leu Val Leu Leu Lys Asn Glu Lys Lys
                405                 410                 415

Thr Leu Pro Leu Asn Pro Thr Asn Ile Gly Asn Leu Asn Ile Phe Gly
            420                 425                 430

Ile Gly Ser Lys Thr Gly Pro Leu Gly Ala Val Cys Gly Glu Asn Met
        435                 440                 445

Gln Cys Ser Asp Gly Ala Leu Ile Glu Gly Trp Gly Ser Gly Ser Val
    450                 455                 460

Tyr Pro Thr Asp Tyr Gln Ser Pro Tyr Asp Ala Ile Lys Glu Arg Ala
465                 470                 475                 480

Ser Lys Asp Asn Ile Thr Ile Gly Gly Thr Thr Gln Ser Trp Gly Asn
                485                 490                 495

Leu Ser Asn Val Glu Ile Leu Ser Ala Ala Ala Asp Ala Ser Val Val
            500                 505                 510

Phe Val Leu Ser Asp Ser Gly Glu Ser Thr Gly Ile Val Asp Gly Asn
        515                 520                 525

Ile Gly Asp Arg Asn Asn Leu Thr Leu Trp His Asn Gly Asp Glu Val
    530                 535                 540

Val Lys Ala Val Ala Ser Lys Asn Pro Asn Thr Ile Val Val Val Thr
545                 550                 555                 560

Thr Val Gly Pro Val Asn Leu Glu Lys Trp Ile Asp Asn Pro Asn Val
                565                 570                 575

Thr Ala Val Leu Leu Thr Gly Pro Ala Gly Asp Phe Gly Gly Arg Ala
            580                 585                 590

Ala Ala Ser Ile Leu Phe Gly Asp Ile Ala Pro Ser Gly Lys Leu Pro
        595                 600                 605

Phe Thr Ile Ala Lys Asn Asp Thr Asp Tyr Ile Pro Leu Thr Thr Lys
    610                 615                 620

Ile Pro Glu Asp Gly Leu Pro Gln Asp Tyr Phe Thr Glu Gly Thr Leu
625                 630                 635                 640

Leu Asp Tyr Lys Arg Phe Asp Glu Asn Gln Val Thr Pro Arg Phe Glu
                645                 650                 655

Phe Gly Tyr Gly Leu Ser Tyr Ser Asn Ile Thr Val Glu Asn Leu Glu
            660                 665                 670

Ala Arg Tyr Ala Phe Pro Ser Ile Pro Glu Phe Leu Pro Thr Pro Phe
        675                 680                 685

Ala Pro Ser Asn Pro Asn Lys Pro Lys Asn Ala Phe Thr Pro His Ala
    690                 695                 700

Asn Glu Ser Val Phe Pro Ser Asp Ile Asp Pro Leu Asn Lys Tyr Val
705                 710                 715                 720

Tyr Pro Tyr Leu Asn Asp Thr Ser Glu Ile Phe Ser Asn Glu Thr His
                725                 730                 735

Tyr Pro Tyr Pro Glu Gly Tyr Ser Ser Glu Gln Ser Asn Ser Thr Asn
            740                 745                 750

Ile Asn Gly Gly Ala Val Gly Gly Asn Pro Ala Leu Trp Leu Ser Ala
        755                 760                 765

Val Tyr Ile Val His Ser Val Ser Asn Tyr Gly Pro Tyr Asp Thr Gly
    770                 775                 780

Val Val Thr Gln Met Tyr Ile Ala Phe Pro Gln Asp Asn Asp Asp Leu
785                 790                 795                 800

Lys Thr Ala Pro Arg Gln Leu Arg Gly Phe Glu Arg Ser Glu Leu Lys
                805                 810                 815

Val Gly Glu Arg Gln Gly Ile Leu Tyr Asp Val Gln Trp Arg Asp Leu
            820                 825                 830

Ala Val Trp Asp Val Lys Leu Gln Ser Trp Arg Val Gln Arg Gly Glu
        835                 840                 845

Tyr Lys Val Tyr Val Gly His Ser Ser Arg Asp Phe Val Leu Thr Thr
    850                 855                 860

Ser Phe Thr Leu Lys
865

<210>  7
<211>  847
<212>  PRT
<213>  Yarrowia galli

<220>
<221>  SIGNAL
<222>  (1)..(16)

<400>  7

Met Ile Phe Ser Leu Gln Leu Leu Leu Thr Thr Val Pro Val Leu Ala
1               5                   10                  15

Ala Tyr Ser Pro Asp Trp Tyr Pro Thr Pro Glu Ile Gly Thr Ile Asn
            20                  25                  30

Asn Asp Trp Ala Asp Ala Leu Glu Gln Ser Met Asp Ile Leu Ser Gln
        35                  40                  45

Leu Thr Leu Pro Glu Lys Val Asn Ile Thr Thr Gly Thr Gly Trp Met
    50                  55                  60

Gly Gly Gln Cys Val Gly Asn Thr Gly Gly Val Pro Arg Leu Gly Ile
65                  70                  75                  80

Lys Gly Leu Cys Leu Gln Asp Gly Pro Leu Gly Val Arg Phe Ala Asp
                85                  90                  95

Phe Val Asn Val Phe Pro Cys Gln Asn Ala Met Ala Ala Thr Phe Asp
            100                 105                 110

Arg Ile Leu Val His Gln Arg Gly Thr Ala Ile Gly His Gln Ser Lys
        115                 120                 125

Leu Lys Gly Val Asp Val His Leu Gly Pro Val Val Gly Pro Ile Gly
    130                 135                 140

Arg His Ala Thr Gly Gly Arg Asn Trp Glu Gly Phe Ser Pro Asp Pro
145                 150                 155                 160

Tyr Leu Ser Gly Lys Leu Ala Tyr Glu Ala Ile Gln Gly Ile Gln Glu
                165                 170                 175

Glu Asn Val Leu Ala Thr Ile Lys His Phe Ile Gly Asn Glu Gln Asp
            180                 185                 190

His Tyr Arg Arg Ala Gln Glu Trp Arg Asp Gly Phe Asn Phe Thr Gly
        195                 200                 205

Leu Lys Leu Pro Val Ser Ser Asn Asn Ile Asp Asp Arg Ala Leu His
    210                 215                 220

Glu Leu Tyr Met Trp Pro Phe Ala Asp Ala Val Lys Ala Gly Val Gly
225                 230                 235                 240

Ser Val Met Cys Ala Tyr Asn Asn Val Asn Gly Thr Gln Ala Cys Gln
                245                 250                 255

Asn Ser Asp Leu Leu Asn Gly Lys Leu Lys Ser Glu Leu Gly Phe Gln
            260                 265                 270

Gly Phe Val Met Ser Asp Trp Phe Ala Gln Gly Asn Gly Val Ala Asn

275                 280                 285

Ala Leu Ala Gly Met Asp Met Ser Met Pro Gly Thr Asp Val Asp Glu
    290                 295                 300

Phe Glu Thr Val Phe Trp Gly Glu Gln Leu Thr Arg Met Val Ala Asn
305                 310                 315                 320

Gly Thr Leu Pro Glu Ser Arg Leu Asp Asp Met Val Leu Arg Ile Leu
                325                 330                 335

Thr Pro Leu Met Tyr Phe Gly Ile Asp Asp Arg Glu Pro Asn Phe Ala
            340                 345                 350

Ser Phe Val Asp Thr Thr Val Gly Ser Pro Tyr Pro Ala Ala Lys Asn
        355                 360                 365

Pro Lys Gln Val Gln Asp Lys Ile Val Asn Tyr His Leu Asp Val Arg
    370                 375                 380

Asn Gln Phe Ser Ala Asn Val Ala Leu Glu Ser Ala Arg Gly Ala Ile
385                 390                 395                 400

Val Leu Leu Phe Asn Ser Gly Val Leu Pro Leu Lys Asp Lys Ser Asn
                405                 410                 415

Ile Gly Val Phe Gly Val Gly Ser Val Ile Gly Pro Asn Gly Ala Ile
            420                 425                 430

Cys Glu Asn Met Gln Cys Ser Asp Gly Ala Leu Ile Glu Gly Trp Gly
        435                 440                 445

Ser Gly Thr Ala Tyr Pro Thr Glu Tyr Glu Ser Pro Tyr Glu Ala Leu
    450                 455                 460

His Lys Lys Ala Ser Leu Leu Asp Val His Val Thr Gly Thr Ser Glu
465                 470                 475                 480

Ser Trp Asp Met Ser Leu Pro Ile Glu Ile Ala Gly Ser Thr Asp Ile
                485                 490                 495

Asn Ile Val Tyr Val Leu Ala Ala Ser Gly Glu Ser Thr Ala Ser Val
            500                 505                 510

Asp Gly Asn Ile Gly Asp Arg Asn Asn Val Ser Leu Trp His Asn Gly
        515                 520                 525

Asp Glu Leu Ile Asn Ala Val Ala Asp Gln Gly Glu Thr Val Val Val
        530                 535                 540

Val Thr Thr Val Gly Gln Val Asp Met Ser Glu Trp Leu Phe His Pro
545                 550                 555                 560

Asn Val Ser Ala Val Leu Leu Thr Ala Pro Ala Gly Asp Tyr Gly Gly
                565                 570                 575

Lys Ala Met Ala Asp Val Leu Phe Gly Glu Val Asn Pro Ser Gly Lys
            580                 585                 590

Leu Pro Tyr Thr Leu Ala Asp Asp Ile Ser Glu Tyr Ile Pro Ile Val
        595                 600                 605

Thr Glu Ile Pro Ala Asp Gly Ala Pro Gln Ser Asp Phe Val Glu Gly
    610                 615                 620

Ile Tyr Leu Asp Tyr Lys Trp Tyr Asp Lys Phe Glu Lys Thr Pro Leu
625                 630                 635                 640

Tyr Glu Phe Gly Tyr Gly Leu Ser Tyr Thr Asn Tyr Thr Phe Thr Asp
                645                 650                 655

Leu Ser Leu Asp Ile Lys Glu Ile Asp Glu Phe Leu Pro Ala Arg Pro
            660                 665                 670

Asp Pro Val Val Val Thr Lys Pro Glu Asp Phe Pro Ile Asp Leu Ala
        675                 680                 685

Asp Leu Tyr Val Pro Asp Gly Phe Glu Met Ile Asp Gly Leu Val Tyr
    690                 695                 700

Pro Trp Ile Gln Asn Val Ser Val Pro Leu Ser Asp Ser Glu Ala Gln
705                 710                 715                 720

Phe Pro Phe Ala Asp Gly Ala Gly His Ile Ser Asp Ala Ser Gly Gly
                725                 730                 735

Val Gly Gly His Pro Trp Leu Trp Ser Val Ala Ala Thr Ile Thr His
            740                 745                 750

Asn Thr Thr Asn Cys Gly Asp Val Ala Gly Arg Ala Val Ser Gln Leu
        755                 760                 765

Tyr Ile Ala Phe Pro Glu Thr Cys Val Asp Val Pro Leu Val Gln Leu
    770                 775                 780

Arg Gly Phe Asp Lys Ser Gly Met Leu Asn Pro Gly Glu Ser Gln Glu
785                 790                 795                 800

Thr Val Tyr Asn Leu His Trp Arg Asp Leu Ala Ile Trp Asp Val Glu
                805                 810                 815

Leu Gln Ser Trp Arg Val Gln Arg Gly Glu Tyr Ala Val Phe Ile Gly
            820                 825                 830

His Ser Ser Arg Asp Phe Glu Leu Phe Asp Ser Phe Thr Leu Glu
        835                 840                 845

<210>  8
<211>  831
<212>  PRT
<213>  Yarrowia galli

<400>  8

Ala Tyr Ser Pro Asp Trp Tyr Pro Thr Pro Glu Ile Gly Thr Ile Asn
1               5                   10                  15

Asn Asp Trp Ala Asp Ala Leu Glu Gln Ser Met Asp Ile Leu Ser Gln
            20                  25                  30

Leu Thr Leu Pro Glu Lys Val Asn Ile Thr Thr Gly Thr Gly Trp Met
        35                  40                  45

Gly Gly Gln Cys Val Gly Asn Thr Gly Gly Val Pro Arg Leu Gly Ile
    50                  55                  60

Lys Gly Leu Cys Leu Gln Asp Gly Pro Leu Gly Val Arg Phe Ala Asp
65                  70                  75                  80

Phe Val Asn Val Phe Pro Cys Gln Asn Ala Met Ala Ala Thr Phe Asp
                85                  90                  95

Arg Ile Leu Val His Gln Arg Gly Thr Ala Ile Gly His Gln Ser Lys
            100                 105                 110

Leu Lys Gly Val Asp Val His Leu Gly Pro Val Val Gly Pro Ile Gly
        115                 120                 125

Arg His Ala Thr Gly Gly Arg Asn Trp Glu Gly Phe Ser Pro Asp Pro
    130                 135                 140

Tyr Leu Ser Gly Lys Leu Ala Tyr Glu Ala Ile Gln Gly Ile Gln Glu
145                 150                 155                 160

Glu Asn Val Leu Ala Thr Ile Lys His Phe Ile Gly Asn Glu Gln Asp
                165                 170                 175

His Tyr Arg Arg Ala Gln Glu Trp Arg Asp Gly Phe Asn Phe Thr Gly
            180                 185                 190

Leu Lys Leu Pro Val Ser Ser Asn Asn Ile Asp Asp Arg Ala Leu His
        195                 200                 205

Glu Leu Tyr Met Trp Pro Phe Ala Asp Ala Val Lys Ala Gly Val Gly
    210                 215                 220

Ser Val Met Cys Ala Tyr Asn Asn Val Asn Gly Thr Gln Ala Cys Gln
225                 230                 235                 240

Asn Ser Asp Leu Leu Asn Gly Lys Leu Lys Ser Glu Leu Gly Phe Gln
                245                 250                 255

Gly Phe Val Met Ser Asp Trp Phe Ala Gln Gly Asn Gly Val Ala Asn
            260                 265                 270

Ala Leu Ala Gly Met Asp Met Ser Met Pro Gly Thr Asp Val Asp Glu
        275                 280                 285

Phe Glu Thr Val Phe Trp Gly Glu Gln Leu Thr Arg Met Val Ala Asn
    290                 295                 300

Gly Thr Leu Pro Glu Ser Arg Leu Asp Asp Met Val Leu Arg Ile Leu
305                 310                 315                 320

Thr Pro Leu Met Tyr Phe Gly Ile Asp Asp Arg Glu Pro Asn Phe Ala
                325                 330                 335

Ser Phe Val Asp Thr Thr Val Gly Ser Pro Tyr Pro Ala Ala Lys Asn
            340                 345                 350

Pro Lys Gln Val Gln Asp Lys Ile Val Asn Tyr His Leu Asp Val Arg
        355                 360                 365

Asn Gln Phe Ser Ala Asn Val Ala Leu Glu Ser Ala Arg Gly Ala Ile
    370                 375                 380

Val Leu Leu Phe Asn Ser Gly Val Leu Pro Leu Lys Asp Lys Ser Asn
385                 390                 395                 400

Ile Gly Val Phe Gly Val Gly Ser Val Ile Gly Pro Asn Gly Ala Ile
                405                 410                 415

Cys Glu Asn Met Gln Cys Ser Asp Gly Ala Leu Ile Glu Gly Trp Gly
            420                 425                 430

Ser Gly Thr Ala Tyr Pro Thr Glu Tyr Glu Ser Pro Tyr Glu Ala Leu
        435                 440                 445

His Lys Lys Ala Ser Leu Leu Asp Val His Val Thr Gly Thr Ser Glu
    450                 455                 460

Ser Trp Asp Met Ser Leu Pro Ile Glu Ile Ala Gly Ser Thr Asp Ile
465                 470                 475                 480

Asn Ile Val Tyr Val Leu Ala Ala Ser Gly Glu Ser Thr Ala Ser Val
                485                 490                 495

Asp Gly Asn Ile Gly Asp Arg Asn Asn Val Ser Leu Trp His Asn Gly
            500                 505                 510

Asp Glu Leu Ile Asn Ala Val Ala Asp Gln Gly Glu Thr Val Val Val
        515                 520                 525

Val Thr Thr Val Gly Gln Val Asp Met Ser Glu Trp Leu Phe His Pro
    530                 535                 540

Asn Val Ser Ala Val Leu Leu Thr Ala Pro Ala Gly Asp Tyr Gly Gly
545                 550                 555                 560

Lys Ala Met Ala Asp Val Leu Phe Gly Glu Val Asn Pro Ser Gly Lys
                565                 570                 575

Leu Pro Tyr Thr Leu Ala Asp Asp Ile Ser Glu Tyr Ile Pro Ile Val
            580                 585                 590

Thr Glu Ile Pro Ala Asp Gly Ala Pro Gln Ser Asp Phe Val Glu Gly
        595                 600                 605

Ile Tyr Leu Asp Tyr Lys Trp Tyr Asp Lys Phe Glu Lys Thr Pro Leu
    610                 615                 620

Tyr Glu Phe Gly Tyr Gly Leu Ser Tyr Thr Asn Tyr Thr Phe Thr Asp
625                 630                 635                 640

Leu Ser Leu Asp Ile Lys Glu Ile Asp Glu Phe Leu Pro Ala Arg Pro
                645                 650                 655

Asp Pro Val Val Val Thr Lys Pro Glu Asp Phe Pro Ile Asp Leu Ala

660                 665                 670

Asp Leu Tyr Val Pro Asp Gly Phe Glu Met Ile Asp Gly Leu Val Tyr
        675                 680                 685

Pro Trp Ile Gln Asn Val Ser Val Pro Leu Ser Asp Ser Glu Ala Gln
    690                 695                 700

Phe Pro Phe Ala Asp Gly Ala Gly His Ile Ser Asp Ala Ser Gly Gly
705                 710                 715                 720

Val Gly Gly His Pro Trp Leu Trp Ser Val Ala Ala Thr Ile Thr His
                725                 730                 735

Asn Thr Thr Asn Cys Gly Asp Val Ala Gly Arg Ala Val Ser Gln Leu
            740                 745                 750

Tyr Ile Ala Phe Pro Glu Thr Cys Val Asp Val Pro Leu Val Gln Leu
        755                 760                 765

Arg Gly Phe Asp Lys Ser Gly Met Leu Asn Pro Gly Glu Ser Gln Glu
    770                 775                 780

Thr Val Tyr Asn Leu His Trp Arg Asp Leu Ala Ile Trp Asp Val Glu
785                 790                 795                 800

Leu Gln Ser Trp Arg Val Gln Arg Gly Glu Tyr Ala Val Phe Ile Gly
                805                 810                 815

His Ser Ser Arg Asp Phe Glu Leu Phe Asp Ser Phe Thr Leu Glu
            820                 825                 830

<210>  9
<211>  854
<212>  PRT
<213>  Artificial Sequence

<220>
<223>  BGL2 consensus sequence


<220>
<221>  VARIANT
<222>  (1)..(1)
<223>  Replace = Phe

<220>
<221>  VARIANT
<222>  (5)..(5)
<223>  Replace = Ala

<220>

<221>  VARIANT
<222>  (7)..(7)
<223>  Replace = Arg

<220>
<221>  VARIANT
<222>  (8)..(8)
<223>  Replace = Ala

<220>
<221>  VARIANT
<222>  (9)..(9)
<223>  Replace = Ser

<220>
<221>  VARIANT
<222>  (25)..(25)
<223>  Replace = Ile

<220>
<221>  VARIANT
<222>  (26)..(26)
<223>  Replace = Leu

<220>
<221>  VARIANT
<222>  (29)..(29)
<223>  Replace = Arg

<220>
<221>  VARIANT
<222>  (32)..(32)
<223>  Replace = Ala

<220>
<221>  VARIANT
<222>  (33)..(33)
<223>  Replace = Thr

<220>
<221>  VARIANT
<222>  (35)..(35)
<223>  Replace = Met

<220>
<221>  VARIANT
<222>  (36)..(36)
<223>  Replace = Glu or Asn

<220>
<221>  VARIANT
<222>  (37)..(37)
<223>  Replace = Ala

<220>
<221>  VARIANT
<222>  (40)..(40)
<223>  Replace = Gly, Asn or Ser

<220>
<221>  VARIANT
<222>  (41)..(41)
<223>  Replace = Leu

<220>
<221>  VARIANT
<222>  (48)..(48)
<223>  Replace = Leu or Tyr

<220>
<221>  VARIANT
<222>  (55)..(55)
<223>  Replace = Ser

<220>
<221>  VARIANT
<222>  (69)..(69)
<223>  Replace = Ser

<220>
<221>  VARIANT
<222>  (70)..(70)
<223>  Replace = Pro

<220>
<221>  VARIANT
<222>  (79)..(79)
<223>  Replace = Ile

<220>
<221>  VARIANT
<222>  (88)..(88)
<223>  Replace = Val

<220>
<221>  VARIANT
<222>  (90)..(90)
<223>  Replace = Leu

<220>
<221>  VARIANT
<222>  (91)..(91)
<223>  Replace = Thr

<220>
<221>  VARIANT
<222>  (93)..(93)
<223>  Replace = Lys

<220>
<221>  VARIANT
<222>  (96)..(96)
<223>  Replace = Ala

<220>
<221>  VARIANT
<222>  (103)..(103)
<223>  Replace = Met

<220>
<221>  VARIANT
<222>  (109)..(109)
<223>  Replace = Arg

<220>
<221>  VARIANT

<222>  (110)..(110)
<223>  Replace = Lys

<220>
<221>  VARIANT
<222>  (113)..(113)
<223>  Replace = Gln

<220>
<221>  VARIANT
<222>  (117)..(117)
<223>  Replace = Ala

<220>
<221>  VARIANT
<222>  (142)..(142)
<223>  Replace = Asn

<220>
<221>  VARIANT
<222>  (145)..(145)
<223>  Replace = Gly

<220>
<221>  VARIANT
<222>  (165)..(165)
<223>  Replace = Gln

<220>
<221>  VARIANT
<222>  (168)..(168)
<223>  Replace = Glu

<220>
<221>  VARIANT
<222>  (172)..(172)
<223>  Replace = Gly

<220>
<221>  VARIANT
<222>  (182)..(182)
<223>  Replace = Met or Leu

<220>
<221>  VARIANT
<222>  (199)..(199)
<223>  Replace = Tyr

<220>
<221>  VARIANT
<222>  (201)..(201)
<223>  Replace = Tyr

<220>
<221>  VARIANT
<222>  (217)..(217)
<223>  Replace = Met

<220>
<221>  VARIANT
<222>  (229)..(229)
<223>  Replace = Ile

<220>
<221>  VARIANT
<222>  (245)..(245)
<223>  Replace = Ser

<220>
<221>  VARIANT
<222>  (252)..(252)
<223>  Replace = Ala

<220>
<221>  VARIANT
<222>  (253)..(253)
<223>  Replace = Phe or Ala

<220>
<221>  VARIANT
<222>  (254)..(254)
<223>  Replace = Leu

<220>
<221>  VARIANT
<222>  (259)..(259)
<223>  Replace = Ile

<220>
<221>  VARIANT
<222>  (263)..(263)
<223>  Replace = Phe

<220>
<221>  VARIANT
<222>  (269)..(269)
<223>  Replace = Ile

<220>
<221>  VARIANT
<222>  (299)..(299)
<223>  Replace = Gly

<220>
<221>  VARIANT
<222>  (300)..(300)
<223>  Replace = Leu

<220>
<221>  VARIANT
<222>  (302)..(302)
<223>  Replace = Ile

<220>
<221>  VARIANT
<222>  (311)..(311)
<223>  Replace = Arg

<220>
<221>  VARIANT
<222>  (312)..(312)
<223>  Replace = Met

<220>
<221>  VARIANT
<222>  (318)..(318)

<223>  Replace = Val

<220>
<221>  VARIANT
<222>  (319)..(319)
<223>  Replace = Glu

<220>
<221>  VARIANT
<222>  (320)..(320)
<223>  Replace = Glu or Gln

<220>
<221>  VARIANT
<222>  (321)..(321)
<223>  Replace = Ser

<220>
<221>  VARIANT
<222>  (334)..(334)
<223>  Replace = Ile

<220>
<221>  VARIANT
<222>  (335)..(335)
<223>  Replace = Leu

<220>
<221>  VARIANT
<222>  (339)..(339)
<223>  Replace = Leu

<220>
<221>  VARIANT
<222>  (342)..(342)
<223>  Replace = Thr or Arg

<220>
<221>  VARIANT
<222>  (343)..(343)
<223>  Replace = Asp or Thr

<220>
<221>  VARIANT
<222>  (352)..(352)
<223>  Replace = Glu

<220>
<221>  VARIANT
<222>  (354)..(354)
<223>  Replace = Tyr

<220>
<221>  VARIANT
<222>  (355)..(355)
<223>  Replace = Gly

<220>
<221>  VARIANT
<222>  (356)..(356)
<223>  Replace = Leu

<220>

<221>  VARIANT
<222>  (357)..(357)
<223>  Replace = Glu or Gln

<220>
<221>  VARIANT
<222>  (358)..(358)
<223>  Replace = Tyr or Phe

<220>
<221>  VARIANT
<222>  (359)..(359)
<223>  Replace = Pro

<220>
<221>  VARIANT
<222>  (361)..(361)
<223>  Replace = Asn

<220>
<221>  VARIANT
<222>  (362)..(362)
<223>  Replace = Asp

<220>
<221>  VARIANT
<222>  (363)..(363)
<223>  Replace = Gly or Arg

<220>
<221>  VARIANT
<222>  (365)..(365)
<223>  Replace = Arg or Pro

<220>
<221>  VARIANT
<222>  (367)..(367)
<223>  Replace = Ile

<220>
<221>  VARIANT
<222>  (369)..(369)
<223>  Replace = Ile

<220>
<221>  VARIANT
<222>  (371)..(371)
<223>  Replace = Gln

<220>
<221>  VARIANT
<222>  (373)..(373)
<223>  Replace = Val

<220>
<221>  VARIANT
<222>  (374)..(374)
<223>  Replace = Glu

<220>
<221>  VARIANT
<222>  (378)..(378)
<223>  Replace = Gln, Ala or Ser

<220>
<221>  VARIANT
<222>  (379)..(379)
<223>  Replace = Tyr

<220>
<221>  VARIANT
<222>  (381)..(381)
<223>  Replace = Ile

<220>
<221>  VARIANT
<222>  (382)..(382)
<223>  Replace = Gln

<220>
<221>  VARIANT
<222>  (385)..(385)
<223>  Replace = Gln

<220>
<221>  VARIANT
<222>  (389)..(389)
<223>  Replace = Thr

<220>
<221>  VARIANT
<222>  (398)..(398)
<223>  Replace = Glu

<220>
<221>  VARIANT
<222>  (399)..(399)
<223>  Replace = Gln

<220>
<221>  VARIANT
<222>  (400)..(400)
<223>  Replace = Lys or Ser

<220>
<221>  VARIANT
<222>  (401)..(401)
<223>  Replace = Thr

<220>
<221>  VARIANT
<222>  (405)..(405)
<223>  Replace = Asn or Asp

<220>
<221>  VARIANT
<222>  (406)..(406)
<223>  Replace = nothing

<220>
<221>  VARIANT
<222>  (407)..(407)
<223>  Replace = Ala or nothing

<220>
<221>  VARIANT

<222>  (408)..(408)
<223>  Replace = Lys

<220>
<221>  VARIANT
<222>  (409)..(409)
<223>  Replace = Leu or Val

<220>
<221>  VARIANT
<222>  (410)..(410)
<223>  Replace = Thr

<220>
<221>  VARIANT
<222>  (411)..(411)
<223>  Replace = Thr or Lys

<220>
<221>  VARIANT
<222>  (412)..(412)
<223>  Replace = Ile

<220>
<221>  VARIANT
<222>  (417)..(417)
<223>  Replace = Val

<220>
<221>  VARIANT
<222>  (420)..(420)
<223>  Replace = Lys or Thr

<220>
<221>  VARIANT
<222>  (423)..(423)
<223>  Replace = Asn

<220>
<221>  VARIANT
<222>  (425)..(425)
<223>  Replace = Ser

<220>
<221>  VARIANT
<222>  (426)..(426)
<223>  Replace = nothing

<220>
<221>  VARIANT
<222>  (427)..(427)
<223>  Replace = nothing

<220>
<221>  VARIANT
<222>  (430)..(430)
<223>  Replace = Gln

<220>
<221>  VARIANT
<222>  (433)..(433)
<223>  Replace = Glu

<220>
<221>  VARIANT
<222>  (436)..(436)
<223>  Replace = Ser

<220>
<221>  VARIANT
<222>  (441)..(441)
<223>  Replace = Gln or Leu

<220>
<221>  VARIANT
<222>  (451)..(451)
<223>  Replace = Ser

<220>
<221>  VARIANT
<222>  (454)..(454)
<223>  Replace = Leu

<220>
<221>  VARIANT
<222>  (458)..(458)
<223>  Replace = Asp or Asn

<220>
<221>  VARIANT
<222>  (465)..(465)
<223>  Replace = Ser or Glu

<220>
<221>  VARIANT
<222>  (466)..(466)
<223>  Replace = Lys

<220>
<221>  VARIANT
<222>  (467)..(467)
<223>  Replace = Asn

<220>
<221>  VARIANT
<222>  (468)..(468)
<223>  Replace = Lys

<220>
<221>  VARIANT
<222>  (470)..(470)
<223>  Replace = Gln

<220>
<221>  VARIANT
<222>  (471)..(471)
<223>  Replace = Ile

<220>
<221>  VARIANT
<222>  (476)..(476)
<223>  Replace = His or Glu

<220>
<221>  VARIANT
<222>  (482)..(482)

<223>  Replace = Thr

<220>
<221>  VARIANT
<222>  (483)..(483)
<223>  Replace = Ile or Arg

<220>
<221>  VARIANT
<222>  (486)..(486)
<223>  Replace = Lys or Gln

<220>
<221>  VARIANT
<222>  (488)..(488)
<223>  Replace = Ala

<220>
<221>  VARIANT
<222>  (490)..(490)
<223>  Replace = Glu

<220>
<221>  VARIANT
<222>  (491)..(491)
<223>  Replace = Ser

<220>
<221>  VARIANT
<222>  (495)..(495)
<223>  Replace = Ile

<220>
<221>  VARIANT
<222>  (508)..(508)
<223>  Replace = Pro

<220>
<221>  VARIANT
<222>  (513)..(513)
<223>  Replace = Tyr

<220>
<221>  VARIANT
<222>  (527)..(527)
<223>  Replace = Glu or Gln

<220>
<221>  VARIANT
<222>  (530)..(530)
<223>  Replace = Arg

<220>
<221>  VARIANT
<222>  (531)..(531)
<223>  Replace = Thr

<220>
<221>  VARIANT
<222>  (535)..(535)
<223>  Replace = Lys or Gln

<220>

<221>  VARIANT
<222>  (536)..(536)
<223>  Replace = Asn

<220>
<221>  VARIANT
<222>  (537)..(537)
<223>  Replace = Ala

<220>
<221>  VARIANT
<222>  (556)..(556)
<223>  Replace = Asp

<220>
<221>  VARIANT
<222>  (557)..(557)
<223>  Replace = His

<220>
<221>  VARIANT
<222>  (575)..(575)
<223>  Replace = Ser or Lys

<220>
<221>  VARIANT
<222>  (580)..(580)
<223>  Replace = Val

<220>
<221>  VARIANT
<222>  (585)..(585)
<223>  Replace = Val

<220>
<221>  VARIANT
<222>  (597)..(597)
<223>  Replace = Arg

<220>
<221>  VARIANT
<222>  (599)..(599)
<223>  Replace = Asn

<220>
<221>  VARIANT
<222>  (603)..(603)
<223>  Replace = Val

<220>
<221>  VARIANT
<222>  (606)..(606)
<223>  Replace = Val

<220>
<221>  VARIANT
<222>  (609)..(609)
<223>  Replace = Ile

<220>
<221>  VARIANT
<222>  (611)..(611)
<223>  Replace = Glu or Ser

<220>
<221>  VARIANT
<222>  (618)..(618)
<223>  Replace = Phe

<220>
<221>  VARIANT
<222>  (632)..(632)
<223>  Replace = Ala

<220>
<221>  VARIANT
<222>  (633)..(633)
<223>  Replace = Leu

<220>
<221>  VARIANT
<222>  (634)..(634)
<223>  Replace = Gln

<220>
<221>  VARIANT
<222>  (635)..(635)
<223>  Replace = Lys

<220>
<221>  VARIANT
<222>  (652)..(652)
<223>  Replace = Val or Leu

<220>
<221>  VARIANT
<222>  (653)..(653)
<223>  Replace = Gly

<220>
<221>  VARIANT
<222>  (656)..(656)
<223>  Replace = Asp or Gln

<220>
<221>  VARIANT
<222>  (657)..(657)
<223>  Replace = Val

<220>
<221>  VARIANT
<222>  (658)..(658)
<223>  Replace = Leu or Lys

<220>
<221>  VARIANT
<222>  (659)..(659)
<223>  Replace = Tyr

<220>
<221>  VARIANT
<222>  (662)..(662)
<223>  Replace = Asn

<220>
<221>  VARIANT

<222>  (663)..(663)
<223>  Replace = Ser

<220>
<221>  VARIANT
<222>  (665)..(665)
<223>  Replace = Ser

<220>
<221>  VARIANT
<222>  (670)..(670)
<223>  Replace = Ala

<220>
<221>  VARIANT
<222>  (672)..(672)
<223>  Replace = Ser

<220>
<221>  VARIANT
<222>  (673)..(673)
<223>  Replace = Pro

<220>
<221>  VARIANT
<222>  (675)..(675)
<223>  Replace = Ser

<220>
<221>  VARIANT
<222>  (676)..(676)
<223>  Replace = Ile or Asp

<220>
<221>  VARIANT
<222>  (678)..(678)
<223>  Replace = Asn

<220>
<221>  VARIANT
<222>  (681)..(681)
<223>  Replace = Lys or Ala

<220>
<221>  VARIANT
<222>  (682)..(682)
<223>  Replace = Asn

<220>
<221>  VARIANT
<222>  (683)..(683)
<223>  Replace = Ala or Arg

<220>
<221>  VARIANT
<222>  (685)..(685)
<223>  Replace = Pro

<220>
<221>  VARIANT
<222>  (686)..(686)
<223>  Replace = Thr

<220>
<221>  VARIANT
<222>  (687)..(687)
<223>  Replace = Hir, Ser or Glu

<220>
<221>  VARIANT
<222>  (688)..(688)
<223>  Replace = Leu

<220>
<221>  VARIANT
<222>  (690)..(690)
<223>  Replace = Glu or Gly

<220>
<221>  VARIANT
<222>  (692)..(692)
<223>  Replace = Ile

<220>
<221>  VARIANT
<222>  (695)..(695)
<223>  Replace = Ser

<220>
<221>  VARIANT
<222>  (696)..(696)
<223>  Replace = Glu

<220>
<221>  VARIANT
<222>  (697)..(697)
<223>  Replace = Phe

<220>
<221>  VARIANT
<222>  (698)..(698)
<223>  Replace = Asp or Lys

<220>
<221>  VARIANT
<222>  (699)..(699)
<223>  Replace = Gln

<220>
<221>  VARIANT
<222>  (700)..(700)
<223>  Replace = Ile

<220>
<221>  VARIANT
<222>  (701)..(701)
<223>  Replace = Asn, Lys or Pro

<220>
<221>  VARIANT
<222>  (702)..(702)
<223>  Replace = Asn

<220>
<221>  VARIANT
<222>  (706)..(706)

<223>  Replace = Thr

<220>
<221>  VARIANT
<222>  (710)..(710)
<223>  Replace = Arg or Ser

<220>
<221>  VARIANT
<222>  (713)..(713)
<223>  Replace = Asp

<220>
<221>  VARIANT
<222>  (714)..(714)
<223>  Replace = Leu

<220>
<221>  VARIANT
<222>  (715)..(715)
<223>  Replace = Ala or Asn

<220>
<221>  VARIANT
<222>  (716)..(716)
<223>  Replace = nothing

<220>
<221>  VARIANT
<222>  (719)..(719)
<223>  Replace = Ile

<220>
<221>  VARIANT
<222>  (720)..(720)
<223>  Replace = Ser

<220>
<221>  VARIANT
<222>  (721)..(721)
<223>  Replace = His or Arg

<220>
<221>  VARIANT
<222>  (728)..(728)
<223>  Replace = Phe

<220>
<221>  VARIANT
<222>  (730)..(730)
<223>  Replace = Ser or Val

<220>
<221>  VARIANT
<222>  (731)..(731)
<223>  Replace = Glu or Val

<220>
<221>  VARIANT
<222>  (735)..(735)
<223>  Replace = Ser or Gly

<220>

<221>  VARIANT
<222>  (752)..(752)
<223>  Replace = Pro or Thr

<220>
<221>  VARIANT
<222>  (753)..(753)
<223>  Replace = Ala

<220>
<221>  VARIANT
<222>  (754)..(754)
<223>  Replace = Leu

<220>
<221>  VARIANT
<222>  (756)..(756)
<223>  Replace = Val

<220>
<221>  VARIANT
<222>  (757)..(757)
<223>  Replace = Ala

<220>
<221>  VARIANT
<222>  (760)..(760)
<223>  Replace = Ile

<220>
<221>  VARIANT
<222>  (763)..(763)
<223>  Replace = His

<220>
<221>  VARIANT
<222>  (770)..(770)
<223>  Replace = Ala

<220>
<221>  VARIANT
<222>  (774)..(774)
<223>  Replace = Leu

<220>
<221>  VARIANT
<222>  (777)..(777)
<223>  Replace = Ser

<220>
<221>  VARIANT
<222>  (783)..(783)
<223>  Replace = Glu

<220>
<221>  VARIANT
<222>  (784)..(784)
<223>  Replace = Gly

<220>
<221>  VARIANT
<222>  (786)..(786)
<223>  Replace = Arg or Gln

<220>
<221>  VARIANT
<222>  (801)..(801)
<223>  Replace = Pro

<220>
<221>  VARIANT
<222>  (804)..(804)
<223>  Replace = Asp

<220>
<221>  VARIANT
<222>  (805)..(805)
<223>  Replace = Gln

<220>
<221>  VARIANT
<222>  (806)..(806)
<223>  Replace = Gln or Arg

<220>
<221>  VARIANT
<222>  (808)..(808)
<223>  Replace = Val

<220>
<221>  VARIANT
<222>  (809)..(809)
<223>  Replace = Gln

<220>
<221>  VARIANT
<222>  (811)..(811)
<223>  Replace = Asp

<220>
<221>  VARIANT
<222>  (812)..(812)
<223>  Replace = Ile

<220>
<221>  VARIANT
<222>  (823)..(823)
<223>  Replace = Ser

<220>
<221>  VARIANT
<222>  (824)..(824)
<223>  Replace = Ile

<220>
<221>  VARIANT
<222>  (833)..(833)
<223>  Replace = Lys or Asp

<220>
<221>  VARIANT
<222>  (835)..(835)
<223>  Replace = Thr or Glu

<220>
<221>  VARIANT

<222>  (836)..(836)
<223>  Replace = Ile

<220>
<221>  VARIANT
<222>  (837)..(837)
<223>  Replace = Phe

<220>
<221>  VARIANT
<222>  (838)..(838)
<223>  Replace = Ile

<220>
<221>  VARIANT
<222>  (845)..(845)
<223>  Replace = Leu

<220>
<221>  VARIANT
<222>  (848)..(848)
<223>  Replace = Ser or Ala

<220>
<221>  VARIANT
<222>  (850)..(850)
<223>  Replace = Thr or Lys

<220>
<221>  VARIANT
<222>  (854)..(854)
<223>  Replace = Lys or Asn

<400>  9

Ala Asp Pro Phe Ser Asp Lys Asp Ala Tyr Lys His Ser Pro Pro Tyr
1               5                   10                  15

Tyr Pro Ala Pro Glu Ile Gly Arg Val Pro Thr Asp Leu Arg Trp Arg
            20                  25                  30

Ala Ala Leu Lys Val Ala Gln Asp Met Val Ala Asn Met Thr Leu Ile
        35                  40                  45

Glu Lys Val Asn Ile Thr Thr Gly Thr Gly Trp Glu Met Gly Pro Cys
    50                  55                  60

Val Gly Asn Thr Gly Thr Val Glu Arg Leu Gly Ile Lys Ser Leu Cys
65                  70                  75                  80

Leu Gln Asp Gly Pro Leu Gly Ile Arg Phe Ala Asp Leu Ile Thr Thr
                85                  90                  95

Phe Pro Ala Gly Ile Thr Ile Ala Ser Thr Phe Ser Lys Gln Leu Val
            100                 105                 110

Arg Glu Arg Gly Glu Ala Met Gly Arg Glu Asn Arg Arg Lys Gly Val
        115                 120                 125

Asp Ile Thr Leu Ser Pro Val Val Gly Pro Leu Gly Arg His Ala Asn
    130                 135                 140

Ala Gly Arg Ile Trp Glu Gly Phe Ser Ala Asp Pro Tyr Leu Ala Gly
145                 150                 155                 160

Lys Leu Ala Ala Glu Ala Val Thr Gly Ile Gln Ser Gln Asn Val Met
                165                 170                 175

Ala Val Val Lys His Ile Val Gly Asn Glu Gln Glu His Phe Arg Gln
            180                 185                 190

Leu Gly Glu Trp Gln Gly Phe Gly Phe Lys Asp Leu Lys Gln Pro Leu
        195                 200                 205

Ser Ser Asn Ile Asp Asp Arg Thr Leu Asn Glu Ala Tyr Leu Trp Pro
    210                 215                 220

Phe Ala Asp Ala Val Arg Ala Asn Val Gly Ser Val Met Cys Ser Tyr
225                 230                 235                 240

Gln Gln Ile Asn Gly Ser Gln Gly Cys Gln Asn Ser His Ile Leu Asn
                245                 250                 255

Gly Lys Leu Lys Glu Glu Met Gly Phe Gln Gly Phe Val Met Ser Asp
            260                 265                 270

Trp Leu Ala Gln Arg Ser Gly Val Ala Ser Val Leu Ala Gly Leu Asp
        275                 280                 285

Met Ser Met Pro Gly Asp Gly Leu Val Trp Ala Asp Gly Val Pro Leu
    290                 295                 300

Met Gly Tyr Glu Leu Thr Lys Ser Val Leu Asn Gly Thr Ile Asp Ile
305                 310                 315                 320

Asn Arg Val Asp Asp Met Val Thr Arg Ile Leu Thr Pro Leu Met Tyr
                325                 330                 335

Leu Ser Ile Thr Pro Gly Asn Pro Asn Phe Ser Ser Trp Thr Asn Asp
            340                 345                 350

Thr Thr Ser Tyr Lys Ile Tyr Gly Ala Lys Ala Gly Gly Asn Val Thr
        355                 360                 365

Val Asn Arg His Ile Asp Val Arg Asp Pro Trp Thr Thr Lys Ala Ala
    370                 375                 380

Leu Asp Gly Ala Asn Ala Ala Leu Val Leu Leu Lys Asn Val Lys Asn
385                 390                 395                 400

Ala Leu Pro Leu Lys Pro Thr Asn Ile Gly Asn Leu Asn Ile Phe Gly
                405                 410                 415

Ile Gly Ser Ala Thr Gly Pro Leu Gly Ala Val Cys Gly Glu Asn Met
            420                 425                 430

Gln Cys Ser Asp Gly Ala Leu Ile Glu Gly Trp Gly Ser Gly Ser Val
        435                 440                 445

Tyr Pro Thr Asp Tyr Gln Ser Pro Tyr Glu Ala Ile Lys Glu Arg Ala
    450                 455                 460

Ala Gln Asp Asn Ile Thr Val Gly Gly Thr Thr Gln Ser Trp Gly Asn
465                 470                 475                 480

Leu Ser Asn Val Glu Ile Leu Ser Ala Ala Ala Asp Ala Ser Val Val
                485                 490                 495

Phe Val Leu Ser Asp Ser Gly Glu Ser Thr Gly Ile Val Asp Gly Asn
            500                 505                 510

Ile Gly Asp Arg Asn Asn Leu Thr Leu Trp His Asn Gly Asp Ala Val
        515                 520                 525

Val Lys Ala Val Ala Ser Arg Asn Pro Asn Thr Ile Val Val Val Thr
    530                 535                 540

Thr Val Gly Pro Val Asn Leu Glu Lys Trp Ile Asn Asn Pro Asn Val
545                 550                 555                 560

Thr Ala Val Leu Leu Thr Gly Pro Ala Gly Asp Phe Gly Gly Arg Ala
                565                 570                 575

Ala Ala Ser Ile Leu Phe Gly Asp Ile Ala Pro Ser Gly Lys Leu Pro
            580                 585                 590

Phe Thr Ile Ala Lys Asn Asp Thr Asp Tyr Ile Pro Leu Thr Thr Lys
        595                 600                 605

Val Pro Lys Asp Gly Leu Pro Gln Asp Tyr Phe Thr Glu Gly Thr Leu
    610                 615                 620

Leu Asp Tyr Lys Arg Phe Asp Glu Asn Lys Val Thr Pro Arg Phe Glu
625                 630                 635                 640

Phe Gly Tyr Gly Leu Ser Tyr Ser Asn Ile Thr Met Glu Asn Leu Glu
                645                 650                 655

Ala Arg Phe Ala Phe Pro Asn Ile Pro Glu Phe Leu Pro Thr Pro Phe
            660                 665                 670

Ala Pro Leu Asn Pro His Lys Pro Asn Asp Val Phe Thr Pro Asn Ala
        675                 680                 685

Asn Gln Ser Val Phe Pro Asp Asp Ile His Pro Leu His Lys Tyr Val
    690                 695                 700

Tyr Pro Tyr Leu Asn Asp Thr Ser Glu Ile Phe Thr Ser Asn Glu Thr
705                 710                 715                 720

Asn Tyr Pro Tyr Pro Glu Gly Tyr Ser Gln Lys Gln Ser Asn Thr Thr
                725                 730                 735

Asn Ile Asn Gly Gly Ala Val Gly Gly Asn Pro Ala Leu Trp Leu Ser
            740                 745                 750

Ser Val Tyr Ile Val His Ser Val Ser Asn Tyr Gly Pro Tyr Asp Thr
        755                 760                 765

Gly Val Val Thr Gln Met Tyr Ile Ala Phe Pro Gln Asp Asn Asp Asp
    770                 775                 780

Leu Lys Thr Ala Pro Arg Gln Leu Arg Gly Phe Glu Arg Ser Glu Leu
785                 790                 795                 800

Lys Val Gly Glu Arg His Gly Ile Leu Tyr Asn Val Gln Trp Arg Asp
                805                 810                 815

Leu Ala Val Trp Asp Val Lys Leu Gln Ser Trp Arg Val Gln Arg Gly
            820                 825                 830

Glu Tyr Lys Val Tyr Val Gly His Ser Ser Arg Asp Phe Val Leu Thr
        835                 840                 845

Thr Ser Phe Thr Leu Gln
    850

<210>  10

<211>  874
<212>  PRT
<213>  Yarrowia galli

<220>
<221>  SIGNAL
<222>  (1)..(20)

<400>  10

Met Leu Ala Phe Val Leu Leu Leu Thr Thr Leu Gln Ala Leu Leu Thr
1               5                   10                  15

Ala Val Leu Ala Ala Asp Pro Phe Ser Asp Lys Asp Ala Tyr Lys His
            20                  25                  30

Ser Pro Pro Tyr Tyr Pro Ala Pro Glu Ile Gly Arg Val Pro Thr Asp
        35                  40                  45

Leu Arg Trp Arg Ala Ala Leu Lys Val Ala Gln Asp Met Val Ala Asn
    50                  55                  60

Met Thr Leu Ile Glu Lys Val Asn Ile Thr Thr Gly Thr Gly Trp Glu
65                  70                  75                  80

Met Gly Pro Cys Val Gly Asn Thr Gly Thr Val Glu Arg Leu Gly Ile
                85                  90                  95

Lys Ser Leu Cys Leu Gln Asp Gly Pro Leu Gly Ile Arg Phe Ala Asp
            100                 105                 110

Leu Ile Thr Thr Phe Pro Ala Gly Ile Thr Ile Ala Ser Thr Phe Ser
        115                 120                 125

Lys Gln Leu Val Arg Glu Arg Gly Glu Ala Met Gly Arg Glu Asn Arg
    130                 135                 140

Arg Lys Gly Val Asp Ile Thr Leu Ser Pro Val Val Gly Pro Leu Gly
145                 150                 155                 160

Arg His Ala Asn Ala Gly Arg Ile Trp Glu Gly Phe Ser Ala Asp Pro
                165                 170                 175

Tyr Leu Ala Gly Lys Leu Ala Ala Glu Ala Val Thr Gly Ile Gln Ser
            180                 185                 190

Gln Asn Val Met Ala Val Val Lys His Ile Val Gly Asn Glu Gln Glu
        195                 200                 205

His Phe Arg Gln Leu Gly Glu Trp Gln Gly Phe Gly Phe Lys Asp Leu
    210                 215                 220

Lys Gln Pro Leu Ser Ser Asn Ile Asp Asp Arg Thr Leu Asn Glu Ala
225                 230                 235                 240

Tyr Leu Trp Pro Phe Ala Asp Ala Val Arg Ala Asn Val Gly Ser Val
                245                 250                 255

Met Cys Ser Tyr Gln Gln Ile Asn Gly Ser Gln Gly Cys Gln Asn Ser
            260                 265                 270

His Ile Leu Asn Gly Lys Leu Lys Glu Glu Met Gly Phe Gln Gly Phe
        275                 280                 285

Val Met Ser Asp Trp Leu Ala Gln Arg Ser Gly Val Ala Ser Val Leu
    290                 295                 300

Ala Gly Leu Asp Met Ser Met Pro Gly Asp Gly Leu Val Trp Ala Asp
305                 310                 315                 320

Gly Val Pro Leu Met Gly Tyr Glu Leu Thr Lys Ser Val Leu Asn Gly
                325                 330                 335

Thr Ile Asp Ile Asn Arg Val Asp Asp Met Val Thr Arg Ile Leu Thr
            340                 345                 350

Pro Leu Met Tyr Leu Ser Ile Thr Pro Gly Asn Pro Asn Phe Ser Ser
        355                 360                 365

Trp Thr Asn Asp Thr Thr Ser Tyr Lys Ile Tyr Gly Ala Lys Ala Gly
    370                 375                 380

Gly Asn Val Thr Val Asn Arg His Ile Asp Val Arg Asp Pro Trp Thr
385                 390                 395                 400

Thr Lys Ala Ala Leu Asp Gly Ala Asn Ala Ala Leu Val Leu Leu Lys
                405                 410                 415

Asn Val Lys Asn Ala Leu Pro Leu Lys Pro Thr Asn Ile Gly Asn Leu
            420                 425                 430

Asn Ile Phe Gly Ile Gly Ser Ala Thr Gly Pro Leu Gly Ala Val Cys
        435                 440                 445

Gly Glu Asn Met Gln Cys Ser Asp Gly Ala Leu Ile Glu Gly Trp Gly
    450                 455                 460

Ser Gly Ser Val Tyr Pro Thr Asp Tyr Gln Ser Pro Tyr Glu Ala Ile
465                 470                 475                 480

Lys Glu Arg Ala Ala Gln Asp Asn Ile Thr Val Gly Gly Thr Thr Gln
                485                 490                 495

Ser Trp Gly Asn Leu Ser Asn Val Glu Ile Leu Ser Ala Ala Ala Asp
            500                 505                 510

Ala Ser Val Val Phe Val Leu Ser Asp Ser Gly Glu Ser Thr Gly Ile
        515                 520                 525

Val Asp Gly Asn Ile Gly Asp Arg Asn Asn Leu Thr Leu Trp His Asn
    530                 535                 540

Gly Asp Ala Val Val Lys Ala Val Ala Ser Arg Asn Pro Asn Thr Ile
545                 550                 555                 560

Val Val Val Thr Thr Val Gly Pro Val Asn Leu Glu Lys Trp Ile Asn
                565                 570                 575

Asn Pro Asn Val Thr Ala Val Leu Leu Thr Gly Pro Ala Gly Asp Phe
            580                 585                 590

Gly Gly Arg Ala Ala Ala Ser Ile Leu Phe Gly Asp Ile Ala Pro Ser
        595                 600                 605

Gly Lys Leu Pro Phe Thr Ile Ala Lys Asn Asp Thr Asp Tyr Ile Pro
    610                 615                 620

Leu Thr Thr Lys Val Pro Lys Asp Gly Leu Pro Gln Asp Tyr Phe Thr
625                 630                 635                 640

Glu Gly Thr Leu Leu Asp Tyr Lys Arg Phe Asp Glu Asn Lys Val Thr
                645                 650                 655

Pro Arg Phe Glu Phe Gly Tyr Gly Leu Ser Tyr Ser Asn Ile Thr Met
            660                 665                 670

Glu Asn Leu Glu Ala Arg Phe Ala Phe Pro Asn Ile Pro Glu Phe Leu
        675                 680                 685

Pro Thr Pro Phe Ala Pro Leu Asn Pro His Lys Pro Asn Asp Val Phe
    690                 695                 700

Thr Pro Asn Ala Asn Gln Ser Val Phe Pro Asp Asp Ile His Pro Leu
705                 710                 715                 720

His Lys Tyr Val Tyr Pro Tyr Leu Asn Asp Thr Ser Glu Ile Phe Thr
                725                 730                 735

Ser Asn Glu Thr Asn Tyr Pro Tyr Pro Glu Gly Tyr Ser Gln Lys Gln
            740                 745                 750

Ser Asn Thr Thr Asn Ile Asn Gly Gly Ala Val Gly Gly Asn Pro Ala
        755                 760                 765

Leu Trp Leu Ser Ser Val Tyr Ile Val His Ser Val Ser Asn Tyr Gly
    770                 775                 780

Pro Tyr Asp Thr Gly Val Val Thr Gln Met Tyr Ile Ala Phe Pro Gln
785                 790                 795                 800

Asp Asn Asp Asp Leu Lys Thr Ala Pro Arg Gln Leu Arg Gly Phe Glu
                805                 810                 815

Arg Ser Glu Leu Lys Val Gly Glu Arg His Gly Ile Leu Tyr Asn Val
            820                 825                 830

Gln Trp Arg Asp Leu Ala Val Trp Asp Val Lys Leu Gln Ser Trp Arg
        835                 840                 845

Val Gln Arg Gly Glu Tyr Lys Val Tyr Val Gly His Ser Ser Arg Asp
    850                 855                 860

Phe Val Leu Thr Thr Ser Phe Thr Leu Gln
865                 870

<210>  11
<211>  854
<212>  PRT
<213>  Yarrowia galli

<400>  11

Ala Asp Pro Phe Ser Asp Lys Asp Ala Tyr Lys His Ser Pro Pro Tyr
1               5                   10                  15

Tyr Pro Ala Pro Glu Ile Gly Arg Val Pro Thr Asp Leu Arg Trp Arg
            20                  25                  30

Ala Ala Leu Lys Val Ala Gln Asp Met Val Ala Asn Met Thr Leu Ile
        35                  40                  45

Glu Lys Val Asn Ile Thr Thr Gly Thr Gly Trp Glu Met Gly Pro Cys
    50                  55                  60

Val Gly Asn Thr Gly Thr Val Glu Arg Leu Gly Ile Lys Ser Leu Cys
65                  70                  75                  80

Leu Gln Asp Gly Pro Leu Gly Ile Arg Phe Ala Asp Leu Ile Thr Thr
                85                  90                  95

Phe Pro Ala Gly Ile Thr Ile Ala Ser Thr Phe Ser Lys Gln Leu Val
            100                 105                 110

Arg Glu Arg Gly Glu Ala Met Gly Arg Glu Asn Arg Arg Lys Gly Val
        115                 120                 125

Asp Ile Thr Leu Ser Pro Val Val Gly Pro Leu Gly Arg His Ala Asn
    130                 135                 140

Ala Gly Arg Ile Trp Glu Gly Phe Ser Ala Asp Pro Tyr Leu Ala Gly
145                 150                 155                 160

Lys Leu Ala Ala Glu Ala Val Thr Gly Ile Gln Ser Gln Asn Val Met
                165                 170                 175

Ala Val Val Lys His Ile Val Gly Asn Glu Gln Glu His Phe Arg Gln
            180                 185                 190

Leu Gly Glu Trp Gln Gly Phe Gly Phe Lys Asp Leu Lys Gln Pro Leu
        195                 200                 205

Ser Ser Asn Ile Asp Asp Arg Thr Leu Asn Glu Ala Tyr Leu Trp Pro
    210                 215                 220

Phe Ala Asp Ala Val Arg Ala Asn Val Gly Ser Val Met Cys Ser Tyr
225                 230                 235                 240

Gln Gln Ile Asn Gly Ser Gln Gly Cys Gln Asn Ser His Ile Leu Asn
                245                 250                 255

Gly Lys Leu Lys Glu Glu Met Gly Phe Gln Gly Phe Val Met Ser Asp
            260                 265                 270

Trp Leu Ala Gln Arg Ser Gly Val Ala Ser Val Leu Ala Gly Leu Asp
        275                 280                 285

Met Ser Met Pro Gly Asp Gly Leu Val Trp Ala Asp Gly Val Pro Leu
    290                 295                 300

Met Gly Tyr Glu Leu Thr Lys Ser Val Leu Asn Gly Thr Ile Asp Ile

305                 310                 315                 320

Asn Arg Val Asp Asp Met Val Thr Arg Ile Leu Thr Pro Leu Met Tyr
                325                 330                 335

Leu Ser Ile Thr Pro Gly Asn Pro Asn Phe Ser Ser Trp Thr Asn Asp
            340                 345                 350

Thr Thr Ser Tyr Lys Ile Tyr Gly Ala Lys Ala Gly Gly Asn Val Thr
        355                 360                 365

Val Asn Arg His Ile Asp Val Arg Asp Pro Trp Thr Thr Lys Ala Ala
    370                 375                 380

Leu Asp Gly Ala Asn Ala Ala Leu Val Leu Leu Lys Asn Val Lys Asn
385                 390                 395                 400

Ala Leu Pro Leu Lys Pro Thr Asn Ile Gly Asn Leu Asn Ile Phe Gly
                405                 410                 415

Ile Gly Ser Ala Thr Gly Pro Leu Gly Ala Val Cys Gly Glu Asn Met
            420                 425                 430

Gln Cys Ser Asp Gly Ala Leu Ile Glu Gly Trp Gly Ser Gly Ser Val
        435                 440                 445

Tyr Pro Thr Asp Tyr Gln Ser Pro Tyr Glu Ala Ile Lys Glu Arg Ala
    450                 455                 460

Ala Gln Asp Asn Ile Thr Val Gly Gly Thr Thr Gln Ser Trp Gly Asn
465                 470                 475                 480

Leu Ser Asn Val Glu Ile Leu Ser Ala Ala Ala Asp Ala Ser Val Val
                485                 490                 495

Phe Val Leu Ser Asp Ser Gly Glu Ser Thr Gly Ile Val Asp Gly Asn
            500                 505                 510

Ile Gly Asp Arg Asn Asn Leu Thr Leu Trp His Asn Gly Asp Ala Val
        515                 520                 525

Val Lys Ala Val Ala Ser Arg Asn Pro Asn Thr Ile Val Val Val Thr
    530                 535                 540

Thr Val Gly Pro Val Asn Leu Glu Lys Trp Ile Asn Asn Pro Asn Val
545                 550                 555                 560

Thr Ala Val Leu Leu Thr Gly Pro Ala Gly Asp Phe Gly Gly Arg Ala
                565                 570                 575

Ala Ala Ser Ile Leu Phe Gly Asp Ile Ala Pro Ser Gly Lys Leu Pro
            580                 585                 590

Phe Thr Ile Ala Lys Asn Asp Thr Asp Tyr Ile Pro Leu Thr Thr Lys
        595                 600                 605

Val Pro Lys Asp Gly Leu Pro Gln Asp Tyr Phe Thr Glu Gly Thr Leu
    610                 615                 620

Leu Asp Tyr Lys Arg Phe Asp Glu Asn Lys Val Thr Pro Arg Phe Glu
625                 630                 635                 640

Phe Gly Tyr Gly Leu Ser Tyr Ser Asn Ile Thr Met Glu Asn Leu Glu
                645                 650                 655

Ala Arg Phe Ala Phe Pro Asn Ile Pro Glu Phe Leu Pro Thr Pro Phe
            660                 665                 670

Ala Pro Leu Asn Pro His Lys Pro Asn Asp Val Phe Thr Pro Asn Ala
        675                 680                 685

Asn Gln Ser Val Phe Pro Asp Asp Ile His Pro Leu His Lys Tyr Val
    690                 695                 700

Tyr Pro Tyr Leu Asn Asp Thr Ser Glu Ile Phe Thr Ser Asn Glu Thr
705                 710                 715                 720

Asn Tyr Pro Tyr Pro Glu Gly Tyr Ser Gln Lys Gln Ser Asn Thr Thr
                725                 730                 735

Asn Ile Asn Gly Gly Ala Val Gly Gly Asn Pro Ala Leu Trp Leu Ser
            740                 745                 750

Ser Val Tyr Ile Val His Ser Val Ser Asn Tyr Gly Pro Tyr Asp Thr
        755                 760                 765

Gly Val Val Thr Gln Met Tyr Ile Ala Phe Pro Gln Asp Asn Asp Asp
    770                 775                 780

Leu Lys Thr Ala Pro Arg Gln Leu Arg Gly Phe Glu Arg Ser Glu Leu
785                 790                 795                 800

Lys Val Gly Glu Arg His Gly Ile Leu Tyr Asn Val Gln Trp Arg Asp
                805                 810                 815

Leu Ala Val Trp Asp Val Lys Leu Gln Ser Trp Arg Val Gln Arg Gly
            820                 825                 830

Glu Tyr Lys Val Tyr Val Gly His Ser Ser Arg Asp Phe Val Leu Thr
        835                 840                 845

Thr Ser Phe Thr Leu Gln
    850

<210>  12
<211>  867
<212>  PRT
<213>  Yarrowia yakushimensis

<220>
<221>  SIGNAL
<222>  (1)..(17)

<400>  12

Met Leu Ala Phe Val Leu Leu Leu Thr Ser Leu Leu Thr Ala Val Leu
1               5                   10                  15

Ala Asp Pro Phe Ser Asp Lys Ala Ala Tyr Lys His Ser Pro Pro Tyr
            20                  25                  30

Tyr Pro Ala Pro Glu Ile Gly Arg Ile Pro Thr Asp Leu Arg Trp Ala
        35                  40                  45

Ala Ala Leu Glu Val Ala Gln Asn Leu Val Ala Asn Met Thr Leu Ile
    50                  55                  60

Glu Lys Val Asn Ile Thr Thr Gly Thr Gly Trp Glu Met Gly Pro Cys
65                  70                  75                  80

Val Gly Asn Thr Ser Pro Val Glu Arg Leu Gly Ile Lys Ser Leu Cys
                85                  90                  95

Leu Gln Asp Gly Pro Leu Gly Val Arg Leu Thr Asp Leu Ile Thr Ala
            100                 105                 110

Phe Pro Ala Gly Ile Thr Met Ala Ser Thr Phe Ser Arg Gln Leu Val
        115                 120                 125

Arg Glu Arg Gly Ala Ala Met Gly Arg Glu Asn Arg Arg Lys Gly Val
    130                 135                 140

Asp Ile Thr Leu Ser Pro Val Val Gly Pro Leu Gly Arg Asn Ala Asn
145                 150                 155                 160

Ala Gly Arg Ile Trp Glu Gly Phe Ser Ala Asp Pro Tyr Leu Ala Gly
                165                 170                 175

Lys Leu Ala Ala Glu Ala Val Thr Gly Ile Gln Ser Gln Asn Val Met
            180                 185                 190

Ala Val Val Lys His Leu Val Gly Asn Glu Gln Glu His Phe Arg Gln
        195                 200                 205

Leu Gly Glu Trp Gln Gly Tyr Gly Tyr Lys Asp Leu Lys Gln Pro Leu
    210                 215                 220

Ser Ser Asn Ile Asp Asp Arg Thr Leu Asn Glu Ala Tyr Leu Trp Pro
225                 230                 235                 240

Phe Ala Asp Ala Ile Arg Ala Asn Val Gly Ser Val Met Cys Ser Tyr
                245                 250                 255

Gln Gln Ile Asn Ser Ser Gln Gly Cys Gln Asn Ser Phe Ile Leu Asn
            260                 265                 270

Gly Lys Ile Lys Glu Glu Met Gly Phe Gln Gly Phe Ile Met Ser Asp
        275                 280                 285

Trp Leu Ala Gln Arg Ser Gly Val Ala Ser Val Leu Ala Gly Leu Asp
    290                 295                 300

Met Ser Met Pro Gly Asp Gly Leu Val Trp Gly Leu Gly Ile Pro Leu
305                 310                 315                 320

Met Gly Tyr Glu Leu Thr Arg Ser Val Leu Asn Gly Thr Ile Asp Glu
                325                 330                 335

Ser Arg Val Asp Asp Met Val Thr Arg Ile Leu Thr Pro Ile Leu Tyr
            340                 345                 350

Leu Ser Leu Thr Pro Arg Thr Pro Asn Phe Ser Ser Trp Thr Asn Asp
        355                 360                 365

Thr Tyr Gly Tyr Glu Tyr Tyr Gly Asn Asp Gly Gly Arg Asn Ile Thr
    370                 375                 380

Ile Asn Arg His Val Asp Val Arg Asp Ala Tyr Thr Ile Gln Ala Ala
385                 390                 395                 400

Gln Asp Gly Ala Asn Ala Ala Leu Val Leu Leu Lys Asn Glu Lys Asn

                405                 410                 415

Ala Leu Pro Leu Asp Pro Ala Lys Leu Gly Thr Ile Asn Ile Phe Gly
            420                 425                 430

Ile Gly Ser Lys Thr Gly Asn Leu Ser Cys Gly Gln Asn Met Glu Cys
        435                 440                 445

Ser Asn Gly Ala Leu Ile Gln Gly Trp Gly Ser Gly Ser Val Tyr Pro
    450                 455                 460

Thr Asp Tyr Leu Ser Pro Tyr Asn Ala Ile Lys Glu Arg Ala Ala Gln
465                 470                 475                 480

Asn Asn Ile Thr Val Gly Gly Thr Thr His Ser Trp Gly Asn Leu Ser
                485                 490                 495

Ile Val Glu Lys Leu Ser Ala Ala Ala Asp Ala Ser Val Val Phe Val
            500                 505                 510

Leu Ser Asp Ser Gly Glu Ser Thr Gly Pro Val Asp Gly Asn Tyr Gly
        515                 520                 525

Asp Arg Asn Asn Leu Thr Leu Trp His Asn Gly Asp Gln Val Val Lys
    530                 535                 540

Ala Val Ala Ser Lys Asn Pro Asn Thr Ile Val Val Val Thr Thr Val
545                 550                 555                 560

Gly Pro Val Asn Leu Glu Lys Trp Ile Asp His Pro Asn Val Thr Ala
                565                 570                 575

Val Leu Leu Thr Gly Pro Ala Gly Asp Phe Gly Gly Ser Ala Ala Ala
            580                 585                 590

Ser Ile Leu Phe Gly Asp Ile Ala Pro Ser Gly Lys Leu Pro Phe Thr
        595                 600                 605

Ile Ala Lys Asn Asn Thr Asp Tyr Ile Pro Leu Val Thr Lys Val Pro
    610                 615                 620

Glu Asp Gly Leu Pro Gln Asp Tyr Phe Thr Glu Gly Thr Leu Leu Asp
625                 630                 635                 640

Tyr Lys Arg Phe Asp Glu Asn Lys Val Thr Pro Arg Phe Glu Phe Gly
                645                 650                 655

Tyr Gly Leu Ser Tyr Ser Asn Ile Thr Val Glu Asn Leu Asp Val Leu
            660                 665                 670

Phe Ala Phe Pro Asn Ile Ser Glu Phe Leu Pro Ala Pro Ser Ala Pro
        675                 680                 685

Leu Ile Pro His Lys Pro Lys Asn Arg Phe Thr Pro Ser Leu Asn Glu
    690                 695                 700

Ser Ile Phe Pro Ser Gly Phe Lys Gln Leu Lys Asn Tyr Val Tyr Thr
705                 710                 715                 720

Tyr Leu Asn Arg Thr Ser Asp Ile Ala Ser Asn Ile Thr His Tyr Pro
                725                 730                 735

Tyr Pro Glu Gly Tyr Ser Ser Val Gln Ser Asn Thr Thr Asn Ile Asn
            740                 745                 750

Gly Gly Ala Val Gly Gly Asn Pro Ala Leu Trp Leu Pro Ala Leu Tyr
        755                 760                 765

Val Val His Ser Val Ser Asn Tyr Gly Pro Tyr Asp Thr Gly Ala Val
    770                 775                 780

Thr Gln Leu Tyr Ile Ser Phe Pro Gln Asp Asn Glu Asp Leu Arg Thr
785                 790                 795                 800

Ala Pro Arg Gln Leu Arg Gly Phe Glu Arg Ser Glu Leu Lys Val Gly
                805                 810                 815

Asp Gln Gln Gly Val Leu Tyr Asp Ile Gln Trp Arg Asp Leu Ala Val
            820                 825                 830

Trp Asp Val Lys Leu Gln Ser Trp Arg Val Gln Arg Gly Lys Tyr Thr
        835                 840                 845

Val Tyr Ile Gly His Ser Ser Arg Asp Leu Val Leu Ser Thr Thr Phe
    850                 855                 860

Thr Leu Lys
865

<210>  13
<211>  850
<212>  PRT
<213>  Yarrowia yakushimensis

<400>  13

Asp Pro Phe Ser Asp Lys Ala Ala Tyr Lys His Ser Pro Pro Tyr Tyr
1               5                   10                  15

Pro Ala Pro Glu Ile Gly Arg Ile Pro Thr Asp Leu Arg Trp Ala Ala
            20                  25                  30

Ala Leu Glu Val Ala Gln Asn Leu Val Ala Asn Met Thr Leu Ile Glu
        35                  40                  45

Lys Val Asn Ile Thr Thr Gly Thr Gly Trp Glu Met Gly Pro Cys Val
    50                  55                  60

Gly Asn Thr Ser Pro Val Glu Arg Leu Gly Ile Lys Ser Leu Cys Leu
65                  70                  75                  80

Gln Asp Gly Pro Leu Gly Val Arg Leu Thr Asp Leu Ile Thr Ala Phe
                85                  90                  95

Pro Ala Gly Ile Thr Met Ala Ser Thr Phe Ser Arg Gln Leu Val Arg
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg Asn Ala Asn Ala
    130                 135                 140

Gly Arg Ile Trp Glu Gly Phe Ser Ala Asp Pro Tyr Leu Ala Gly Lys
145                 150                 155                 160

Leu Ala Ala Glu Ala Val Thr Gly Ile Gln Ser Gln Asn Val Met Ala
                165                 170                 175

Val Val Lys His Leu Val Gly Asn Glu Gln Glu His Phe Arg Gln Leu
            180                 185                 190

Gly Glu Trp Gln Gly Tyr Gly Tyr Lys Asp Leu Lys Gln Pro Leu Ser
        195                 200                 205

Ser Asn Ile Asp Asp Arg Thr Leu Asn Glu Ala Tyr Leu Trp Pro Phe
    210                 215                 220

Ala Asp Ala Ile Arg Ala Asn Val Gly Ser Val Met Cys Ser Tyr Gln
225                 230                 235                 240

Gln Ile Asn Ser Ser Gln Gly Cys Gln Asn Ser Phe Ile Leu Asn Gly
                245                 250                 255

Lys Ile Lys Glu Glu Met Gly Phe Gln Gly Phe Ile Met Ser Asp Trp
        260                 265                 270

Leu Ala Gln Arg Ser Gly Val Ala Ser Val Leu Ala Gly Leu Asp Met
    275                 280                 285

Ser Met Pro Gly Asp Gly Leu Val Trp Gly Leu Gly Ile Pro Leu Met
    290                 295                 300

Gly Tyr Glu Leu Thr Arg Ser Val Leu Asn Gly Thr Ile Asp Glu Ser
305                 310                 315                 320

Arg Val Asp Asp Met Val Thr Arg Ile Leu Thr Pro Ile Leu Tyr Leu
                325                 330                 335

Ser Leu Thr Pro Arg Thr Pro Asn Phe Ser Ser Trp Thr Asn Asp Thr
            340                 345                 350

Tyr Gly Tyr Glu Tyr Tyr Gly Asn Asp Gly Gly Arg Asn Ile Thr Ile
        355                 360                 365

Asn Arg His Val Asp Val Arg Asp Ala Tyr Thr Ile Gln Ala Ala Gln
    370                 375                 380

Asp Gly Ala Asn Ala Ala Leu Val Leu Leu Lys Asn Glu Lys Asn Ala
385                 390                 395                 400

Leu Pro Leu Asp Pro Ala Lys Leu Gly Thr Ile Asn Ile Phe Gly Ile
                405                 410                 415

Gly Ser Lys Thr Gly Asn Leu Ser Cys Gly Gln Asn Met Glu Cys Ser
            420                 425                 430

Asn Gly Ala Leu Ile Gln Gly Trp Gly Ser Gly Ser Val Tyr Pro Thr
        435                 440                 445

Asp Tyr Leu Ser Pro Tyr Asn Ala Ile Lys Glu Arg Ala Ala Gln Asn
    450                 455                 460

Asn Ile Thr Val Gly Gly Thr Thr His Ser Trp Gly Asn Leu Ser Ile
465                 470                 475                 480

Val Glu Lys Leu Ser Ala Ala Ala Asp Ala Ser Val Val Phe Val Leu
                485                 490                 495

Ser Asp Ser Gly Glu Ser Thr Gly Pro Val Asp Gly Asn Tyr Gly Asp
            500                 505                 510

Arg Asn Asn Leu Thr Leu Trp His Asn Gly Asp Gln Val Val Lys Ala
        515                 520                 525

Val Ala Ser Lys Asn Pro Asn Thr Ile Val Val Val Thr Thr Val Gly
    530                 535                 540

Pro Val Asn Leu Glu Lys Trp Ile Asp His Pro Asn Val Thr Ala Val
545                 550                 555                 560

Leu Leu Thr Gly Pro Ala Gly Asp Phe Gly Gly Ser Ala Ala Ala Ser
                565                 570                 575

Ile Leu Phe Gly Asp Ile Ala Pro Ser Gly Lys Leu Pro Phe Thr Ile
            580                 585                 590

Ala Lys Asn Asn Thr Asp Tyr Ile Pro Leu Val Thr Lys Val Pro Glu
        595                 600                 605

Asp Gly Leu Pro Gln Asp Tyr Phe Thr Glu Gly Thr Leu Leu Asp Tyr
    610                 615                 620

Lys Arg Phe Asp Glu Asn Lys Val Thr Pro Arg Phe Glu Phe Gly Tyr
625                 630                 635                 640

Gly Leu Ser Tyr Ser Asn Ile Thr Val Glu Asn Leu Asp Val Leu Phe
                645                 650                 655

Ala Phe Pro Asn Ile Ser Glu Phe Leu Pro Ala Pro Ser Ala Pro Leu
            660                 665                 670

Ile Pro His Lys Pro Lys Asn Arg Phe Thr Pro Ser Leu Asn Glu Ser
        675                 680                 685

Ile Phe Pro Ser Gly Phe Lys Gln Leu Lys Asn Tyr Val Tyr Thr Tyr
    690                 695                 700

Leu Asn Arg Thr Ser Asp Ile Ala Ser Asn Ile Thr His Tyr Pro Tyr
705                 710                 715                 720

Pro Glu Gly Tyr Ser Ser Val Gln Ser Asn Thr Thr Asn Ile Asn Gly
                725                 730                 735

Gly Ala Val Gly Gly Asn Pro Ala Leu Trp Leu Pro Ala Leu Tyr Val
            740                 745                 750

Val His Ser Val Ser Asn Tyr Gly Pro Tyr Asp Thr Gly Ala Val Thr

755                 760                 765

Gln Leu Tyr Ile Ser Phe Pro Gln Asp Asn Glu Asp Leu Arg Thr Ala
    770                 775                 780

Pro Arg Gln Leu Arg Gly Phe Glu Arg Ser Glu Leu Lys Val Gly Asp
785                 790                 795                 800

Gln Gln Gly Val Leu Tyr Asp Ile Gln Trp Arg Asp Leu Ala Val Trp
                805                 810                 815

Asp Val Lys Leu Gln Ser Trp Arg Val Gln Arg Gly Lys Tyr Thr Val
            820                 825                 830

Tyr Ile Gly His Ser Ser Arg Asp Leu Val Leu Ser Thr Thr Phe Thr
        835                 840                 845

Leu Lys
    850

<210>  14
<211>  866
<212>  PRT
<213>  Yarrowia alimentaria

<220>
<221>  SIGNAL
<222>  (1)..(16)

<400>  14

Met Phe Ser Leu Leu Leu Leu Leu Thr Leu Leu Ser Thr Ala Leu Ala
1               5                   10                  15

Asp Pro Phe Ala Asp Arg Asp Ser Tyr Lys His Ser Pro Pro Tyr Tyr
            20                  25                  30

Pro Ala Pro Glu Ile Gly Arg Val Leu Thr Asp Arg Arg Trp Arg Thr
        35                  40                  45

Ala Met Asn Ala Ala Gln Ser Leu Val Ala Asn Met Thr Leu Tyr Glu
    50                  55                  60

Lys Val Asn Ile Thr Ser Gly Thr Gly Trp Glu Met Gly Pro Cys Val
65                  70                  75                  80

Gly Asn Thr Gly Thr Val Glu Arg Leu Gly Ile Lys Ser Ile Cys Leu
                85                  90                  95

Gln Asp Gly Pro Leu Gly Ile Arg Phe Ala Asp Lys Ile Thr Ala Phe
            100                 105                 110

Pro Ala Gly Ile Thr Ile Ala Ser Thr Phe Ser Arg Lys Leu Val Gln
        115                 120                 125

Glu Arg Gly Ala Ala Met Gly Arg Glu Asn Arg Arg Lys Gly Val Asp
    130                 135                 140

Ile Thr Leu Ser Pro Val Val Gly Pro Leu Gly Arg His Ala Asn Gly
145                 150                 155                 160

Gly Arg Ile Trp Glu Gly Phe Ser Ala Asp Pro Tyr Leu Ala Gly Lys
                165                 170                 175

Leu Ala Ala Gln Ala Val Glu Gly Ile Gln Ser Gln Asn Val Met Ala
            180                 185                 190

Val Val Lys His Met Val Gly Asn Glu Gln Glu His Phe Arg Gln Leu
        195                 200                 205

Gly Glu Trp Gln Gly Phe Gly Phe Lys Asp Leu Lys Gln Pro Leu Ser
    210                 215                 220

Ser Asn Ile Asp Asp Arg Thr Met Asn Glu Ala Tyr Leu Trp Pro Phe
225                 230                 235                 240

Ala Asp Ala Val Arg Ala Asn Val Gly Ser Val Met Cys Ser Tyr Gln
                245                 250                 255

Gln Ile Asn Gly Ser Gln Gly Cys Gln Asn Ser Ala Leu Leu Asn Gly
            260                 265                 270

Lys Leu Lys Glu Glu Phe Gly Phe Gln Gly Phe Val Met Ser Asp Trp
        275                 280                 285

Leu Ala Gln Arg Ser Gly Val Ala Ser Val Leu Ala Gly Leu Asp Met
    290                 295                 300

Ser Met Pro Gly Asp Gly Leu Val Trp Ala Asp Gly Ile Pro Leu Met
305                 310                 315                 320

Gly Tyr Glu Leu Thr Lys Met Val Leu Asn Gly Thr Val Glu Gln Ser
                325                 330                 335

Arg Val Asp Asp Met Val Thr Arg Ile Leu Thr Pro Leu Leu Tyr Leu
            340                 345                 350

Ser Ile Thr Pro Gly Asn Pro Asn Phe Ser Ser Trp Thr Asn Glu Thr
        355                 360                 365

Thr Gly Leu Gln Phe Pro Gly Ala Asp Arg Gly Pro Asn Ile Thr Val
    370                 375                 380

Asn Gln His Val Glu Val Arg Asp Ser Tyr Thr Thr Lys Ala Ala Leu
385                 390                 395                 400

Asp Gly Ala Thr Ala Ala Leu Val Leu Leu Lys Asn Val Gln Ser Ala
                405                 410                 415

Leu Pro Leu Lys Asn Val Thr Lys Leu Asn Ile Phe Gly Val Gly Ser
            420                 425                 430

Thr Thr Gly Pro Leu Gly Ala Val Cys Gly Glu Asn Met Gln Cys Ser
        435                 440                 445

Asp Gly Ala Leu Ile Leu Gly Trp Gly Ser Gly Ser Val Tyr Pro Ser
    450                 455                 460

Asp Tyr Gln Ser Pro Tyr Glu Ala Ile Lys Glu Arg Ala Glu Gln Asp
465                 470                 475                 480

Lys Ile Gln Val Gly Gly Thr Thr Glu Ser Trp Gly Asn Leu Thr Arg
                485                 490                 495

Val Glu Gln Leu Ala Ala Glu Ser Asp Ala Ser Ile Val Phe Val Leu
            500                 505                 510

Ser Asp Ser Gly Glu Ser Thr Gly Ile Val Asp Gly Asn Ile Gly Asp
        515                 520                 525

Arg Asn Asn Leu Thr Leu Trp His Asn Gly Asp Glu Val Val Arg Thr
    530                 535                 540

Val Ala Ser Gln Ser Ala Asn Thr Ile Val Val Val Thr Thr Val Gly
545                 550                 555                 560

Pro Val Asn Leu Glu Lys Trp Ile Asn His Pro Asn Val Thr Ala Val
                565                 570                 575

Leu Leu Thr Gly Pro Ala Gly Asp Phe Gly Gly Lys Ala Ala Ala Ser
            580                 585                 590

Val Leu Phe Gly Asp Val Ala Pro Ser Gly Lys Leu Pro Phe Thr Ile
        595                 600                 605

Ala Arg Asn Asp Thr Asp Tyr Val Pro Leu Thr Thr Lys Val Pro Ser
    610                 615                 620

Asp Gly Leu Pro Gln Asp Phe Phe Thr Glu Gly Thr Leu Leu Asp Tyr
625                 630                 635                 640

Lys Arg Phe Asp Ala Leu Lys Lys Thr Pro Arg Phe Glu Phe Gly Tyr
                645                 650                 655

Gly Leu Ser Tyr Ser Asn Ile Thr Leu Gly Asn Leu Gln Ala Lys Phe
            660                 665                 670

Ala Phe Asn Asn Ile Pro Glu Phe Leu Pro Thr Pro Phe Pro Pro Leu
        675                 680                 685

Asp Pro His Lys Pro Ala Asn Val Phe Pro Thr Glu Leu Asn Gly Ser
    690                 695                 700

Val Phe Pro Ser Asp Ile Asp Pro Ile Pro Lys Tyr Val Tyr Pro Tyr
705                 710                 715                 720

Leu Asn Ser Thr Ser Glu Leu Asn Ser Asn Ile Ser Arg Tyr Pro Tyr
                725                 730                 735

Pro Glu Gly Phe Ser Val Val Gln Ser Asn Gly Thr Asn Ile Asn Gly
            740                 745                 750

Gly Ala Val Gly Gly Asn Pro Ala Leu Trp Leu Thr Ala Leu Tyr Val
        755                 760                 765

Ala His Ser Ile Ser Asn His Gly Pro Tyr Asp Thr Gly Val Val Thr
    770                 775                 780

Gln Leu Tyr Ile Ala Phe Pro Gln Asp Asn Glu Gly Leu Gln Thr Ala
785                 790                 795                 800

Pro Arg Gln Leu Arg Gly Phe Glu Arg Ser Glu Leu Pro Val Gly Glu
                805                 810                 815

Gln Arg Gly Val Gln Tyr Asp Ile Gln Trp Arg Asp Leu Ala Val Trp
            820                 825                 830

Asp Val Ser Ile Gln Ser Trp Arg Val Gln Arg Gly Asp Tyr Glu Ile
        835                 840                 845

Phe Val Gly His Ser Ser Arg Asp Leu Val Leu Ala Thr Lys Phe Thr

850                 855                 860

Leu Asn
865

<210>  15
<211>  850
<212>  PRT
<213>  Yarrowia alimentaria

<400>  15

Asp Pro Phe Ala Asp Arg Asp Ser Tyr Lys His Ser Pro Pro Tyr Tyr
1               5                   10                  15

Pro Ala Pro Glu Ile Gly Arg Val Leu Thr Asp Arg Arg Trp Arg Thr
            20                  25                  30

Ala Met Asn Ala Ala Gln Ser Leu Val Ala Asn Met Thr Leu Tyr Glu
        35                  40                  45

Lys Val Asn Ile Thr Ser Gly Thr Gly Trp Glu Met Gly Pro Cys Val
    50                  55                  60

Gly Asn Thr Gly Thr Val Glu Arg Leu Gly Ile Lys Ser Ile Cys Leu
65                  70                  75                  80

Gln Asp Gly Pro Leu Gly Ile Arg Phe Ala Asp Lys Ile Thr Ala Phe
                85                  90                  95

Pro Ala Gly Ile Thr Ile Ala Ser Thr Phe Ser Arg Lys Leu Val Gln
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg His Ala Asn Gly
    130                 135                 140

Gly Arg Ile Trp Glu Gly Phe Ser Ala Asp Pro Tyr Leu Ala Gly Lys
145                 150                 155                 160

Leu Ala Ala Gln Ala Val Glu Gly Ile Gln Ser Gln Asn Val Met Ala
                165                 170                 175

Val Val Lys His Met Val Gly Asn Glu Gln Glu His Phe Arg Gln Leu
            180                 185                 190

Gly Glu Trp Gln Gly Phe Gly Phe Lys Asp Leu Lys Gln Pro Leu Ser

195                 200                 205

Ser Asn Ile Asp Asp Arg Thr Met Asn Glu Ala Tyr Leu Trp Pro Phe
    210                 215                 220

Ala Asp Ala Val Arg Ala Asn Val Gly Ser Val Met Cys Ser Tyr Gln
225                 230                 235                 240

Gln Ile Asn Gly Ser Gln Gly Cys Gln Asn Ser Ala Leu Leu Asn Gly
                245                 250                 255

Lys Leu Lys Glu Glu Phe Gly Phe Gln Gly Phe Val Met Ser Asp Trp
            260                 265                 270

Leu Ala Gln Arg Ser Gly Val Ala Ser Val Leu Ala Gly Leu Asp Met
        275                 280                 285

Ser Met Pro Gly Asp Gly Leu Val Trp Ala Asp Gly Ile Pro Leu Met
    290                 295                 300

Gly Tyr Glu Leu Thr Lys Met Val Leu Asn Gly Thr Val Glu Gln Ser
305                 310                 315                 320

Arg Val Asp Asp Met Val Thr Arg Ile Leu Thr Pro Leu Leu Tyr Leu
                325                 330                 335

Ser Ile Thr Pro Gly Asn Pro Asn Phe Ser Ser Trp Thr Asn Glu Thr
            340                 345                 350

Thr Gly Leu Gln Phe Pro Gly Ala Asp Arg Gly Pro Asn Ile Thr Val
        355                 360                 365

Asn Gln His Val Glu Val Arg Asp Ser Tyr Thr Thr Lys Ala Ala Leu
    370                 375                 380

Asp Gly Ala Thr Ala Ala Leu Val Leu Leu Lys Asn Val Gln Ser Ala
385                 390                 395                 400

Leu Pro Leu Lys Asn Val Thr Lys Leu Asn Ile Phe Gly Val Gly Ser
                405                 410                 415

Thr Thr Gly Pro Leu Gly Ala Val Cys Gly Glu Asn Met Gln Cys Ser
            420                 425                 430

Asp Gly Ala Leu Ile Leu Gly Trp Gly Ser Gly Ser Val Tyr Pro Ser
        435                 440                 445

Asp Tyr Gln Ser Pro Tyr Glu Ala Ile Lys Glu Arg Ala Glu Gln Asp
    450                 455                 460

Lys Ile Gln Val Gly Gly Thr Thr Glu Ser Trp Gly Asn Leu Thr Arg
465                 470                 475                 480

Val Glu Gln Leu Ala Ala Glu Ser Asp Ala Ser Ile Val Phe Val Leu
                485                 490                 495

Ser Asp Ser Gly Glu Ser Thr Gly Ile Val Asp Gly Asn Ile Gly Asp
            500                 505                 510

Arg Asn Asn Leu Thr Leu Trp His Asn Gly Asp Glu Val Val Arg Thr
        515                 520                 525

Val Ala Ser Gln Ser Ala Asn Thr Ile Val Val Val Thr Thr Val Gly
    530                 535                 540

Pro Val Asn Leu Glu Lys Trp Ile Asn His Pro Asn Val Thr Ala Val
545                 550                 555                 560

Leu Leu Thr Gly Pro Ala Gly Asp Phe Gly Gly Lys Ala Ala Ala Ser
                565                 570                 575

Val Leu Phe Gly Asp Val Ala Pro Ser Gly Lys Leu Pro Phe Thr Ile
            580                 585                 590

Ala Arg Asn Asp Thr Asp Tyr Val Pro Leu Thr Thr Lys Val Pro Ser
        595                 600                 605

Asp Gly Leu Pro Gln Asp Phe Phe Thr Glu Gly Thr Leu Leu Asp Tyr
    610                 615                 620

Lys Arg Phe Asp Ala Leu Lys Lys Thr Pro Arg Phe Glu Phe Gly Tyr
625                 630                 635                 640

Gly Leu Ser Tyr Ser Asn Ile Thr Leu Gly Asn Leu Gln Ala Lys Phe
                645                 650                 655

Ala Phe Asn Asn Ile Pro Glu Phe Leu Pro Thr Pro Phe Pro Pro Leu
            660                 665                 670

Asp Pro His Lys Pro Ala Asn Val Phe Pro Thr Glu Leu Asn Gly Ser
        675                 680                 685

Val Phe Pro Ser Asp Ile Asp Pro Ile Pro Lys Tyr Val Tyr Pro Tyr
    690                 695                 700

Leu Asn Ser Thr Ser Glu Leu Asn Ser Asn Ile Ser Arg Tyr Pro Tyr
705                 710                 715                 720

Pro Glu Gly Phe Ser Val Val Gln Ser Asn Gly Thr Asn Ile Asn Gly
                725                 730                 735

Gly Ala Val Gly Gly Asn Pro Ala Leu Trp Leu Thr Ala Leu Tyr Val
            740                 745                 750

Ala His Ser Ile Ser Asn His Gly Pro Tyr Asp Thr Gly Val Val Thr
        755                 760                 765

Gln Leu Tyr Ile Ala Phe Pro Gln Asp Asn Glu Gly Leu Gln Thr Ala
    770                 775                 780

Pro Arg Gln Leu Arg Gly Phe Glu Arg Ser Glu Leu Pro Val Gly Glu
785                 790                 795                 800

Gln Arg Gly Val Gln Tyr Asp Ile Gln Trp Arg Asp Leu Ala Val Trp
                805                 810                 815

Asp Val Ser Ile Gln Ser Trp Arg Val Gln Arg Gly Asp Tyr Glu Ile
            820                 825                 830

Phe Val Gly His Ser Ser Arg Asp Leu Val Leu Ala Thr Lys Phe Thr
        835                 840                 845

Leu Asn
    850

<210>  16
<211>  37
<212>  DNA
<213>  Artificial Sequence

<220>
<223>  Primer

<400>  16
cgggatcccg cgatgatctt ctctctgcaa ctactac                              37

<210>  17
<211>  33
<212>  DNA
<213>  Artificial Sequence

<220>
<223>  Primer

<400>  17
cgcctaggct acaaagtgaa agtctcacat agc                                  33

<210>  18
<211>  30
<212>  DNA
<213>  Artificial Sequence

<220>
<223>  Primer

<400>  18
cccaagcttg ggtttggagg gggtgaaaaa                                        30


<210>  19
<211>  38
<212>  DNA
<213>  Artificial Sequence

<220>
<223>  Primer

<400>  19
cccaagcttg ggctaaagac ctaaccaatt cttagtct                               38


<210>  20
<211>  31
<212>  DNA
<213>  Artificial Sequence

<220>
<223>  Primer

<400>  20
cgggatcccg cgatgattgc aaaaataccc c                                      31


<210>  21
<211>  30
<212>  DNA
<213>  Artificial Sequence

<220>
<223>  Primer

<400>  21
cgcctaggct actggagagt aaaggactcg                                        30


<210>  22
<211>  31
<212>  DNA
<213>  Artificial Sequence

<220>
<223>  Primer

<400>  22
cgggatcccg cgatgctcgc attcgtccta c                                      31


<210>  23

<211>  32
<212>  DNA
<213>  Artificial Sequence

<220>
<223>  Primer

<400>  23
cgggatcccg ctacttgaga gtgaagctgg tg                                    32


<210>  24
<211>  33
<212>  DNA
<213>  Artificial Sequence

<220>
<223>  Primer

<400>  24
cgggatcccg cgatggctcc acccccgcct cct                                   33


<210>  25
<211>  33
<212>  DNA
<213>  Artificial Sequence

<220>
<223>  Primer

<400>  25
cgcctaggtt aagcaatcgt gatgcgacca agg                                   33


<210>  26
<211>  32
<212>  DNA
<213>  Artificial Sequence

<220>
<223>  Primer

<400>  26
cgcctaggcg cgatggagga attatcggag gc                                    32


<210>  27
<211>  29
<212>  DNA
<213>  Artificial Sequence

<220>
<223>  Primer

<400>  27
cgcctaggct accggctgaa cttctcttc                                        29


<210>  28
<211>  69
<212>  DNA
<213>  Artificial Sequence

<220>
<223>  Primer

<400>  28
cgcctaggtt aatgatggtg atgatggtgg ctgccgcgcg gcaccagcct aggcaaagtg      60

aaagtctca                                                              69


<210>  29
<211>  76
<212>  DNA
<213>  Artificial Sequence

<220>
<223>  Primer

<400>  29
cccaagcttg ggttaatgat ggtgatgatg gtggctgccg cgcggcacca gcctaggaag      60

acctaaccaa ttctta                                                      76


<210>  30
<211>  68
<212>  DNA
<213>  Artificial Sequence

<220>
<223>  Primer

<400>  30
cgcctaggtt aatgatggtg atgatggtgg ctgccgcgcg gcaccagcct aggctggaga      60

gtaaagga                                                               68


<210>  31
<211>  70
<212>  DNA
<213>  Artificial Sequence

<220>
<223>  Primer

<400>  31
cgggatcccg ttaatgatgg tgatgatggt ggctgccgcg cggcaccagc ctaggcttga      60

gagtgaagct                                                             70


<210>  32
<211>  67
<212>  DNA
<213>  Artificial Sequence

<220>
<223>  Primer

<400>  32
cgcctaggtt aatgatggtg atgatggtgg ctgccgcgcg gcaccagcct aggagcaatc      60

gtgatgc                                                                 67

<210>  33
<211>  68
<212>  DNA
<213>  Artificial Sequence

<220>
<223>  Primer

<400>  33
cgcctaggtt aatgatggtg atgatggtgg ctgccgcgcg gcaccagcct aggctgaact        60

tctcttcc                                                                68

<210>  34
<211>  16
<212>  PRT
<213>  Yarrowia lipolytica

<400>  34

Met Ile Phe Ser Leu Gln Leu Leu Leu Thr Thr Ala Leu Ala Ala Ser
1               5                   10                  15

<210>  35
<211>  16
<212>  PRT
<213>  Yarrowia galli

<400>  35

Met Ile Phe Ser Leu Gln Leu Leu Leu Thr Thr Val Pro Val Leu Ala
1               5                   10                  15

<210>  36
<211>  17
<212>  PRT
<213>  Yarrowia lipolytica

<400>  36

Met Leu Ala Phe Val Leu Leu Leu Thr Met Leu Leu Ala Ala Ala Leu
1               5                   10                  15

Ala

<210>  37
<211>  20
<212>  PRT
<213>  Yarrowia galli

<400>  37

Met Leu Ala Phe Val Leu Leu Leu Thr Thr Leu Gln Ala Leu Leu Thr
1               5                   10                  15

Ala Val Leu Ala
            20

<210>  38
<211>  17
<212>  PRT
<213>  Yarrowia yakushimensis

<400>  38

Met Leu Ala Phe Val Leu Leu Leu Thr Ser Leu Leu Thr Ala Val Leu
1               5                   10                  15

Ala

<210>  39
<211>  16
<212>  PRT
<213>  Yarrowia alimentaria

<400>  39

Met Phe Ser Leu Leu Leu Leu Leu Thr Leu Leu Ser Thr Ala Leu Ala
1               5                   10                  15